# EUROPEAN PATENT SPECIFICATION

(11) **EP 3 464 308 B1**
(45) Date of publication and mention of the grant of the patent: **19.08.2020**
(21) Application number: 17728920.4
(22) Date of filing: 31.05.2017
(51) Int. Cl.: C07H 19/20, A61K 31/7076, A61P 35/00, A61P 35/02

(54) **8-CHLOROADENOSINE PHOSPHORAMIDATE DERIVATIVES FOR USE IN THE TREATMENT OF CANCER**
8-CHLOROADENOSIN-PHOSPHORAMIDATDERIVATE FÜR DEREN VERWENDUNG IN DER BEHANDLUNG VON KREBS
DÉRIVÉS DE PHOSPHORAMIDATES DE LA 8-CHLOROADÉNOSINE ET LEUR UTILISATION DANS LE TRAITTEMENT DU CANCER

(30) Priority: 01.06.2016 GB 201609602
(43) Date of publication of application: 10.04.2019
(73) Proprietor: Nucana PLC, Edinburgh EH12 9DT (GB)
(72) Inventor: GRIFFITH, Hugh, Edinburgh EH12 9DT (GB); SERPI, Michaela, Cardiff South Glamorgan CF10 3NB (GB); SLUSARCZYK, Magdalena, Cardiff South Glamorgan CF10 3NB (GB)
(74) Representative: HGF
(86) International application number: PCT/GB2017/051554
(87) International publication number: WO 2017/207989

(56) References cited:
- WO-A1-2006/100439
- WO-A1-2014/076490
- WO-A2-2015/081133
- TADEUSZ ROBAK: "New nucleoside analogs for patients with hematological malignancies", EXPERT OPINION ON INVESTIGATIONAL D, INFORMA HEALTHCARE, UK, vol. 20, no. 3, 1 January 2011 (2011-01-01), pages 343-359, XP009186868, ISSN: 1354-3784, DOI: 10.1517/13543784.2011.554822
- JONG HYUN CHO ET AL: "Efficient synthesis of nucleoside aryloxy phosphoramidate prodrugs utilizing benzyloxycarbonyl protection", TETRAHEDRON, vol. 67, no. 30, 1 July 2011 (2011-07-01), pages 5487-5493, XP055092851, ISSN: 0040-4020, DOI: 10.1016/j.tet.2011.05.046

## Description

The present invention relates to chemical compounds, their preparation and their use in the treatment of cancer. Particularly, although not exclusively, the present invention relates to chemical compounds useful in the treatment of leukaemia in *homo sapiens.*

WO 2006/100439 A discloses phosphoramidates of cladribine, isocladribine, fludarabine and clofarabine and their use in treating cancer.

WO 2015/081133 A2 discloses nucleoside analogues linked to a 5'-D-amino acid phosphoramidate group useful for the treatment of liver cancer.

WO 2014/076490 A1 discloses a process for preparing enantiomers of phosphoramidates of nucleosides, provided in an enriched amount.

Expert Opin. Investig. Drugs (2011) 20(3):343-359 summarises recent discoveries in the properties and activities of purine and pyrimidine nucleoside analogues potentially active in lymphoid and myeloid malignancies.

8-Chloroadenosine (8-Cl-A) is a nucleoside analogue that has shown some cytotoxic activity in many cancers, including leukaemia. 8-Cl-A is currently under phase I clinical study for chronic lymphocytic leukaemia. 8-Cl-A is composed of an adenine base which has a chlorine group attached at position 8. 8-Cl-A is believed to exert cytotoxicity by causing cell cycle arrest at G₂/M phase, mediating catastrophic mitotic division, thereby inducing apoptosis and autophagy.

8-Cl-A uptake into the cells is mediated by nucleoside transporters such as equilibrative nucleoside transporters (ENT) type and concentrative nucleoside transporters (CNT) type. ENTs, including human ENT 1 (hENT1), are crucial for the drugs to be therapeutically effective. Once inside the cancer cells 8-Cl-A goes through a series of phosphorylation steps by adenosine kinases (AK) to form the active anti-cancer metabolite 8-Chloro-adenosine triphosphate, 8-Cl-ATP. Adenosine kinases (AK) phosphorylate 8-Cl-A by transferring γ-phosphate from ATP to 5'-hydroxyl of adenosine of 8-Cl-A, to produce 8-Chloro-adenosine monophosphate (8-Cl-AMP) and as a result 8-Cl-A becomes activated, ultimately generating 8-Cl-ATP. Therefore, as the 8-Cl-ATP accumulates, the intracellular ATP pool decreases. As a result the ratio of 8-Cl-ATP to ATP in the cell increases and favours the incorporation of 8-Cl-ATP into DNA as a false nucleotide, causing double-strand breaks in the DNA and preventing repair, ultimately leading to cell death through apoptosis.

The development of resistance mechanisms of cancer cells to 8-Cl-A can limit its effectiveness. There are several mechanisms proposed through which resistance occurs: limited cellular uptake (hENT1); poor activation (AK); extensive degradation by Adenosine Deaminase (ADA).

**hENT1.** Cancer cells may downregulate hENT1 or express a mutated form of the protein which may be less functional or non-functional. For example, single missense mutations can lead to the substitution of glycine to arginine at position 24 (G24) in the transmembrane 1 domain of hENT1, generating an aberrant protein, which cannot bind to nucleoside analogues. Therefore, the mutated hENT1 protein cannot mediate the uptake of 8-Cl-A across the cell membrane into the cancer cell.

AK. Cancer cells can develop mutations in the catalytic core of AK, preventing efficient 8-Cl-A binding and phosphorylation. Therefore 8-Cl-A remains largely in an inactive non-phosphorylated form in the cells and can no longer exert its cytotoxic effects. *In vitro* studies have shown that when AK is inhibited, cells become resistant to 8-Cl-A and no detectable 8-Cl-ATP is produced. Therefore, adenosine kinase is required to phosphorylate and activate 8-Cl-A.

**ADA.** Adenosine deaminase degrades 8-Cl-A in the plasma and intracellularly, thereby decreasing the drug levels and limiting the anti-cancer effects.

The putative existence of a cancer stem cell has been suggested in many human cancers including leukaemias and solid tumours. The cancer stem cell hypothesis considers that only a small sub-population of tumour cells is responsible for the formation and maintenance of the bulk of the tumour.

It is an object of certain embodiments of the present invention to provide a therapeutic agent that, in use, has therapeutic efficacy, particularly enhanced therapeutic efficacy in the prophylaxis or treatment of cancer.

It is an object of certain embodiments of the present invention to provide a means for delivering 8-Cl-ATP to cancer cells. It is an object of certain embodiments to provide a means of generating 8-Cl-ATP which overcomes, at least in part, the abovementioned resistance mechanisms to 8-Cl-A.

A further object of certain embodiments of the present invention is to provide a therapeutic agent having therapeutic efficacy against cancer stem cells.

Certain embodiments of the present invention solve some or all of the above stated objects.

### Summary of the Invention

According to a first aspect of the present invention there is provided the compound (2S)-benzyl 2-(((((2*R*,3*S*,4*R*,5*R*)-5-(6-amino-8-chloro-9*H*-purin-9-yl)-3,4-dihydroxy-tetrahydrofuran-2-yl)methoxy)(naphthalen-1-yloxy)phosphoryl)amino)propanoate (compound D from the examples below).

The compound may be (2*S*)-benzyl 2-(((((2*R*,3*S*,4*R*,S*R*)-5-(6-amino-8-chloro-9*H-*purin-9-yl)-3,4-dihydroxy-tetrahydrofuran-2-yl)methoxy)(naphthalen-1-yloxy)-(*Sp*)-phosphoryl)amino)propanoate in substantially diastereoisomerically pure form.

The compound may be (2*S*)-benzyl 2-(((((2*R*,3*S*,4R,5*R*)-5-(6-amino-8-chloro-9*H*-purin-9-yl)-3,4-dihydroxy-tetrahydrofuran-2-yl)methoxy)(naphthalen-1-yloxy)-(*Rp*)-phosphoryl)amino)propanoate in substantially diastereoisomerically pure form.

The compound may be isomer A of (2*S*)-benzyl 2-(((((2*R*,3*S*,4*R*,5*R*)-5-(6-amino-8-chloro-9*H-*purin-9-yl)-3,4-dihydroxy-tetrahydrofuran-2-yl)methoxy)(naphthalen-1-yloxy)phosphoryl)amino)propanoate in substantially diastereoisomerically pure form. Thus, the compound may be the isomer of (2*S*)-benzyl 2-(((((2*R*,3*S*,4*R*,5*R*)-5-(6-amino-8-chloro-9*H*-purin-9-yl)-3,4-dihydroxy-tetrahydrofuran-2-yl)methoxy)(naphthalen-1-yloxy)phosphoryl)amino)propanoate that has a ³¹P NMR peak at 3.93±0.04 when the NMR spectrum has been obtained on a 202 MHz NMR machine in CD₃OD, said isomer being in substantially diastereoisomerically pure form. The compound may be the isomer of (2*S*)-benzyl 2-(((((2*R*,3*S*,4*R*,5*R*)-5-(6-amino-8-chloro-9*H*-purin-9-yl)-3,4-dihydroxy-tetrahydrofuran-2-yl)methoxy)(naphthalen-1-yloxy)phosphoryl)amino)propanoate that has a retention time of 16.43 ± 0.10 minutes when analytical HPLC is performed on a Varian Pursuit XRs 5 C18, 150 x 4.6 mm eluting with H₂O/ CH₃CN from 100/10 to 0/100 in 30 min at 1 mL/min, , said isomer being in substantially diastereoisomerically pure form.

The compound may be isomer B of (2*S*)-Benzyl 2-(((((2*R*,3*S*,4*R*,5*R*)-5-(6-amino-8-chloro-9*H*-purin-9-yl)-3,4-dihydroxy-tetrahydrofuran-2-yl)methoxy)(naphthalen-1-yloxy) phosphoryl)amino)propanoate in substantially diastereoisomerically pure form. Thus, the compound may be the isomer of (2*S*)-Benzyl 2-(((((2*R*,3*S*,4*R*,5*R*)-5-(6-amino-8-chloro-9*H-*purin-9-yl)-3,4-dihydroxy-tetrahydrofuran-2-yl)methoxy)(naphthalen-1-yloxy)phosphoryl) amino)propanoate that has a ³¹P NMR peak at 3.83±0.04 when the NMR spectrum has been obtained on a 202 MHz NMR machine in CD₃OD, said isomer being in substantially diastereoisomerically pure form. The compound may be the isomer of (2*S*)-benzyl 2-(((((2*R*,3*S*,4*R*,5*R*)-5-(6-amino-8-chloro-9*H*-purin-9-yl)-3,4-dihydroxy-tetrahydrofuran-2-yl)methoxy)(naphthalen-1-yloxy)phosphoryl)amino)propanoate that has a retention time of 16.59 ± 0.10 minutes when analytical HPLC is performed on a Varian Pursuit XRs 5 C18, 150 x 4.6 mm eluting with H₂O/ CH₃CN from 100/10 to 0/100 in 30 min at 1 mL/min, said isomer being in substantially diastereoisomerically pure form.

Compounds in accordance with the said specific embodiments of the first aspect of present invention have surprisingly been shown to provide enhanced targeting of cancer stem cells, compared to 8-chloroadenosine.

According to a second aspect of the present invention, there is provided a compound of the present invention for use in a method of treatment.

In certain embodiments, there is provided a compound of the present invention for use in the prophylaxis or treatment of cancer.

According to an aspect of the present disclosure there is provided use of a compound of the present invention in the manufacture of a medicament for the prophylaxis or treatment of cancer.

According to a further aspect of the present disclosure, there is provided a method of prophylaxis or treatment of cancer comprising administration to a patient in need of such treatment an effective dose of a compound of the present invention.

The second aspect of the invention can comprise embodiments for treating cancer employed in combination with other cancer therapy. Examples of other cancer therapy include radiotherapy and/or other chemotherapy.

With respect to the second aspects of the present invention, embodiments of the invention comprise a cancer selected from among haematological and solid tumours. In particular, the cancer can be selected from the group consisting of leukaemia, multiple myeloma, lung cancer, liver cancer, breast cancer, head and neck cancer, neuroblastoma, thyroid carcinoma, skin cancer (including melanoma), oral squamous cell carcinoma, urinary bladder cancer, Leydig cell tumour, colon cancer, colorectal cancer and gynaecological cancers, including ovarian cancer, uterine cancer and cervical cancer, including epithelia cervix carcinoma. In preferred embodiments, the cancer is leukaemia and can be selected from the group consisting of acute lymphoblastic leukaemia, acute myelogenous leukaemia (also known as acute myeloid leukaemia or acute nonlymphocytic leukaemia), acute promyelocytic leukaemia, acute lymphocytic leukaemia, chronic myelogenous leukaemia (also known as chronic myeloid leukaemia, chronic myelocytic leukaemia or chronic granulocytic leukaemia), chronic lymphocytic leukaemia, monoblastic leukaemia and hairy cell leukaemia. In further preferred embodiments, the cancer is acute lymphoblastic leukaemia.

Compounds embodying the present invention have been found to have enhanced anti-cancer activity, compared to the 8-chloroadenosine, in treating solid tumours, as well as leukaemia. Examples of solid tumours that are suitable for treatment by compounds of the present invention include breast cancer, prostate cancer, lung cancer, colon cancer, cervical cancer and lymphomas. Examples of lymphomas suitable for treatment by compounds of the invention include Hodgkin Lymphoma and non-Hodgkin Lymphoma. Examples of leukaemia which are suitable for treatment by compounds of the present invention include myeloid leukaemia, multiple myeloma, chronic myelogenous leukaemia, acute myelogenous leukaemia and acute lymphocytic leukaemia.

It is believed that the compounds of the present invention enter into cancer cells and are incorporated into the cell's RNA and/or DNA.

Without being bound by any theory, it is believed that the efficacy, particularly the anti-cancer efficacy, exhibited by compounds of the present invention demonstrates that compounds of the present invention are phosphorylated to 8-chloroadenosine triphosphate and it is believed that enzymatic cleavage within the cell converts a compound of the invention directly into 8-chloroadenosine monophosphate prior to phosphorylation to the triphosphate.

The anti-cancer potency of the compounds of the present invention is believed additionally to be due to their cellular membrane permeability.

The invention provides a compound of the invention for use in targeting cancer stem cells.

The disclosure provides the use of a compound of the invention in the manufacture of a medicament for targeting cancer stem cells.

The disclosure provides a method of targeting cancer stem cells, the method comprising providing a population of cancer stem cells with an amount of a compound of the invention sufficient to target such cancer stem cells.

The targeting of cancer stem cells referred to in the present invention may be employed in the prevention or treatment of cancer. In such embodiments the population of cancer stem cells may be in a cancer or pre-cancerous condition in a patient in need of such targeting, and the method may comprise administering a therapeutically effective amount of a compound of the invention to the patient.

The invention provides a compound of the invention for use as an anti-cancer stem cell medicament. This use of a compound of the invention may also be employed in the prevention or treatment of cancer.

According to a third aspect of the invention, there is provided a pharmaceutical composition comprising a compound of the present invention, in combination with a pharmaceutically acceptable carrier, diluent or excipient.

In certain embodiments of the third aspect of the present invention, the pharmaceutical composition comprises a compound of the invention and a second anti-cancer compound, in combination with a pharmaceutically acceptable carrier, diluent or excipient.

Various aspects of the invention are based upon the finding that a compound of the invention is able to preferentially reduce cancer stem cell numbers. This finding is surprising in that cancer stem cells are known to be resistant to many chemotherapeutic agents, and there has previously been no suggestion that either a compound of the invention or 8-chloroadenosine, the parent prodrug compound from which a compound of the invention is derived, were able to target cancer stem cells. Thus the finding that a compound of the invention is able to target cancer stem cells and thus reduce their numbers, a finding which the inventors have confirmed is applicable across a broad range of cancers, represents a surprising breakthrough that enables a range of new therapeutic applications of a compound of the invention.

The biological activities exerted by the compounds of the invention indicate that they will be useful in the treatment of cancer. These activities may be useful in inhibiting the development or progression of cancers. In particular, the biological properties disclosed herein, which have not previously been reported, indicate that these compounds are able to provide treatment that is likely to be effective in patients with relapsed or refractory cancers. Treatment of this sort, using the compounds of the invention, may bring about a reduction in tumour size and/or a reduction in clinically relevant biomarkers, either of which may be associated with more favourable prognosis. Furthermore, treatment with a compound of the invention may help to maintain a reduction in the size of tumours in patients with relapsed or refractory cancer. Accordingly, treatment using a compound of the invention may achieve a high, durable Disease Control Rate (DCR) in patients with relapsed or refractory cancers.

Without wishing to be bound by any hypothesis, the inventors believe that the ability of the compounds of the invention to target cancer stem cells contributes to the therapeutic utility of these compounds in the treatment of relapsed or refractory cancer.

Except for where the context requires otherwise, references within this disclosure to a "use" of a compound of the invention in accordance with the invention may be taken as applying to any of the medical uses of compounds of the invention described herein.

The ability of a compound of the invention to target cancer stem cells provides new therapies directed against those cancer cells that are considered most difficult to treat, and that are considered to play a major role in the resistance that limits effectiveness of many existing cancer therapies. This ability also provides a way of targeting cells that are believed to be associated with the development, progression, recurrence, and propagation of cancers. Accordingly, it will be recognised that this anti-cancer stem cell activity of a compound of the invention yields benefits in contexts in which new and effective therapies have long been sought.

### Brief Description of the Drawings

Embodiments of the invention are further described hereinafter with reference to the accompanying drawings, in which:
Figure 1 compares the LD₅₀ values for 8-chloroadenosine and compounds D, B and E in cells of the acute myeloid leukaemia cell line KG1a. All data are the mean ± Standard Deviation (± SD) of three independent experiments.
Figure 2 shows, in Figure 2(i), the individual sigmoidal dose response curve for 8-chloroadenosine and phosphoramidate compounds D, B and E. In Figure 2(ii) the dose response curves for compounds B and E have been removed, to highlight the significant differences (p<0.05) between the responses to 8-chloroadenosine and compound D.
Figure 3 shows the intracellular concentration of 8-Cl-A (A), compound A (B), compound B (C), and compound D (D) in HL-60 leukaemic cells in the absence or presence of the nucleoside transporter inhibitor NBTI and the adenosine kinase inhibitor A-134974, mimicking drug resistance mechanisms.
Figure 4 shows the intracellular concentration of 8-Cl-A (A), compound A (B), compound B (C), and compound D (D) in CTS leukaemic cells in the absence or presence of inhibitors (NBTI, A-134974).
Figure 5 shows the intracellular concentration of 8-Cl-A (A), compound A (B), compound B (C), and compound D (D) in K-562 leukaemic cells in the absence or presence of inhibitors (NBTI, A-134974).
Figure 6 shows the stability of 8-Cl-A and compounds A, B and C in human plasma (µg/ml) in the absence or presence of the adenosine deaminase inhibitor EHNA.
Figure 7 shows individual sigmoidal dose response curves for Compound E, and Isomers (A and B) of Compounds B and D and Compound AC.
Figure 8 shows the results of a study investigating the ability of Compound E, and Isomers (A and B) of Compounds B and D, and Compound AC, to selectively target cancer stem cells.
Figure 9 shows HPLC traces for (2*S*)-Benzyl 2-(((((2*R*,3*S*,4*R*,5*R*)-5-(6-amino-8-chloro-9*H-*purin-9-yl)-3,4-dihydroxy-tetrahydrofuran-2-yl)methoxy)(naphthalen-1-yloxy)phosphoryl)amino)propanoate: A) both isomers; B) isomer A; and C) isomer B.
Figure 10 shows HPLC traces for benzyl 2-(((((2*R*,3*S*,4*R*,S*R*)-5-(6-amino-8-chloro-9*H*-purin-9-yl)-3,4-dihydroxytetra-hydrofuran-2-yl)methoxy)(phenoxy)phosphoryl)amino)acetate: A) both isomers; B) isomer A; and C) isomer B.

### Detailed description

As used herein, the term "stereoisomer" defines all possible compounds made up of the same atoms bonded by the same sequence of bonds but having different three-dimensional structures which the compounds of the present invention may possess.

Compounds according to this invention have at least one chiral centre and accordingly exist as enantiomers. Where the compounds possess two or more chiral centres, they may additionally exist as diastereoisomers. Where the processes for the preparation of the compounds according to the invention give rise to mixture of stereoisomers, these isomers may be separated by conventional techniques such as preparative chromatography. The compounds may be prepared in stereochemically mixed form or individual enantiomers may be prepared by standard techniques known to those skilled in the art, for example, by enantiospecific synthesis or resolution, formation of diastereoisomeric pairs by salt formation with an optically active acid, followed by fractional crystallization and regeneration of the free base. The compounds may also be resolved by formation of diastereoisomeric esters or amides, followed by chromatographic separation and removal of the chiral auxiliary. Alternatively, the compounds may be resolved using a chiral HPLC column. It is to be understood that all such isomers and mixtures thereof are encompassed within the scope of the present invention.

The phosphate centre is chiral in the compounds of the present invention and the compounds may exist as R_{P} and S_{P} diastereoisomers. The composition of the compound may be mixed Rp and Sp or one pure diastereoisomer. In a preferred embodiment, the compound is a either R_{P} or S_{P} in substantially diastereoisomerically pure form Throughout this specification, by "substantially diastereoisomerically pure form" is meant that the compound consists of 90% (e.g. 95% or more or 98% or more) or more of either the R_{P} or the S_{P} diastereoisomer. In another embodiment, there may be a mixture of 1:1 R_{P} to S_{P} diastereoisomers. Alternatively, the compound may comprise a mixture of R_{P} and S_{P} diastereoisomers in a ratio of R_{P} to S_{P} diastereoisomers of 1:90 to 90:1, 1:50 to 50:1, 1:20 to 20:1, 1:15 to 15:1, 1:10 to 10:1, 1:9 to 9:1, 1:8 to 8:1, 1:7 to 7:1, 1:6 to 6:1, 1:5 to 5:1, 1:4 to 4:1, 1:3 to 3:1 or 1:2 to 2:1. In preferred embodiments, the compound of the invention may comprise a ratio of R_{P} to S_{P} diastereoisomers of greater than 1:2, 1:3, 1:4, 1:5, 1:6, 1:7, 1:8, 1:9, 1:10, 1:15, 1:20, 1:50, 1:90, 1:95 or 1:99 or vice versa.

Compound D may be the R_{P} diastereoisomer. Compound D may consist of at least 98wt% of the R_{P} diastereoisomer. Compound D may be the S_{P} diastereoisomer. Compound D may consist of at least 98wt% of the S_{P} diastereoisomer.

The term "solvate" means a compound as defined herein, wherein molecules of a suitable solvent are incorporated in the crystal lattice. A suitable solvent is physiologically tolerable at the dosage administered. Examples of suitable solvents are ethanol, water and the like. When water is the solvent, the molecule is referred to as a hydrate.

The compounds of the present invention may also be present in the form of pharmaceutical acceptable salts. For use in medicine, the salts of the compounds of this invention refer to "pharmaceutically acceptable salts." FDA approved pharmaceutical acceptable salt forms (Ref. International J. Pharm. 1986, 33, 201-217; J. Pharm. Sci., 1977, Jan, 66 (1)) include pharmaceutically acceptable acidic/anionic or basic/cationic salts.

Pharmaceutically acceptable acidic/anionic salts include, and are not limited to acetate, benzenesulfonate, benzoate, bicarbonate, bitartrate, bromide, calcium edetate, camsylate, carbonate, chloride, citrate, dihydrochloride, edetate, edisylate, estolate, esylate, fumarate, glyceptate, gluconate, glutamate, glycollylarsanilate, hexylresorcinate, hydrabamine, hydrobromide, hydrochloride, hydroxynaphthoate, iodide, isethionate, lactate, maleate, mandelate, mesylate, methylbromide, methylnitrate, methylsulfate, mucate, napsylate, nitrate, pamoate, pantothenate, phosphate, diphospate, polygalacturonate, salicylate, stearate, subacetate, succinate, sulphate, tannate, tartrate, teoclate, tosylate and triethiodide.

Pharmaceutically acceptable basic/cationic salts include, and are not limited to aluminium, benzathine, calcium, chloroprocaine, choline, diethanolamine, ethylenediamine, lithium, magnesium, potassium, procaine, sodium and zinc.

The compound or pharmaceutical composition according to the present invention can be administered to a patient, which may be *homo sapiens* or other animal, by any suitable means.

The medicaments employed in the present invention can be administered by oral or parenteral routes, including intravenous, intramuscular, intraperitoneal, subcutaneous, transdermal, airway (aerosol), rectal, vaginal and topical (including buccal and sublingual) administration.

For oral administration, the compounds of the invention will generally be provided in the form of tablets or capsules, as a powder or granules, or as an aqueous solution or suspension.

Tablets for oral use may include the active ingredient mixed with pharmaceutically acceptable excipients such as inert diluents, disintegrating agents, binding agents, lubricating agents, sweetening agents, flavouring agents, colouring agents and preservatives. Suitable inert diluents include sodium and calcium carbonate, sodium and calcium phosphate, and lactose, while cornstarch and alginic acid are suitable disintegrating agents. Binding agents may include starch and gelatin, while the lubricating agent, if present, will generally be magnesium stearate, stearic acid or talc. If desired, the tablets may be coated with a material such as glyceryl monostearate or glyceryl distearate, to delay absorption in the gastrointestinal tract.

Capsules for oral use include hard gelatin capsules in which the active ingredient is mixed with a solid diluent, and soft gelatin capsules wherein the active ingredient is mixed with water or an oil such as peanut oil, liquid paraffin or olive oil.

Formulations for rectal administration may be presented as a suppository with a suitable base comprising for example cocoa butter or a salicylate.

Formulations suitable for vaginal administration may be presented as pessaries, tampons, creams, gels, pastes, foams or spray formulations containing in addition to the active ingredient such carriers as are known in the art to be appropriate.

For intramuscular, intraperitoneal, subcutaneous and intravenous use, the compounds of the invention will generally be provided in sterile aqueous solutions or suspensions, buffered to an appropriate pH and isotonicity. Suitable aqueous vehicles include Ringer's solution and isotonic sodium chloride. Aqueous suspensions according to the invention may include suspending agents such as cellulose derivatives, sodium alginate, polyvinyl-pyrrolidone and gum tragacanth, and a wetting agent such as lecithin. Suitable preservatives for aqueous suspensions include ethyl and n-propyl p-hydroxybenzoate.

The formulation (and in particular the formulation for intravenous administration) may comprise a polar aprotic organic solvent, e.g. dimethylsulfoxide (DMSO), N-methylpyrrolidinone (NMP) or N, N-dimethylacetamide (DMA). It may be that the formulation (e.g. the formulation for intravenous administration) comprises DMA.

The compounds of the invention may also be presented as liposome formulations.

In general a suitable dose will be in the range of 0.1 to 350 mg per kilogram body weight of recipient per day. A more suitable dose may be in the range of 0.5 mg to 150 mg per kilogram body weight of recipient per day, in the range of 0.5 to 100 mg per kilogram body weight of recipient per day, in the range of 1 to 50 mg per kilogram body weight of recipient per day, or in the range of 1 to 10 mg per kilogram body weight of recipient per day. A suitable a lower dose may be 0.5 mg per kilogram body weight of recipient per day or 1 mg per kilogram body weight of recipient per day. Alternatively, a suitable dose may be in the range of 1 to 100 mg per m² of body surface area of recipient per day or 5 to 50 mg per m² of body surface area of recipient per day. Suitable doses may be 6, 12, 24 or 48 mg per m² of body surface area of recipient per day. The desired dose may be presented and administered as a single daily dose or as two, three, four, five or six or more sub-doses administered at appropriate intervals throughout the day. Doses may be administered in unit dosage forms, for example, containing 10 to 1500 mg, preferably 20 to 1000 mg, and most preferably 50 to 700 mg of active ingredient per unit dosage form. The total daily dose is suitably 1000 to 3000 mg, whether taken as a single dose or as sub-doses at intervals throughout the day.

### Detailed Description of the Invention

### "Cancer stem cells"

Cancer stem cells, which are sometimes otherwise referred to as "tumour initiating cells", are well known to those skilled in the art. As used herein, the term "cancer stem cell" is to be interpreted in accordance with its widely accepted meaning, which is a cell that possesses the capacity to self-renew through asymmetric division, to initiate tumour formation, and to give rise to more mature non-stem cell cancer progeny by differentiation.

Cancer stem cells play a major role in the development, progression, recurrence and propagation of cancers. Accordingly, the finding that compounds of the invention are able to target cancer stem cells, and thereby reduce their numbers, offers therapeutic possibilities in preventing or treating these activities.

As discussed in more detail elsewhere in the specification, cancer stem cells are found in pre-cancerous conditions, where their presence is believed to contribute to the development of such conditions into cancers. Accordingly the medical uses of the invention, in which a compound of the invention is used to target cancer stem cells, may be used to reduce cancer stem cell numbers in pre-cancerous conditions (such as myelodyplastic syndrome, or other conditions considered elsewhere in the specification), and thus to prevent progression of such pre-cancerous conditions into cancer.

As referred to above, asymmetric cell division of cancer stem cells gives rise to differentiated non-stem cancer cells. Thus cancer stem cells are responsible for the formation and maintenance of the bulk of the tumour.

The accumulation of such non-stem cancer cells plays a major role in the progression of cancers. Targeting of cancer stem cells by a compound of the invention is able to reduce cancer stem cell numbers, which in turn reduces the number of non-stem cancer cell progeny. Thus medical uses of a compound of the invention in accordance with the present invention are of benefit in treating cancer by preventing cancer progression. Such embodiments are described in more details elsewhere in the present specification.

Cancer stem cells are also able to act as a reservoir of cancer cells that may cause the recurrence of cancer after remission. Even in the event that the majority of a patient's cancer cells have been removed (for example by surgery, radiotherapy, or chemotherapy, either alone or in combination), so that no observable signs of a cancer remain, the continued presence of cancer stem cells may nucleate the recurrence of the cancer over time. Targeting of cancer stem cells by a compound of the invention provides a new mode by which cancer stem cell numbers may be reduced and cancer stem cells killed. Accordingly, and as discussed in more detail elsewhere in the specification, in suitable embodiments the present invention provides medical uses in which a compound of the invention prevents or delays recurrence of cancer.

Furthermore, movement of cancer stem cells from the site of a cancer to another location within the body can contribute to propagation of cancer, for example by giving rise to metastases. Consequently, the ability of a compound of the invention to target cancer stem cells therefore provides new medical uses in preventing or treating cancer propagation.

In addition to their biological activities, cancer stem cells may be identified by their expression of certain characteristic cell surface markers. Cancer stem cells identified in haematological malignancies are typically CD34⁺, while in solid tumours, CD44⁺, CD133⁺ and CD90⁺ have been identified as cancer stem cell markers. The following table summarises examples of known cancer stem cell surface phenotypes. It is expected that each of these forms of cancer stem cell can be targeted using a compound of the invention in accordance with the invention, and so uses employing a compound of the invention may be used in the prevention or treatment of cancers associated with cancer stem cells expressing any of these sets of markers.

| **Tumour type** | **Reported cell surface markers for cancer stem cells** |
|---|---|
| *Solid Tumours* | |
| Breast | CD44⁺/CD24⁻/^{low} /Lineage⁻/ESA⁺ |
| CNS | CD133⁺ |
| Colon | CD133⁺ |
| Colon | ESA^{high}/CD44⁺/Lineage⁻ /(CD 166⁺) |
| Ewing's | CD133⁺ |
| Head and Neck | CD44⁺/Lineage⁻ |
| Melanoma | ABCB5⁺ |
| Liver | CD90⁺/CD45⁻/(CD44⁺) |
| Cholangiocarinoma | CD44⁺/GLII⁺ (Glioma-associated oncogene homolog-1) |
| Ovarian | CD44⁺/CD117⁺ |
| Pancreas | CD44⁺/CD24⁺/ESA⁺ |
| Pancreas | CD133⁺ |
| Non-small-cell lung cancer | CD44⁺/Ber-EP4⁺ |
| Bladder cancer | CD44⁺/ALDH1A1⁺ |
| | |

| *Haematological tumours* | |
|---|---|
| Acute myeloid leukaemia | Lin⁻/CD34⁺/CD38⁻/CD123⁺ |
| B-Acute lymphoblastic leukaemia | CD34⁺/CD10⁻ or CD34⁺/CD19⁻ |
| B-Acute lymphoblastic leukaemia | CD34⁺/CD38⁻/CD19⁺ |
| Multiple myeloma | CD34⁻/CD138⁻ |
| T-Acute lymphoblastic leukaemia | CD34⁺/CD4⁻ or CD34⁺/CD7⁻ |

The data presented in the Examples demonstrate that a compound of the invention is able to target cancer stem cells of leukaemic stem cell lines, specifically cancer stem cells present in the acute myeloid leukaemia cell line KG1a. This cell line manifests a minor stem cell-like compartment with a distinct immunophenotype (Lin⁻/CD34⁺/CD38⁻/CD123⁺) which is targeted by a compound of the invention. Accordingly, medical uses of a compound of the invention in accordance with the present invention may be used to prevent or treat leukaemia or other cancers associated with cancer stem cells expressing these characteristic markers.

The present invention also provides medical uses in which patients are selected for prevention or treatment of cancer, utilising a compound of the invention, on the basis of the identification of the presence of cancer stem cells in a biological sample representative of the patient's cancer or pre-cancerous condition. The markers set out above provide suitable examples that can be used to identify the presence of cancer stem cells in accordance with such embodiments of the invention. Suitable techniques by which expression of these markers may be investigated in a biological sample are considered further elsewhere in this specification.

### "Targeting of cancer stem cells"

The present invention provides the first indication that compounds of the invention can be used for targeting cancer stem cells. The ability of compounds of the invention to target cancer stem cells is illustrated in the Examples disclosed in this specification.

It can be seen from the Examples that when a compound of the invention is provided to populations of cancer cells containing cancer stem cells it targets the cancer stem cells present, leading to a reduction in the total number of cancer cells and in the proportion of total cancer cells exhibiting phenotypic markers of cancer stem cells.

While the parent prodrug compound 8-chloroadenosine is able to target cancer stem cells at certain, higher, concentrations, the compounds of the invention demonstrate the ability to achieve such targeting across a broader range of concentrations. Notably, *in vitro* studies, the results of which are reported in the present application, demonstrate that the compounds of the invention are able to target cancer stem cells at low concentrations more effectively than 8-chloroadenosine. At concentrations in the region of 1 x 10⁻⁶ to 5 x 10⁻⁶, the improvement in cancer stem cell targeting is such that the proportion of cancer stem cells remaining in a population of cells treated with a compound of the invention is significantly lower than for a population of cells treated with 8-chloroadenosine. It will be appreciated that the ability to achieve effective targeting of cancer stem cells at low concentrations of cytotoxic agents will generally be desirable since this reduces the likelihood of unwanted side effects.

It is believed that the compounds of the present invention enter into cancer cells and are incorporated into the nucleic acids (RNA and/or DNA). Without being bound by any theory, it is believed that the efficacy, particularly the anti-cancer efficacy, exhibited by compounds of the present invention demonstrates that compounds of the present invention are phosphorylated to 8-chloroadenosine triphosphate and it is believed that enzymatic cleavage within the cell converts a compound of the invention directly into 8-chloroadenosine monophosphate prior to phosphorylation to the triphosphate.

It is also believed that the compounds of the invention possess enhanced cellular membrane permeability (as compared to 8-chloroadenosine), and that this contributes to the enhanced anti-cancer potency of the compounds of the invention compared to the parent from which they are derived.

Without wishing to be bound by any hypothesis, the inventors believe that the reduction in cancer stem cell numbers arises as a result of targeted killing of the cancer stem cells among the cancer cell population. That is to say, that compounds of the invention appear to kill cancer stem cells preferentially as compared to killing of non-stem cancer cells, thereby causing the death of cancer stem cells, and a reduction of the proportion of cancer stem cells among the total cancer cell population.

While the inventors believe that compounds of the invention preferentially kill cancer stem cells as compared to non-stem cancer cells, other mechanisms may also contribute to the reduction in the proportion of cancer stem cells caused by a compound of the invention's targeting of these cells.

Merely by way of example, treatment with a compound of the invention may cause an increase in cancer stem cell differentiation, thereby reducing cancer stem cell numbers and also the proportion of total cancer cells represented by cancer stem cells. Alternatively, a compound of the invention may cause cancer stem cells to lose their stem cell phenotype, for example losing their ability to self-renew, thereby reducing cancer stem cell numbers.

References to targeting of cancer stem cells in the present disclosure should be interpreted accordingly. For the purposes of the present disclosure, "targeting" of cancer stem cells may be taken as encompassing any mechanism by which a compound of the invention reduces the proportion of cancer stem cells present in a population of cells, whether *in vitro* or *in vivo.* In particular targeting of cancer stem cells may be taken as encompassing preferential killing of cancer stem cells as compared to other cell types, particularly as compared to non-stem cancer cells.

### "Prevention or treatment of cancer"

The invention provides medical uses in which a compound of the invention is used for the prevention or treatment of cancer. In the context of the present invention, "prevention" of cancer is to be considered as relating to prophylactic applications of a compound of the invention used before the development of cancer, and with an aim of stopping cancer from developing. On the other hand "treatment" of cancer is taken as concerning the use of a compound of the invention after cancer has occurred, with a view to ameliorating cancer by slowing or stopping cancer cell proliferation and tumour growth. Advantageously treatment of cancer may cause partial or total reduction in cancer cell numbers and tumour size. Effective treatment of cancer may bring about disease that either "stabilizes" or "responds" in accordance with the RECIST (Response Evaluation Criteria In Solid Tumours) rules.

As described in more detail below, prevention of cancer in accordance with the present invention may be of particular benefit in patients who have a pre-cancerous condition that increases their likelihood of developing cancer.

### "Prevention of cancer"

Prevention of cancer in accordance with the present invention may be effected by treatment of a pre-cancerous condition using a compound of the invention in accordance with the various aspects or embodiments of the invention described herein.

In particular, prevention of cancer, in the context of the present invention, may be achieved by the medical uses of the invention in which a compound of the invention is provided to a patient with a pre-cancerous condition. Medical uses in accordance with this embodiment may prevent development of the treated pre-cancerous condition into cancer, thereby providing effective prevention of cancer.

References to prevention of cancer in the context of the present invention may also encompass other prophylactic applications of a compound of the invention. For example, the ability of a compound of the invention to target cancer stem cells and thereby prevent the development of cancer, and/or prevent the progression of cancer, and/or prevent the recurrence of cancer, and/or prevent the propagation of cancer.

### "Pre-cancerous conditions"

Cancer is frequently preceded by the development of a pre-cancerous condition, which is not itself cancerous, but is associated with an increased risk of cancer. Accumulation of genetic or epigenetic changes may cause previously normal cells to develop a cancer stem cell phenotype. Accordingly, cancer stem cells may also be present in such pre-cancerous conditions, as well as in cancerous conditions.

It is believed that the presence of cancer stem cells in pre-cancerous conditions contributes to the development of these conditions into cancer. The medical uses of the invention may be employed to target cancer stem cells present in pre-cancerous conditions, and thereby treat such conditions. It will be appreciated that the new and unexpected finding that compounds of the invention target cancer stem cells means that treatment of pre-cancerous conditions with such compounds may be used to prevent the treated conditions developing into cancer. This represents a way in which a compound of the invention can be used medically in the prevention of cancer, as considered elsewhere in this specification.

Examples of pre-cancerous conditions that may be treated in accordance with the present invention include, but are not limited to, those selected from the group consisting of: actinic keratosis, Barrett's oesophagus, atrophic gastritis, dyskeratosis congenital, Sideropenic dysphagia, Lichen planus, oral submucous fibrosis, solar elastosis, cervical dysplasia, leukoplakia, erythroplakia, monoclonal gammopathy of unknown significance (MGUS), monoclonal B-cell lymphocytosis (MBL), myelodysplastic syndromes, as well as pre-cancerous conditions of the stomach such as atrophic gastritis, gastric ulcer, pernicious anaemia, gastric stumps, gastric polyps, and Ménétrier's disease. Among the listed pre-cancerous conditions of the stomach, atrophic gastritis, pernicious anaemia, gastric stumps, and certain types of gastric polyp may have particularly heightened risk of developing into cancers.

Pre-cancerous conditions often take the form of lesions comprising dysplastic or hyperplastic cells. Accordingly, the presence of dysplasia or hyperplasia, as an alternative or addition to the presence of cells with expressed markers or phenotypes characteristic of cancer stem cells, may be used in the identification of pre-cancerous conditions.

The severity of dysplasia can vary between different pre-cancerous conditions, or with the development of a single pre-cancerous condition over time. Generally, the more advanced dysplasia associated with a pre-cancerous condition is, the more likely it is that the pre-cancerous condition will to develop into cancer. Dysplasia is typically classified as mild, moderate or severe. Severe dysplasia usually develops into cancer if left untreated. Suitably, medical uses employing a compound of the invention may therefore be used to treat a patient with a pre-cancerous condition associated with severe dysplasia.

In a suitable embodiment of the invention a compound of the invention is used to treat a patient with severe cervical dysplasia. Severe cervical dysplasia may be diagnosed by means of a smear test. In another embodiment of the invention a compound of the invention is used to treat severe oesophageal dysplasia ("Barrett's oesophagus"). Severe oesophageal dysplasia may be diagnosed following a tissue biopsy.

It has recently been reported that pre-malignancies can also be identified by detecting somatic mutations in cells in individuals not known to have cancer. In particular, it has been reported that age-related clonal haematopoiesis is a common pre-malignant condition that is associated with increased overall mortality and increased risk of cardiometabolic disease. The majority of mutations detected in blood cells occurred in three genes: DNMT3A, TET2, and ASXL1. Accordingly, patients that will benefit from the use of a compound of the invention to target cancer stem cells, and thereby treat a pre-cancerous condition, may be identified by assaying a sample comprising blood cells for the presence of genetic mutations indicative of a pre-cancerous condition in at least one of the genes: DNMT3A and/or TET2 and/or ASXL1.

Pre-cancerous conditions that may benefit from treatment with a compound of the invention in accordance with the invention to target cancer stem cells may also be identified by determination of the presence of cancer stem cells with reference to any of the techniques based upon expression of markers characteristic of cancer stem cells, or cancer stem cell phenotypes, discussed elsewhere in the specification.

### "Treatment of cancer"

The skilled person will appreciate that there are many measurements by which "treatment" of cancer may be assessed. Merely by way of example, any reduction or prevention of cancer development, cancer progression, cancer recurrence, or cancer propagation may be considered to indicate effective treatment of cancer.

In certain embodiments, a compound of the invention may be used: to reduce the proportion of cancer stem cells in a population of cancer cells; and/or to inhibit tumour growth; and/or to reduce tumourigenicity; and/or to prevent or treat a primary cancer; and/or to prevent or treat a relapsed cancer; and/or to prevent or treat a metastatic or secondary cancer; and/or to treat, prevent or inhibit metastasis or recurrence; and/or to treat or prevent refractory cancer.

The ability of cancer treatment using a compound of the invention to bring about a reduction in tumour size, and also to maintain the reduction in tumour size during/after the period in which the treatment is administered represents a particularly relevant indication of effective cancer treatment. As set out in the Examples, the treatments or medical uses of the invention have proven surprisingly effective in this respect, even in models using cells representative of relapsed or refractory cancers that have previously been resistant to treatment with other therapies.

The data presented in the Examples illustrate that treatment with a compound of the invention reduces the proportion of cancer stem cells in a population of cancer cells. Characteristic biological activities or cell surface markers by which cancer stem cells may be identified are described elsewhere in the specification. In a suitable embodiment, treatment of cancer in accordance with the present invention may give rise to a reduction in the proportion of cancer stem cells present in a patient's cancer of at least 10%, at least 20%, at least 30%, or at least 40%. In suitable embodiments, treatment of cancer in accordance with the present invention may give rise to a reduction in the proportion of cancer stem cells present in a patient's cancer of at least 50%, at least 60%, at least 70%, or at least 80%. Treatment of cancer in accordance with the invention may give rise to a reduction in the proportion of cancer stem cells present in a patient's cancer of at least 85%, at least 90%, or at least 95%. Indeed, treatment of cancer in accordance with the present invention may give rise to a reduction in the proportion of cancer stem cells present in a patient's cancer of at least 96%, at least 97%, at least 98%, at least 99%, or even 100% (such that substantially no detectable cancer stem cells remain).

Asymmetric division of cancer stem cells contributes to the growth of tumours. Treatment of cancer with a compound of the invention in accordance with the present invention may bring about an inhibition of tumour growth of at least 10%, at least 20%, at least 30%, or at least 40%. Suitably treatment of cancer in accordance with the invention may give rise to an inhibition of tumour growth of at least 50%, at least 60%, at least 70%, or at least 80%. Treatment of cancer in accordance with the invention may give rise to an inhibition of tumour growth of at least 85%, at least 90%, or at least 95% in a patient so treated. Indeed, treatment of cancer in accordance with the invention may give rise to an inhibition of tumour growth of at least 96%, at least 97%, at least 98%, at least 99%, or even 100% in a treated cancer.

Tumour growth may be assessed by any suitable method in which the change in size of a tumour is assessed over time. Suitably, the size of a tumour prior to cancer treatment may be compared with the size of the same tumour during or after cancer treatment. A number of ways in which the size of a tumour may be assessed are known. For example, the size of a tumour may be assessed by imaging of the tumour *in situ* within a patient. Suitable techniques, such as imaging techniques, may allow the size of a tumour to be determined, and changes in tumour size to be assessed.

As shown in the results set out in the Examples of this specification, the medical uses of a compound of the invention are able not only to arrest tumour growth, but are actually able to bring about a reduction in tumour size in patients with cancers, including patients with relapsed or refractory cancers. Suitably, treatment of cancer in accordance with the present invention may give rise to a reduction in tumour size of at least 10%, at least 20%, at least 30%, or at least 40%. In suitable embodiments, treatment of cancer in accordance with the present invention may give rise to a reduction in tumour size of at least 50%, at least 60%, at least 70%, or at least 80%. Treatment of cancer in accordance with the invention may give rise to a reduction in tumour size of at least 85%, at least 90%, or at least 95%. Indeed, treatment of cancer in accordance with the present invention may give rise to a reduction in tumour size of at least 96%, at least 97%, at least 98%, at least 99%, or even 100%.

A reduction in tumour size of the sort described above can be calculated with reference to a suitable control. For example, in studies carried out *in vitro* or *in vivo* in suitable animal models, the reduction in tumour size may be determined by direct comparison between the size of a tumour treated with a compound of the invention and the size of a control tumour (which may be untreated, or may have received treatment other than with a compound of the invention). It will be appreciated that such models requiring lack of treatment of a tumour may not be ethically acceptable in the context of clinical studies or therapeutic management of patients, and in this case a reduction in tumour size may be assessed by comparing the size of a treated tumour with the size of the same tumour prior to treatment, or with a predicted size that would have been attained by the tumour had no treatment been administered.

The medical uses of a compound of the invention may bring about a reduction in biomarkers indicative of cancer. The reduction of such biomarkers provides a further assessment by which effective treatment of cancer may be demonstrated. Suitable examples of such biomarkers may be selected on the basis of the type of cancer to be treated. For example, in the case of gynaecological cancers CA125 represents a suitable biomarker, while in the case of pancreatic or biliary cancers CA19.9 represents a suitable biomarker, and in the case of colorectal cancers CEA may be a suitable biomarker.

Suitably, treatment of cancer in accordance with the present invention may give rise to a reduction in the expression levels of cancer biomarkers by at least 10%, at least 20%, at least 30%, or at least 40%. In suitable embodiments, treatment of cancer in accordance with the invention may give rise to a reduction in the expression levels of cancer biomarkers by at least 50%, at least 60%, at least 70%, or at least 80%. Treatment of cancer in accordance with the invention may give rise to a reduction in the expression levels of cancer biomarkers by at least 85%, at least 90%, or at least 95%. Indeed, treatment of cancer in accordance with the invention may give rise to a reduction in the expression levels of cancer biomarkers by at least 96%, at least 97%, at least 98%, at least 99%, or even 100%.

Beneficial effects, such as a reduction in the proportion of cancer stem cells present, reduction in tumour growth, or reduction in tumour size or the expression levels of cancer biomarkers, observed on treatment of cancer in accordance with the present invention may be maintained for at least one month. Suitably, such beneficial effects may be maintained for at least two months, at least three months, at least four months, at least five months, or at least six months. Indeed, such beneficial effects may be maintained for at least 12 months, at least 18 months, or at least 24 months. Suitably, the beneficial effects may be maintained for at least three years, at least four years, at least five years, at least six years, at least seven years, at least eight years, at least nine years, or for ten years or more.

In a suitable embodiment the invention provides the use of a compound of the invention in a method of preventing or treating cancer or a pre-malignant condition, wherein the method reduces the tumourigenicity of one or more cancer stem cells. Suitably, such methods may prevent the progression of cancer, or inhibit tumour growth.

When a compound of the invention is used in methods or medical uses of the present invention to prevent or treat the progression of a cancer, such prevention or treatment may cause the cancer progression to be slowed, delayed or stopped entirely.

The progress of a cancer is typically determined by assigning a stage to the cancer. Staging is usually carried out by assigning a number from I to IV to the cancer, with I being an isolated cancer and IV being a cancer that has spread to the limit of what the assessment measures. Specifics of staging vary between cancers, but the stage generally takes into account the size of a tumour, whether it has invaded adjacent organs, how many regional (nearby) lymph nodes it has spread to (if any), and whether it has appeared in more distant locations (metastasised).

Generally, Stage I is localised to one part of the body and may be treated by surgical resection (for solid tumours that are small enough). Stage II is locally advanced, and is treatable by chemotherapy, radiation therapy, surgery, or a combination thereof. Stage III is also locally advanced and the designation of Stage II or Stage III depends on the specific type of cancer, although Stage III is generally accepted to be "late" locally advanced. Stage IV cancers have often metastasised to a second organ. Treatment of cancer using a compound of the invention in the methods or medical uses of the present invention may be used to treat a stage I, II, III or IV cancer by targeting cancer stem cells. Treatment with a compound of the invention may be used to prevent the progression of a cancer from one stage to the next. In one embodiment, treatment with a compound of the invention is used to prevent progression from Stage I to Stage II. In another embodiment, treatment with a compound of the invention is used to prevent progression from Stage II to Stage III. In still another embodiment, treatment with a compound of the invention is used to prevent progression from Stage III to Stage IV.

Preventing or inhibiting progression of the cancer is particularly important for preventing the spread of the cancer, for example the progression from Stage I to Stage II where the cancer spreads locally, or the progression from Stage III to Stage IV where the cancer metastasises to other organs. Cancer stem cells are tumourigenic and so are believed to play a critical role in the spread of cancer, both locally and metastatically. Methods of treatment or medical uses of the invention employing a compound of the invention can therefore be used to prevent the spread of cancer, by targeting tumourigenic cancer stem cells and thus reducing their numbers.

### "Cancers"

Compounds of the invention demonstrate increased anti-cancer activity as compared to 8-chloroadenosine from which they are derived. This increased anti-cancer activity appears to be provided as a result of increased activity against both cancer stem cells and non-stem cancer cells.

Cancer stem cells play a role in the biological activity of a wide range of cancers. Accordingly, there are a wide range of cancers that may be prevented or treated in accordance with the present invention.

As discussed elsewhere herein, cancer stem cells are known to be present in many tumour types including liquid tumours (including haematological tumours such as leukaemias and lymphomas) and solid tumours (such as breast, lung, colon, prostate, ovarian, skin, bladder, biliary and pancreas tumours). Methods of treatment and medical uses of a compound of the invention to target cancer stem cells are therefore expected to be useful in the prevention or treatment of such cancers.

Suitably, a compound of the invention may be used in the prevention or treatment of a cancer selected from the group consisting of: leukaemia, lymphoma, multiple myeloma, lung cancer, liver cancer, breast cancer, head and neck cancer, neuroblastoma, thyroid carcinoma, skin cancer (including melanoma), oral squamous cell carcinoma, urinary bladder cancer, Leydig cell tumour, biliary cancer, such as cholangiocarcinoma or bile duct cancer, pancreatic cancer, colon cancer, colorectal cancer and gynaecological cancers, including ovarian cancer, endometrial cancer, fallopian tube cancer, uterine cancer and cervical cancer, including epithelia cervix carcinoma. In suitable embodiments, the cancer is leukaemia and can be selected from the group consisting of acute lymphoblastic leukaemia, acute myelogenous leukaemia (also known as acute myeloid leukaemia or acute non-lymphocytic leukaemia), acute promyelocytic leukaemia, acute lymphocytic leukaemia, chronic myelogenous leukaemia (also known as chronic myeloid leukaemia, chronic myelocytic leukaemia or chronic granulocytic leukaemia), chronic lymphocytic leukaemia, monoblastic leukaemia and hairy cell leukaemia. In further preferred embodiments, the cancer is acute lymphoblastic leukaemia. In a suitable embodiment the cancer is lymphoma, which may be selected from the group consisting of: Hodgkin's lymphoma; non-Hodgkin lymphoma; Burkitt's lymphoma; and small lymphocytic lymphoma.

Suitably, targeting cancer stem cells in such cancers may achieve effective treatment of the cancer by preventing or treating the development of the cancer, by preventing or treating the progression of the cancer, by preventing or treating the recurrence of the cancer, or by preventing or treating the propagation of the cancer.

In a suitable embodiment the present invention provides a compound of the invention for use in targeting cancer stem cells in the prevention or treatment of metastatic cancer.

In a suitable embodiment the present invention provides a compound of the invention for use in targeting cancer stem cells in the treatment of relapsed or refractory cancer.

In a suitable embodiment the present invention provides a compound of the invention for use in targeting cancer stem cells in the treatment of a primary cancer. Suitably, the primary cancer treated may be a second primary cancer.

The invention provides a compound of the invention for use in targeting cancer stem cells in the treatment of secondary cancer. In a suitable embodiment the secondary cancer is a metastatic cancer.

In a suitable embodiment the present invention provides a compound of the invention for use in targeting cancer stem cells, wherein the targeting of cancer stem cells prevents or inhibits: (i) recurrence of a cancer; (ii) occurrence of second primary cancer; or (iii) metastasis of a cancer.

Medical uses in which a compound of the invention is employed on the basis of its ability to target cancer stem cells may be used in the treatment of relapsed or refractory cancer. The considerations regarding relapsed or refractory cancer in such embodiments are, except for where the context requires otherwise, the same as for the treatment of relapsed or refractory cancer in connection with the other aspects of the invention.

### "Relapsed or refractory cancer"

As noted above, certain aspects and embodiments of the invention particularly relate to the use of a compound of the invention in the treatment of relapsed or refractory cancers.

For the purposes of the present invention, refractory cancers may be taken as cancers that demonstrate resistance to treatment by anti-cancer therapies other than those utilising a compound of the invention. For example, a compound of the invention may be used in the treatment of refractory cancers that are resistant to treatment with radiotherapy. Alternatively, or additionally, a compound of the invention may be used in the treatment of refractory cancers that are resistant to biological agents used in the treatment of cancer. In a suitable embodiment a compound of the invention may be used in the treatment of refractory cancers that are resistant to treatment with chemotherapeutic agents other than a compound of the invention.

In particular, refractory cancers that may benefit from the medical uses of the invention employing a compound of the invention include those cancers that are resistant to 8-chloroadenosine.

Relapsed cancers (or recurrent cancers) are those that return after a period of remission during which the cancer cannot be detected. Cancer recurrence may occur at the site of the original cancer (local cancer recurrence), at a site close to that of the original cancer (regional cancer recurrence), or at a site distant from that of the original cancer (distal cancer recurrence). Cancer stem cells are believed to play a role in the recurrence of cancer, providing a source from which cells of the relapsed cancer are generated. Accordingly, the medical uses of a compound of the invention in accordance with the invention, which enable targeting of cancer stem cells, may be of great benefit in the context of relapsed cancers. The ability of a compound of the invention to target cancer stem cells may be used to reduce, or even remove, the populations of such cells that are able to give rise to recurrence, thus preventing incidences of relapsed cancer. The anti-cancer stem cell activity of a compound of the invention may also be used to target cancer stem cells in cancers that have recurred, as well as potentially exerting cytotoxic effects on non-stem cancer cells, thereby providing treatment of relapsed cancers.

In view of the above, it will be appreciated that a compound of the invention may be used in the uses of the invention for the prevention or treatment of a relapsed cancer. A compound of the invention may be used in the uses of the invention for the prevention or treatment of a local, regional or distant relapsed cancer.

A compound of the invention may be used in the uses of the invention to prevent the recurrence of cancer by providing at least 2 months, at least 6 months, at least 12 months, at least 18 months, at least 24 months, or at least 30 months of remission. Indeed, a compound of the invention may be used to prevent recurrence of cancer by providing at least 4 years, at least 5 years, at least 6 years, at least 7 years, at least 8 years, at least 9 years, or at least 10 years of remission.

A compound of the invention may be used in the uses of the invention to treat a relapsed cancer which has recurred after at least 2 months, at least 6 months, at least 12 months, at least 18 months, at least 24 months, or at least 30 months of remission. Indeed, a compound of the invention may be used to treat a relapsed cancer which has recurred after at least 4 years, at least 5 years, at least 6 years, at least 7 years, at least 8 years, at least 9 years, or at least 10 years of remission.

The ability of the compounds of the invention to target cancer stem cells gives rise to the ability of these compounds to prevent or treat cancers in accordance with the medical uses of the invention. However, it should be noted that compounds of the invention also exert a direct cytotoxic effect upon non-stem cancer cells that make up the bulk of tumours. While cancer stem cells may underlie much of the difficulty to treat relapsed or refractory cancers, non-stem cancer cells are also a major constituent of such relapsed or refractory cancers.

Compounds of the invention exert greater cytotoxic effects on non-stem cancer cells than does 8-chloroadenosine, the chemotherapeutic molecule from which the compounds of the invention are derived. Accordingly, the mechanism by which a compound of the invention acts in the treatment of relapsed or refractory cancer may not be limited solely to the anti-cancer stem cell activity of this compound, but may also make use of the action of a compound of the invention on non-stem cancer cells. In such uses treatment with a compound of the invention will reduce the total number of both cancer stem cells and non-stem cancer cells, but will preferentially reduce the proportion of cancer stem cells that remain after treatment.

### Therapeutically effective doses of a compound of the invention

A therapeutically effective amount of a compound of the invention may be an amount sufficient to induce death of cancer cells. A therapeutically effective amount of a compound of the invention may be an amount sufficient to induce death of cancer stem cells. In some embodiments, particularly those relating to the treatment of relapsed or refractory cancer, a therapeutically effective amount of a compound of the invention may be an amount sufficient to induce death of cancer stem cells and also to induce death of non-stem cancer cells.

There are various different ways in which the amount of a therapeutically effective compound, such as a compound of the invention, to be administered to a patient may be calculated and expressed. One such way which is considered particularly relevant in doses of agents for the prevention or treatment of cancer, is in the amount of the agent to be administered per unit of body surface area of the patient. Such doses are typically expressed in terms of the amount of the agent (which may be determined by mass) per square meter (m²) of surface area.

Uses of a compound of the invention for the prevention or treatment of cancer may utilise a weekly dose of between 250 mg/m² and 1000 mg/m². Such treatments may, for example utilise a weekly dose of between 375 mg/m² and 900 mg/m². For example, effective treatment of relapsed or refractory cancers may be provided when patients are provided with weekly doses of a compound of the invention that range between approximately 500 mg/m² and 825 mg/m².

Without wishing to be bound by any hypothesis, the inventors believe that the ability of a compound of the invention to target cancer stem cells allows therapeutic effectiveness to be achieved using lower doses of this compound than would otherwise be expected. Merely by way of example, weekly doses of a compound of the invention that are as low as 825 mg/m², 750 mg/m², 600 mg/m², or 500 mg/m² may prove therapeutically effective in the uses of the invention.

A chosen weekly dose of a compound of the invention may be provided in a single incidence of administration, or in multiple incidences of administration during a week. For example, a weekly dose of a compound of the invention may be provided in two incidences of administration, in three incidences of administration, or more. Thus, in the case of a weekly dose of 750 mg/m², this may be achieved by three administrations of 250 mg/m² over the course of a week, or two administrations of 375 mg/m² during a week. Similarly, in the case of a weekly dose of 600 mg/m², this may be achieved by three administrations of 200 mg/m² over the course of a week, or two administrations of 300 mg/m² during a week.

A suitable amount of a compound of the invention to be administered in a single incidence of treatment in order to provide a required dose of this compound over the course of week may be between approximately 100 mg/m² and 300 mg/m².

The weekly dose of a compound of the invention provided may decrease over the course of treatment. For example, treatment may be started at a weekly dose of around 1000 mg/m², 900 mg/m², 825 mg/m², 750 mg/m², or 725 mg/m², and over the course of treatment the dose needed may decrease to around 750 mg/m² (in cases where the initial dose is above this amount), around 650 mg/m², around 625 mg/m², or even around 500 mg/m² or around 375 mg/m².

Doses of a compound of the invention can, of course, be presented in other manners. The most common of these is the amount of the active agent to be provided per unit body mass. It has been calculated that for an average human patient a dose of 1 mg/m² is equivalent to approximately 0.025 mg/kg body mass. Accordingly, the data indicate that a compound of the invention is effective for the treatment of relapsed or refractory cancer at doses ranging from approximately 6.25 mg/kg to approximately 25 mg/kg. A suitable dose may, for example, be of between about 9.5 mg/kg and 22.5 mg/kg. In a suitable embodiment a compound of the invention achieves effective treatment of relapsed or refractory cancers when patients are provided with weekly doses ranging between approximately 12.5 mg/kg and 20.5 mg/kg.

Considerations regarding formulations of a compound of the invention suitable for use in the medical uses of the present invention are described elsewhere in this disclosure. In the case of injectable formulations of a compound of the invention, these may be administered intravenously. Intravenous administration may be achieved over any suitable time frame, for example in a ten minute injection, or the like.

### Types of treatment

In a suitable embodiment a compound of the invention may be used for targeting cancer stem cells as a first line treatment of cancer.

However, the finding that compounds of the invention are able to target cancer stem cells and thereby treat relapsed or refractory cancer illustrates that a compound of the invention is able to provide effective treatment of cancer in contexts in which other treatments have proved ineffective. Accordingly, in a suitable embodiment the present invention provides a compound of the invention for targeting cancer stem cells as a second line treatment of cancer. Indeed, in a suitable embodiment the present invention provides a compound of the invention for targeting cancer stem cells as a third, or further, line treatment of cancer.

In a suitable embodiment there is provided a compound of the invention for use as a neoadjuvant in the treatment of cancer. A neoadjuvant is an agent provided to a patient in order to reduce the size of a tumour prior to a "main" anti-cancer therapy, such as surgical removal of cancer. A compound of the invention may be used as a neoadjuvant therapy for a patient who will subsequently undergo surgical treatment of cancer and/or radiotherapy for cancer.

Alternatively, or additionally, the invention provides a compound of the invention for use as an adjuvant in the treatment of cancer. An adjuvant is an agent provided to a patient after a "main" anti-cancer therapy, such as surgical removal of cancer, in order to prevent the return of cancer after the main therapy. A compound of the invention may be used as an adjuvant for a patient who has undergone surgical treatment of cancer and/or radiotherapy for cancer.

A compound of the invention may be employed in the uses of the invention in a monotherapy, which is to say in preventions or treatments in which a compound of the invention provides substantially all of the therapeutic activity that is made use of in the prevention or treatment.

Alternatively, the uses of the invention may employ a compound of the invention in a combination therapy. In such embodiments a compound of the invention is used in conjunction with at least one further cancer therapy. The further cancer therapy may comprise surgery and/or radiotherapy. Additionally, or alternatively, the further cancer therapy may comprise use of at least one further therapeutic agent that contributes to the prevention or treatment of cancer to be achieved. Suitably, such an agent may be a chemotherapeutic agent or a biological agent used in the prevention or treatment of cancer.

In a suitable embodiment of a combination therapy a compound of the invention and a further therapeutic agent may be provided to a patient at the same time. In a suitable example, the compound of the invention and a further therapeutic agent may be formulated as part of the same pharmaceutical composition. Alternatively the compound of the invention and a further therapeutic agent may be formulated separately for provision to the patient at substantially the same time. The amount of the compound of the invention required may be substantially reduced when the compounds are used in such embodiments.

In another suitable embodiment of a combination therapy, a compound of the invention and a further therapeutic agent may be provided to a patient at different times. The compound of the invention and a further therapeutic agent may be provided to a patient sequentially. For example, the compound of the invention may be provided to the patient prior to provision of the further therapeutic agent. Alternatively a compound of the invention may be provided to the patient after provision of the further therapeutic agent.

### "Further therapeutic agents"

A compound of the invention may be used in combination with a wide range of further therapeutic agents for the prevention or treatment of cancer. These include biological agents, immunotherapeutic agents, and chemotherapeutic agents that may be used for the prevention or treatment of cancer.

While specific examples of suitable further agents are considered in the following paragraphs, these should not be taken as limiting the range of further therapeutic agents suitable for use with a compound of the invention. Indeed, the ability of a compound of the invention to target cancer stem cells indicates that it may be beneficially used in combination with any further therapeutic agent used in the prevention or treatment of cancer, whether such further agent targets cancer stem cells, non-stem cancer cells, or other cells or constituents involved in the development, maintenance, recurrence and propagation of cancer.
Examples of further therapeutic agents that may be used in combination with a compound of the invention include:
(a) an anti-angiogenic agent, optionally wherein the anti-angiogenic agent is: (i) an inhibitor of the VEGF pathway, optionally bevacizumab; (ii) a tyrosine kinase inhibitor, optionally sorafenib, sunitinib or pazopanib; or (iii) an mTOR inhibitor, optionally everolimus;
(b) an alkylating agent;
(c) an anti-metabolite;
(d) an anti-tumour antibiotic;
(e) a topoisomerase inhibitor;
(f) a mitotic inhibitor;
(g) a monoclonal antibody;
(h) a metallic agent; or
(i) an active or passive immunotherapy.

Except for where the context requires otherwise, the further therapeutic agents set out in the preceding list should all be considered suitable for use in any of the embodiments of combination therapies with a compound of the invention considered above.

### Selection of patients

The inventors' finding that a compound of the invention is able to target cancer stem cells makes possible a number of methods by which it is possible to determine whether a particular patient is likely to benefit from receiving a compound of the invention in the prevention or treatment of cancer, such as relapsed or refractory cancer.

Accordingly, the disclosure provides a method of determining whether a patient with cancer or a pre-cancerous condition will benefit from prevention or treatment of cancer with a compound of the invention, the method comprising: assaying a biological sample representative of cancer or a pre-cancerous condition in the patient for the presence of cancer stem cells; wherein the presence of cancer stem cells in the biological sample indicates that the patient will benefit from treatment with a compound of the invention.

The disclosure further provides a method of determining a suitable treatment regimen for a patient with cancer or a pre-cancerous condition, the method comprising: assaying a biological sample representative of cancer or a pre-cancerous condition in the patient for the presence of cancer stem cells; wherein the presence of cancer stem cells in the biological sample indicates that a suitable treatment regimen will comprise treatment of the patient with a compound of the invention.

The invention also provides a compound of the invention for use in the prevention or treatment of cancer in a patient selected for such treatment by a method comprising: assaying a biological sample representative of cancer or a pre-cancerous condition in the patient for the presence of cancer stem cells; wherein the presence of cancer stem cells in the biological sample indicates that the patient is suitable for treatment with a compound of the invention.

In suitable embodiments cancer stem cells in a biological sample may be identified by their expression of characteristic patterns of markers discussed previously in the application.

The skilled person will appreciate that there are many suitable examples of biological samples that may be used in embodiments of the invention such as those set out above. Suitably, such a sample may include cells from the cancer or pre-cancerous condition. A suitable biological sample may be a tissue sample, such as a sample for use in histology. Cells in such samples may be directly assessed for their expression of cancer stem cell markers, such as those set out above.

Alternatively or additionally, a suitable biological sample may comprise target molecules representative of gene expression by cells of the cancer or pre-cancerous condition. Examples of such target molecules include proteins or nucleic acids, such as mRNA, representative of gene expression.

Suitable examples of techniques by which expression of cancer stem cell markers may be assessed may be selected with reference to the sample type. Techniques for the investigation of expressed markers are frequently used in the context of clinical assessments (such as for diagnostic or prognostic purposes) and their use will be familiar to those required to practice them in the context of the present invention. Merely by way of example, in samples containing proteins the presence of cancer stem cell markers may be assessed by suitable techniques using antibodies that react with the cancer stem cell markers in question. Examples of such samples containing protein cancer stem cell markers include histology samples (where the presence of the markers may be visualised by suitable immunocytochemistry techniques), or samples derived from the circulation. Here the presence of circulating cancer stem cells (which are believed to contribute to the propagation of cancer through metastasis) may be assessed using techniques such as flow cytometry.

In samples containing nucleic acids representative of expression of cancer stem cell markers, such expression may be assessed by suitable molecule biology techniques, such as by polymerase chain reaction (PCR) amplification using suitable primers or RNAscope *in situ* hybridisation technique.

### Synthetic Examples

Examples of the present invention will now be described with respect to the accompanying drawings. The compounds of the invention can be made according to or analogously to the following examples.

### General Experimental

Solvents and Reagents. The following anhydrous solvents were purchased from Sigma-Aldrich: dichloromethane (CH₂Cl₂), trimethylphosphate ((CH₃O)₃PO). Amino acid esters commercially available were purchased from Sigma-Aldrich. All reagents commercially available were used without further purification.

Thin Layer Chromatography (TLC): Precoated aluminium backed plates (60 F254, 0.2 mm thickness, Merck) were visualized under both short and long wave ultraviolet light (254 and 366 nm) or by burning using the following TLC indicators: (i) molybdate ammonium cerium sulphate; (ii) potassium permanganate solution. Preparative TLC plates (20 cm × 20 cm, 500-2000 µm) were purchased from Merck.

Flash Column Chromatography: Flash column chromatography was carried out using silica gel supplied by Fisher (60A, 35-70 µm). Glass columns were slurry packed using the appropriate eluent with the sample being loaded as a concentrated solution in the same eluent or preadsorbed onto silica gel. Fractions containing the product were identified by TLC, and pooled and the solvent was removed *in vacuo.* In an alternative method, flash chromatography was performed with a Biotage Isolera automated purification system using prepacked SNAP KP or SNAP ultra silica gel columns for direct phase chromatography; SNAP Ultra C-18 for reverse phase chromatography.

High Performance Liquid Chromatography (HPLC): The purity of the final compounds was verified to be >95% by HPLC analysis using either I) ThermoSCIENTIFIC, SPECTRA SYSTEM P4000, detector SPECTRA SYSTEM UV2000, Varian Pursuit XRs 5 C18, 150 x 4.6 mm (as an analytic column) or II) Varian Prostar (LC Workstation-Varian Prostar 335 LC detector), Thermo SCIENTIFIC Hypersil Gold C18, 5µ, 150 x 4.6 mm (as an analytic column). For the method of elution see the experimental part.

Nuclear Magnetic Resonance (NMR): ¹H NMR (500 MHz), ¹³C NMR (125 MHz), ³¹P NMR (202 MHz) and ¹⁹F NMR (470 MHz) were recorded on a Bruker Avance 500 MHz spectrometer at 25 °C. Chemical shifts (δ) are quoted in parts per million (ppm) relative to internal MeOH-d₄ (δ 3.34 ¹H-NMR, δ 49.86 ¹³C-NMR) and CHCl₃-d₄ (δ 7.26 ¹H NMR, δ 77.36 ¹³C NMR) or external 85 % H₃PO₄ (δ 0.00 ³¹P NMR). Coupling constants (J) are measured in Hertz. The following abbreviations are used in the assignment of NMR signals: s (singlet), d (doublet), t (triplet), q (quartet), m (multiplet), bs (broad singlet), dd (doublet of doublet), dt (doublet of triplet), app (apparent). The assignment of the signals in ¹H NMR and ¹³C NMR was done based on the analysis of coupling constants and additional two-dimensional experiments (COSY, HSQC, HMBC, PENDANT).

Mass spectrometry (MS): Low resolution mass spectra were performed on Bruker Daltonics microToF-LC, (atmospheric pressure ionization, electron spray mass spectroscopy) in either positive or negative mode.

Purity of final compounds: The ≥95% purity of all the final compounds was confirmed using HPLC analysis.

### Comparative Example 1 - Cyclohexyl 2-(((((2R,3S,4R,5R)-5-(6-amino-8-chloro-9H-purin-9-yl)-3,4-dihydroxy-tetrahydrofuran-2-yl)methoxy)(phenoxy)phosphoryl)amino)acetate A

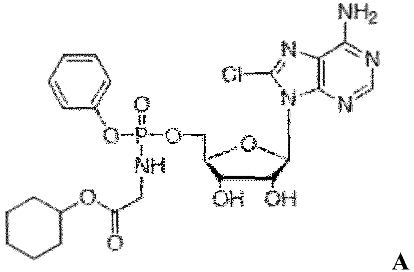

### 8-Chloroadenosine

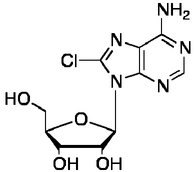

Procedure 1: Adenosine (1.97g, 7.37 mmol) was suspended in anh. DMF (20 mL) and BzCl (0.9 mL, 7.7 mmol) was added. *m*CPBA (70-75 %, 2.03 g, 8.6 mmol) was dissolved in anh. DMF (10 mL) and added dropwise. The reaction mixture was stirred at rt for 20 min before it was poured into ice-cold water (100 mL) and filtered. The filtrate was extracted with Et₂O (3 x 100 mL). The combined aqueous phases were evaporated *in vacuo* to give an oil. The oil was absorbed on silica and purified by silica gel CC (packed in 5% MeOH/CHCl₃, eluted with 7% MeOH/CHCl₃) to yield a white solid (0.53 g, 24 %).
Procedure 2: Adenosine (1.09g, 4.08 mmol) was suspended in DMF (50 mL) and glacial acetic acid (10 mL) was added. A solution of N-chlorosuccinimide (2 g, 15 mmol) in DMF (15 mL) was added dropwise. The reaction mixture was stirred at rt for 48 hours and the volatiles were evaporated *in vacuo* to give yellow gum. The crude was absorbed on silica and purified by silica gel CC (packed in 5% MeOH/CHCl₃, eluted with 7% MeOH/CHCl₃) to yield a white powder (0.65 g, 52 %).

¹H NMR (500 MHz, DMSO-*d6*) δ 8.16 (s, 1H, H-2), 7.55 (br s, 2H, NH₂), 5.86 (d, *J* = 6.8 Hz, 1H, H1'), 5.48 (d, *J* = 6.2 Hz, 1H, 2'OH), 5.45 (d, *J* = 4.0 Hz, 1H, 5'-OH), 5.23 (d, *J* = 4.6 Hz, 1H, 3'OH), 5.08-5.06 (m, 1H, H-2'), 4.23-4.18 (m, 1H, H3'), 4.01-3.96 (m, 1H, H4'), 3.72-3.65 (m, 1H, H5'), 3.57-3.50 (m, 1H, H5').

5-([Cyclohexyloxyglycin-N-yl)-phenyl]-phosphatyl) 8-chloroadenosine, **A,** was prepared according to the following procedure: 8-chloroadenosine (0.21 g, 0.70 mmol) was dissolved in anh. THF (30 mL), and *t*-BuMgCl (1.0 M in THF, 0.70 mmol, 0.70 mL) was added dropwise followed by a solution of phenyl-1-yl-L-glycine cyclohexyl ester phosphorochloridate (0.88 g, 2.65 mmol) dissolved in anh. THF (5 mL). The mixture was stirred at room temperature overnight. Purification first by silica gel CC (0-5% MeOH/CH₂Cl₂) and secondly by prep. TLC (10% MeOH/DCM) yielded 55 mg of the desired compound as a white solid (yield = 14%).
³¹P NMR (202 MHz, CD₃OD) δ 4.7 (s), 4.6 (s).
¹H NMR (500 MHz, CD₃OD) δ 8.19 (s, 0.5H, H2), 8.17 (s, 0.5H, H2), 7.38-7.27 (m, 3H, Ph), 7.24-7.12 (m, 2H, Ph), 6.05 (d, *J* = 4.9 Hz, 1H, H1'), 6.03 (d, *J* = 4.9 Hz, 1H, H1'), 5.34 (t, *J* = 5.2 Hz, 0.5H, H-2'), 5.31 (t, *J* = 5.2 Hz, 0.5H, H-2'), 4.71 (m, 1H, C*H* cyclohexyl), 4.66 (t, *J =* 5.2 Hz, 0.5H, H-3'), 4.60 (t, *J* = 5.2 Hz, 0.5H, H-3'), 4.53-4.33 (m, 2H, H5'), 4.24-4.17 (m, 1H, H4'), 3.71-3.57 (m, 2H, C*H₂* gly), 1.93-1.21 (m, 10H, CH₂ gly).
HPLC Reverse-phase HPLC eluting with H₂O/CH₃OH from 90/10 to 0/100 in 35 minutes, 1 ml/min, λ = 254 nm, two peaks with t_{R} 17.50 min., t_{R} 18.12 min.

### Comparative Example 2 - Benzyl 2-(((((2R,3S,4R,5R)-5-(6-amino-8-chloro-9H-purin-9-yl)-3,4-dihydroxytetra-hydrofuran-2-yl)methoxy)(phenoxy)phosphoryl)amino)acetate B

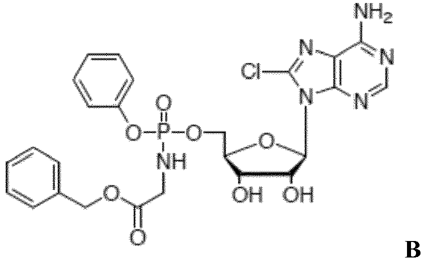

5-([Benzyloxyglycin-N-yl)-phenyl]-phosphatyl) 8-chloroadenosine, **B,** was prepared according to the following procedure: 8-chloroadenosine (0.15 g, 0.50 mmol) was dissolved in anh. THF (20 mL), and *t*-BuMgCl (1.0 M in THF, 0.50 mmol, 0.50 mL) was added dropwise followed by a solution of phenyl-1-yl-L-glycine benzyl ester phosphorochloridate (1.08 g, 3.05 mmol) dissolved in anh. THF (5 mL). The mixture was stirred at room temperature overnight. Purification first by silica gel CC (0-7% MeOH/CH₂Cl₂) and secondly by prep. TLC (10% MeOH/DCM) yielded 82 mg of the desired compound as a white solid (yield = 28%).
³¹P NMR (202 MHz, CD₃OD) δ 4.6 (br s).
¹H NMR (500 MHz, CD₃OD) δ 8.18 (s, 0.5H, H2), 8.16 (s, 0.5H, H2), 7.36-7.14 (m, 10H, Ph), 6.04 (d, *J* = 5.2 Hz, 1H, H1'), 6.02 (d, *J* = 5.2 Hz, 1H, H1'), 5.34 (t, *J* = 5.3 Hz, 0.5H, H-2'), 5.31 (t, *J* = 5.3 Hz, 0.5H, H-2'), 5.16-5.09 (m, 2H, OC*H₂*Ph), 4.64 (t, *J* = 5.1 Hz, 0.5H, H-3'), 4.61 (t, *J* = 5.1 Hz, 0.5H, H-3'), 4.49-4.30 (m, 2H, H5'), 4.23-4.17 (m, 1H, H4'), 3.74-3.65 (m, 2H, C*H₂* gly).
HPLC Reverse-phase HPLC eluting with H₂O/CH₃CN from 90/10 to 0/100 in 35 minutes, 1 ml/min, λ = 254 nm, two peaks with t_{R} 11.30 min., t_{R} 11.01 min.

### Comparative Example 3 - (2S)-Benzyl 2-(((((2R,3S,4R,5R)-5-(6-amino-8-chloro-9H-purin-9-yl)-3,4-dihydroxy-tetrahydrofuran-2-yl)methoxy)(phenoxy)phosphoryl)amino)propanoate C

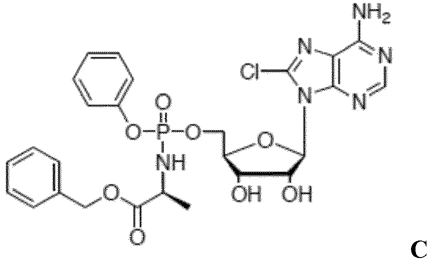

5-([Benzyloxy-L-alanin-*N*-yl)-phenyl]-phosphatyl) 8-chloroadenosine, **C,** was prepared according to the following procedure: 8-chloroadenosine (0.23 g, 0.76 mmol) was dissolved in anh. THF (30 mL), and *t*-BuMgCl (1.0 M in THF, 0.76 mmol, 0.76 mL) was added dropwise followed by a solution of phenyl-1-yl-L-alanine benzyl ester phosphorochloridate (1.08 g, 3.05 mmol) dissolved in anh. THF (5 mL). The mixture was stirred at room temperature overnight. Purification fist by silica gel CC (0-7% MeOH/CH₂Cl₂) and secondly by prep. TLC (7% MeOH/DCM) yielded 9 mg of the desired compound as a white solid (yield = 2%).
³¹P NMR (202 MHz, CD₃OD) δ 3.5 (s), 3.4 (s).
¹H NMR (500 MHz, CD₃OD) δ 8.18 (s, 0.5H, H2), 8.15 (s, 0.5H, H2), 7.44-7.10 (m, 10H, Ph), 6.05-6.00 (m, 1H, H1'), 5.34 (t, *J* = 5.2 Hz, 0.5H, H-2'), 5.28 (t, *J* = 5.2 Hz, 0.5H, H-2'), 5.12-5.05 (m, 2H, OC*H*₂Ph), 4.66 (t, *J* = 5.4 Hz, 0.5H, H-3'), 4.63 (t, *J* = 5.4 Hz, 0.5H, H-3'), 4.45-4.25 (m, 1H, H5'), 4.23-4.15 (m, 2H, H4', H5'), 3.95-3.86 (m, 1H, C*H* L-Ala), 1.30-1.21 (m, 3H, CH₃ L-Ala).
HPLC Reverse-phase HPLC eluting with H₂O/CH₃CN from 90/10 to 0/100 in 35 minutes, 1 ml/min, λ = 254 nm, two peaks with t_{R} 11.30 min., t_{R} 11.01 min.

### Example 4 - (2S)-Benzyl 2-(((((2R,3S,4R,5R)-5-(6-amino-8-chloro-9H-purin-9-yl)-3,4-dihydroxy-tetrahydrofuran-2-yl)methoxynaphthalene-1-yloxy)phosphoryl)amino)propanoate D

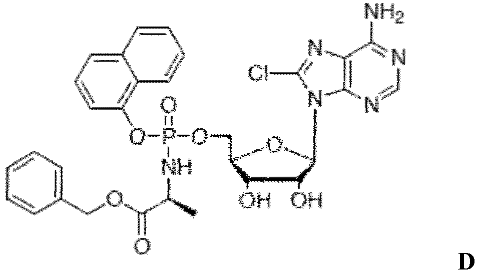

5-([Benzyloxy-L-alanin-*N*-yl)-naphth-1-yl]-phosphatyl) 8-chloroadenosine, **D,** was prepared according to the following procedure: 8-Chloroadenosine (0.40 g, 1.33 mmol) was suspended in acetone (30 mL) and HClO₄ (70%, 0.2 mL) was added dropwise. The mixture was stirred at rt for 30 min before neutralization by NaHCO₃. The solvent was evaporated and the crude purified by silicagel CC (3% MeOH/CHCl₃) to yield 2',3'-O-Isopropylidene-8-chloroadenosine as a white solid (0.36 g, 79%).
2',3'-O-Isopropylidene-8-chloroadenosine (157 mg, 0.52 mmol) was then dissolved in anh. THF (24 mL), and t-BuMgCl (1.0 M in THF, 3eq, 1.56 mL, 1.56 eq) was added slowly followed by naphtha-1-yl-L-alanine benzyl ester phosphorochloridate (540 mg, 1.04 mmol) dissolved in anh. THF (1.5 mL). The mixture was stirred at room temperature overnight. Purification by silica gel CC (0-5% MeOH/DCM) gave a residue that was stirred at room temperature in HCOOH/H₂O (3/2, 10 mL) overnight. The solvents were evaporated in vacuo and the crude purified first by silica gel CC (0-7% MeOH/DCM) and secondly by prep. TLC (7% MeOH/DCM) to yield 67 as a white foam (53 mg, yield 17 % over two steps).
³¹P NMR (202 MHz, CD₃OD) δ 3.8 (s), 3.9 (s).
¹H NMR (500 MHz, CD₃OD) δ 8.12-8.03 (m, 3H, H2, Nap), 7.81-7.83 (m, 1H, Nap), 7.66-7.61 (m, 1H, Nap, Ph), 7.52-7.18 (m, 9H, Nap, Ph) 6.05-6.01 (m, 1H, H1'), 5.35-5.31 (m, 0.5H, H-2'), 5.30-5.26 (m, 0.5 H, H-2'), 5.05-4.91 (m, 2H, OCH₂Ph), 4.69-4.63 (m, 1H, H3'), 4.53-4.45 (m, 1H, H5') 4.43-4.34 (m, 1H, H5'), 4.26-4.20 (m, 1H, H4'), 4.08-4.00 (m, 0.5H, CH ala), 3.97-3.89 (m, 0.5H, CH Ala), 1.28-1.14 (m, 3H, CH₃ ala).
HPLC Reverse-phase HPLC eluting with H₂O/CH₃CN from 90/10 to 0/100 in 35 minutes, 1 ml/min, λ = 254 nm, two peaks with t_{R} 16.48 min., t_{R} 17.07 min.

### Comparative Example 5 - (2S)-Pentyl 2-(((((2R,3S,4R,5R)-5-(6-amino-8-chloro-9H-purin-9-yl)-3,4-dihydroxy-tetrahydrofuran-2-yl)methoxnaphthalene-1-yloxy)phosphoryl)amino)propanoate E

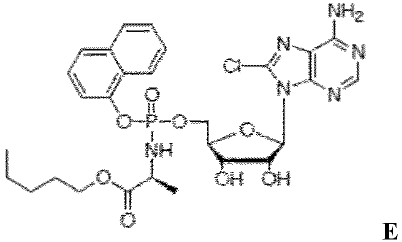

5'-([(Pentyl-1-oxy-L-alanin-*N-*yl)-naphth-1-yl]-phosphatyl) 8-chloroadenosine, **E,** was prepared according to the following procedure: 8-Chloroadenosine (0.40 g, 1.33 mmol) was suspended in acetone (30 mL) and HClO₄ (70%, 0.2 mL) was added dropwise. The mixture was stirred at rt for 30 min before neutralization by NaHCO₃. The solvent was evaporated and the crude purified by silica gel CC (3% MeOH/CHCl₃) to yield 2',3'-O-Isopropylidene-8-chloroadenosine as a white solid (0.36 g, 79%).
2',3'-O-Isopropylidene-8-chloroadenosine (200 mg, 0.59 mmol) was then dissolved in anh. THF (30 mL), and *t*-BuMgCl (1.0 M in THF, 2.93 mmol, 2.93 mL) was added slowly followed by naphthalene-1-yl-L-alanine pent-1-yl ester phosphorochloridate (674.7 mg, 1.76 mmol) dissolved in anh. THF (5mL). The mixture was stirred at rt overnight. Purification first by silica gel CC (0-3% MeOH/DCM) and secondly by prep. TLC (3% MeOH/DCM) gave a residue that was stirred at rt in HCOOH/H₂O (3/2, 10 mL) overnight. The solvents were evaporated *in vacuo* and the crude purified by prep. TLC (10% MeOH/DCM) to yield a white solid (15 mg, yield = 4% over two steps).
³¹P NMR (202 MHz, CD₃OD) δ 3.8 (s), 3.9 (s).
¹H NMR (500 MHz, CD₃OD) δ 8.13 (s, 0.5H, H2) 8.12-8.09 (m, 1H, Nap), 8.06 (s, 0.5 H, H2), 7.88-7.85 (m, 1H, Nap), 7.68 (d, *J* = 8.18 Hz, 1H, Nap), 7.55-7.48 (m, 2H, Nap), 7.45-7.33 (m, 2H, Nap), 6.04 (d, *J* = 4.7 Hz, 0.5H, H1'), 6.03 (d, *J* = 4.7 Hz, 0.5H, H1'), 5.36-5.32 (m, 0.5H, H2'), 5.30-5.27 (m, 0.5 H, H2'), 4.69-4.64 (m, 1H, H3'), 4.55-4.49 (m, 1H, H5'), 4.45-4.36 (m, 1H, H5'), 4.27- 4.22 (m, 1H, H4'), 4.02-3.83 (m, 3H, CH ala, C*H₂*CH₂CH₂CH₂CH₃ Pen), 1.55-1.46 (m, 2H, CH₃ ala), 1.32-1.16 (m, 7H, CH₃ ala, CH₂C*H₂*C*H₂*C*H₂*C*H*₃ Pen), 0.86 (t, *J* = 6.9 Hz, 1.5H, CH₂CH₂CH₂CH₂C*H₃* Pen), 0.82 (t, *J* = 6.9 Hz, 1.5H, CH₂CH₂CH₂CH₂C*H₃* Pen).

HPLC Reverse-phase HPLC eluting with H₂O/CH₃CN from 90/10 to 0/100 in 35 minutes, 1 ml/min, λ = 254 nm, two peaks with t_{R} 18.44 min., t_{R} 17.79 min.

### Comparative Example 6 - 5'-([(Ethyloxy-L-alanin-N-yl)-naphth-1-yl]-phosphatyl) 8-chloroadenosine F

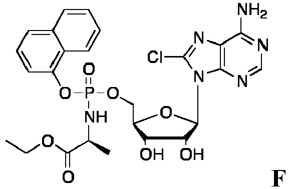

### 2',3'-O-Isopropylidene-8-chloroadenosine

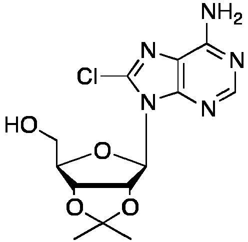

Procedure: 8-Chloroadenosine (0.40 g, 1.33 mmol) was suspended in acetone (30 mL) and HClO₄ (70 %, 0.2 mL) was added dropwise. The mixture was stirred at rt for 30 min before neutralization by NaHCO₃. The solvent was evaporated and the crude purified by silicagel CC (3% MeOH/CHCl₃) to yield a white solid.
Yield: (0.36 g, 79 %).
¹H NMR (500 MHz, MeOD) δ 8.18 (s, 1H, H2), 7.58 (br s, 2H, NH₂), 6.05 (d, *J* = 2.5 Hz, 1H, H1'), 5.68 (dd, *J* = 6.3, 2.5 Hz, 1H, H2'), 5.07 (t, *J* = 5.9 Hz, OH-5'), 5.04 (dd, *J* = 6.3, 3.1 Hz, 1H, H3'), 4.23-4.18 (m, 1H, H-3'), 4.18-4.14 (m, 1H, H-4'), 3.54-3.48 (m, 1H, H5'), 3.46-3.41 (m, 1H, H5'), 1.55 (s, 3H, CH₃), 1.34 (s, 3H, CH₃).

2',3'-*O*-Isopropylidene-8-chloroadenosine (234 mg, 0.69 mmol) was dissolved in anh. THF (31 mL), and t-BuMgCl (1.0 M in THF, 3.41 mL, 3.41 mmol) was added slowly followed by 52 (510 mg, 1.49 mmol) dissolved in anh. THF (2 mL). The mixture was stirred at rt overnight. Purification by silica gel CC (0-5% MeOH/DCM) gave a residue that was stirred at rt in HCOOH/H₂O (3/2, 5 mL) overnight. The solvents were evaporated *in vacuo* and the crude purified twice by preparative TLC (10% MeOH/DCM) to yield 5'-([(Ethyloxy-L-alanin-*N*-yl)-naphth-1-yl]-phosphatyl) 8-chloroadenosine **F** as a white solid.
Yield: (26 mg, 7.4 % over two steps).
¹H NMR (500 MHz, **CD₃OD**) δ 8.12 (s, 0.5H, H2) 8.11-8.07 (m, 1H, Naph) 8.05 (s, 0.5 H, H2), 7.87-7.84 (m, 1H, Nap), 7.67 (d, *J* = 8.1 1H, Nap), 7.54-7.31 (m, 6H, Nap), 6.05-6.02 (m, 1H, H1'), 5.36-5.32 (m, 0.5H, H2'), 5.31-5.28 (m, 0.5H, H2'), 4.72-4.66 (m, 1H, H-3'), 4.55-4.49 (m, 1H, H5') 4.46-4.37 (m, 1H, H5'), 4.28-4.22 (m, 1H, H4'), 4.06-3.91 (m, 2.5H, CH ala, C*H₂*CH₃ Et), 3.87-3.79 (m, 0.5H, CH ala), 1.28-1.25 (m, 1H, CH₃ ala), 1.19-1.08 (m, 5H, CH₃ ala, CH₂C*H₃* Et).
³¹P NMR (202 MHz, **CD₃OD**) δ 3. 98 (s), 3.80 (s).
MS (ES+) m/z: Found: 629.14 (M + Na⁺), 645.11 (M + K⁺), C₂₅H₂₈ClN₆O₈P required: (M⁺) 606.95.
HPLC Reverse-phase HPLC eluting with H₂O/CH₃CN from 90/10 to 0/100 in 35 minutes, 1 ml/min, λ = 254 nm, two peaks with t_{R} 14.43 min., t_{R} 13.73 min.

### Comparative Example 7 - 5'-([(Cyclohexyloxy-L-alanin-N-yl)-naphth-1-yl]-phosphatyl) 8-chloroadenosine G

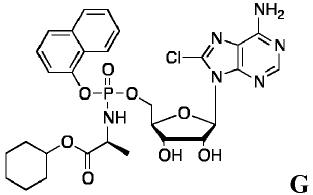

Procedure: Prepared according to the general procedure D.2: 2',3'-*O*-Isopropylidene-8-chloroadenosine (220 mg, 0.64 mmol) was dissolved in anh. THF (30 mL), and *^{t}*BuMgCl (1.0 M in THF, 1.93 mL, 1.93 mmol) was added slowly followed by napht-1-yl(cyclohexyloxy-L-alaninyl) phosphorochloridate (510 mg, 1.29 mmol) dissolved in anh. THF (1.5mL). The mixture was stirred at rt overnight. Purification by silica gel CC (0-2.5% MeOH/DCM) gave a residue that was stirred at rt in HCOOH/H₂O (3/2, 20 mL) overnight. The solvents were evaporated *in vacuo* and the crude purified by silica gel CC (0-4% MeOH/DCM) to yield 69 as a white solid.
Yield: (293 mg, 75 % over two steps).
¹H NMR (500 MHz, CD₃OD) δ 8.13 (s, 0.5H, H2) 8.11-8.07 (m, 1H, Nap), 8.06 (s, 0.5 H, H2), 7.83 (d, *J* = 8.3 Hz, 1H, Nap), 7.64 (d, *J* = 8.3 Hz, 1H, Nap), 7.52-7.39 (m, 3H, Nap), 7.37-7.30 (m, 1H, Nap), 6.06-6.02 (m, 1H, H1'), 5.35-5.31 (m, 0.5H, H2'), 5.30-5.27 (m, 0.5 H, H2'), 4.70-4.65 (m, 1H, H3'), 4.65-4.38 (m, 3H, H5', Ch CHex), 4.29-4.23 (m, 1H, H4'), 4.00-3.92 (m, 0.5H, CH ala), 3.91-3.83 (m, 0.5H, CH ala), 1.79-1.13 (m, 13H, CH₃ ala, CH₂ cHex).
³¹P NMR (202 MHz, CD₃OD) δ 3.93 (s), 3.90 (s).
MS (ES+) m/z: Found: 683.19 (M + Na⁺), 699.17 (M + K⁺), C₂₉H₃₄ClN₆O₈P
required: (M⁺) 660.19.
HPLC Reverse-phase HPLC eluting with H₂O/CH₃CN from 90/10 to 0/100 in 35 minutes, 1 ml/min, λ = 254 nm, two peaks with t_{R} 17.36 min., t_{R} 17.93 min.

### Comparative Example 8 - 5'-([(Benzyloxy-L-leucin-N-yl)-naphth-1-yl]-phosphatyl) 8-chloroadenosine H

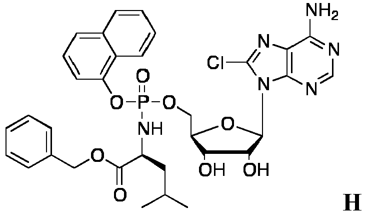

Procedure: Prepared according to the general procedure D.2: 2',3'-*O*-Isopropylidene-8-chloroadenosine (200 mg, 0.59 mmol) was dissolved in anh. THF (30 mL), and t-BuMgCl (1.0 M in THF, 3eq, 1.76 mL, 1.76 eq) was added slowly, followed by Naphth-1-yl-L-leucine benzyl ester phosphorochloridate (657.67 mg, 1.47 mmol) dissolved in anh. THF (3mL). The mixture was stirred at rt overnight. Purification by silica gel CC (0-4% MeOH/DCM) gave a residue that was stirred at rt in HCOOH/H₂O (3/2, 10 mL) overnight. The solvents were evaporated *in vacuo* and the crude purified first by silica gel CC (3-6% MeOH/DCM) and secondly by prep. TLC (10% MeOH/DCM) to yield the product as a white foam.
Yield: (110 mg, 26 % over two steps).
¹H NMR (500 MHz, MeOD) δ 8.10 (s, 0.5H, H2) 8.09-8.05 (m, 1H, H8 Nap), 8.04 (s, 0.5 H, H2), 7.83-7.79 (m, 1H, H5 Nap), 7.62 (d, *J* = 8.37 Hz, 1H, H4 Nap), 7.49- 7.38 (m, 3H, H2, H6, H7 Nap), 7.32-7.19 (m, 6H, H3 Nap, Ph), 6.07-6.02 (m, 1H, H1'), 5.36-5.32 (m, 0.5H, H2'), 5.29-5.25 (m, 0.5H, H2'), 5.02-4.91 (m, 2H, CH₂Ph), 4.67-4.60 (m, 1H, H3'), 4.55-4.41 (m, 2.5H, H5'), 4.40-4.33 (m, 0.5H, H5'), 4.29-4.22 (m, 1H, H4'), 3.99-3.86 (m, 1H, CH leu), 1.65-1.54 (m, 0.5H, CH leu), 1.51-1.31 (m, 2.5 H, CH leu, CH₂ leu, CH₃ Pen), 0.80-0.62 (m, 6H, CH₃ leu).
³¹P NMR (202 MHz, CD₃OD) δ 4.13 (s), 3.95 (s).
MS (ES+) m/z: Found: 733.20 (M + Na⁺), 749.19 (M + K⁺), C₃₃H₃₆CN₆O₈P required: (M⁺) 711.10.
HPLC Reverse-phase HPLC eluting with H₂O/CH₃CN from 90/10 to 0/100 in 35 minutes, 1 ml/min, 1 = 254 nm, two peaks with tR 19.33 min., tR 18.93 min.

### Comparative Example 9 - 5'-([(Pent-1-yloxy-L-leucin-N-yl)-naphth-1-yl]-phosphatyl) 8-chloroadenosine I

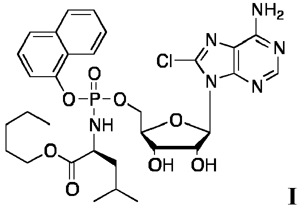

Procedure: Prepared according to the general procedure D.2: 2',3'-*O*-Isopropylidene-8-chloroadenosine (180 mg, 0.53 mmol) was dissolved in anh. THF (30 mL), and *^{t}*BuMgCl (1.0 M in THF, 1.59 mL, 1.59 mmol) was added slowly, followed by Naphth-1-yl-L-leucine pent-1-yl ester phosphorochloridate (677.16 mg, 1.59 mmol) dissolved in anh. THF (3mL). The mixture was stirred at rt overnight. Purification by silica gel CC (0-3% MeOH/DCM) gave a residue that was stirred at rt in HCOOH/H₂O (3/2, 20 mL) overnight. The solvents were evaporated *in vacuo* and the crude purified by silica gel CC (0-6% MeOH/DCM) to yield a white foam. Yield: (132 mg, 36% over two steps).
¹H NMR (500 MHz, MeOD) δ 8.12 (s, 0.5H, H2) 8.11-8.06 (m, 1H, H8 Nap), 8.06 (s, 0.5H, H2), 7.84-7.79 (m, 1H, H5 Nap), 7.65-7.60 (m, 1H, H4 Nap), 7.51- .40 (m, 3H, H2, H6, H7 Nap), 7.32 (t, *J =* 8.0 Hz, 1H, H3 Nap), 6.07-6.03 (m, 1H, H1'), 5.37-5.33 (m, 0.5H, H2'), 5.30-5.26 (m, 0.5H, H2'), 4.69-4.62 (m, 1H, H-3'), 4.57-4.46 (m, 1.5H, H5'), 4.42-4.36 (m, 0.5H, H5'), 4.32-4.24 (m, 1H, H4'), 3.95-3.89 (m, 2H, C*H*₂CH₂CH₂CH₂CH₃ Pen), 3.89-3.83 (m, 1H, CH leu), 1.70-1.57 (m, 0.5H, CH leu), 1.52-1.35 (m, 4.5H, CH₂ leu, CH₂C*H₂*CH₂CH₂CH₃ Pen, CH leu), 1.26-1.15 (m, 4H, CH₂CH₂C*H₂*C*H₂*CH₃ Pen), 0.85-0.80 (m, 3H, CH₂CH₂CH₂CH₂C*H₃* Pen), 0.80-0.68 (m, 6H, CH₃ leu).
³¹P NMR (202 MHz, CD₃OD) δ 4.06 (s), 4.02 (s).
MS (ES+) m/z: Found: 713.24 (M + Na⁺), 729.20 (M + K⁺), C₃₁H₄₀CN₆O₈P
required: (M⁺) 691.11.

HPLC Reverse-phase HPLC eluting with H₂O/CH₃CN from 90/10 to 0/100 in 35 minutes, 1 ml/min, λ = 254 nm, two peaks with t_{R} 21.33 min., t_{R} 20.87 min.

### Comparative Example 10 - 5'-([(Cyclobexyloxy-L-leucin-N-yl)-naphth-1-yl]-phosphatyl) 8-chloroadenosine J

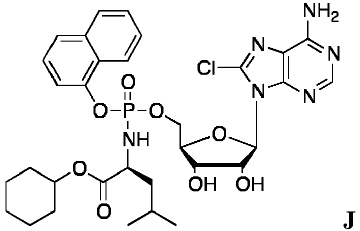

Procedure: Prepared according to the general procedure D.2: 2',3'-*O*-Isopropylidene-8-chloroadenosine (64 mg, 0.19 mmol) was dissolved in anh. THF (15 mL), and *^{t}*BuMgCl (1.0 M in THF, 0.57 mL, 0.57 mmol) was added slowly, followed by Naphth-1-yl-L-leucine cyclohexyl ester phosphorochloridate (250 mg, 0.57 mmol) dissolved in anh. THF (1.5 mL). The mixture was stirred at rt overnight. Purification by silica gel CC (0-3% MeOH/DCM) gave a residue that was stirred at rt in HCOOH/H₂O (3/2, 10 mL) overnight. The solvents were evaporated *in vacuo* and the crude purified twice by prep. TLC (0-5% MeOH/DCM) to yield a white solid.
Yield: (10 mg, 8 % over two steps).
¹H NMR (500 MHz, CD₃OD) δ 8.13 (s, 0.5H, H2) 8.12-8.07 (m, 1H, H8 Nap), 8.07 (s, 0.5 H, H2), 7.89-7.84 (m, 1H, H5 Nap), 7.69-7.64 (m, 1H, H4 Nap), 7.54-7.40 (m, 3H, H2, H6, H7 Nap), 7.37-7.32 (m, 1H, H3 Nap), 6.06-6.02 (m, 1H, H1'), 5.36-5.32 (m, 0.5H, H2'), 5.29-5.25 (m, 0.5H, H2'), 4.67-4.45 (m, 4.5H, H3', H5', CH cHex), 4.41-4.34 (m, 0.5H, H5'), 4.30-4.21 (m, 1H, H4'), 3.91-3.79 (m, 1H, CH leu), 1.74-1.22 (m, 13H, CH₂CH leu, CH₂ cHex), 0.85-0.70 (m, 6H, CH₃ leu).
³¹P NMR (202 MHz, **CD₃OD**) δ 4.12 (s), 4.02 (s).
MS (ES-VE) m/z: Found: 701.21 (M - H⁺), 737.19 (M + Cl⁻), C₃₂H₄₀CN₆O₈P required: (M⁺) 702.2.
HPLC Reverse-phase HPLC eluting with H₂O/CH₃CN from 90/10 to 0/100 in 35 minutes, 1 ml/min, λ = 254 nm, two peaks with t_{R} 20.53 min., t_{R} 20.00 min.

### Comparative Example 11 - 5'-([(ethyloxy-L-leucin-N-yl)-naphth-1-yl]-phosphatyl) 8-chloroadenosine K

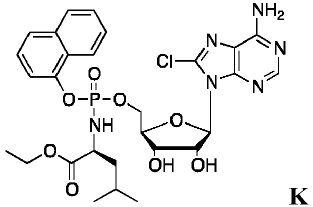

Procedure: 2',3'-*O*-Isopropylidene-8-chloroadenosine (80 mg, 0.23 mmol) was dissolved in anh. THF (15 mL), and *^{t}*BuMgCl (1.0 M in THF, 0.69 mL, 0.69 mmol) was added slowly, followed by Naphth-l-yl-L-leucine ethyl ester phosphorochloridate (254.5 mg, 0.69 mmol) dissolved in anh. THF (1.5 mL). The mixture was stirred at rt overnight. Purification by silica gel CC (0-3% MeOH/DCM) gave a residue that was stirred at rt in HCOOH/H₂O (3/2, 10 mL) overnight. The solvents were evaporated *in vacuo* and the crude purified twice by prep. TLC (10% MeOH/DCM) to yield a white solid.
Yield: (30 mg, 35 % over two steps).
¹H NMR(500 MHz, CD₃OD) δ 8.12 (s, 0.5H, H2) 8.10-8.06 (m, 1H, H8 Nap), 8.05 (s, 0.5H, H2), 7.86-7.82 (m, 1H, H5 Nap), 7.67-7.63 (m, 1H, H4 Nap), 7.53-7.39 (m, 3H, H2, H6, H7 Nap), 7.36-7.31 (m, 1H, H3 Nap), 6.06-6.03 (m, 1H, H1'), 5.36-5.33 (m, 0.5H, H2'), 5.30-5.27 (m, 0.5H, H2'), 4.68-4.62 (m, 1H, H3'), 4.57-4.46 (m, 1.5H, H5'), 4.41-4.35 (m, 0.5H, H5'), 4.30-4.23 (m, 1H, H4'), 4.02-3.92 (m, 2H, C*H*₂CH₃ Et), 3.91-3.81 (m, 1H, CH leu), 1.70-1.61 (m, 0.5H, CH leu), 1.50-1.35 (m, 2.5H, CH₂CH leu), 1.16-1.08 (m, 3H, CH₂C*H₃* Et), 0.84-0.68 (m, 6H, CH₃ leu).
³¹P NMR (202 MHz, CD₃OD) δ 4.11 (s), 4.04 (s).
MS (-S -VE) m/z: Found: 647.22 -M - H⁺), 683.14 (M + Cl⁻), C₂₈H₃₄ClN₆O₈P
required: (M⁺) 649.03.
HPLC Reverse-phase HPLC eluting with H₂O/CH₃CN from 90/10 to 0/100 in 35 minutes, 1 ml/min, λ = 254 nm, two peaks with tR 17.88 min., tR 17.43 min.

### Comparative Example 12 - 5'-([(benzylo-y-glycin-N-yl)-naphth-1-yl]-phosphatyl) 8-chloroadenosine L

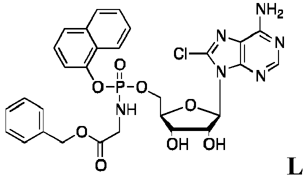

Procedure: 8-Chloroadenosine (150 mg, 0.50 mmol) was dissolved in anh. THF (40 mL), and t-BuMgCl (1.0 M in THF, 1eq, 0.50 mL, 0.50 eq) was added slowly, followed by Naphth-1-yl-glycine benzyl ester phosphorochloridate (779.54 mg, 2 mmol) dissolved in anh. THF (5 mL). The mixture was stirred at rt overnight. The crude was purified first by silica gel CC (0-6% MeOH/DCM) and secondly by prep. TLC (5% MeOH/DCM) to yield 76 as a white powder.
Yield: (13 mg, 4%).
¹H NMR (500 MHz, CD₃OD) δ 8.13-8.06 (m, 1.5H, H2, H8 Nap), 8.05 (s, 0.5H, H2), 7.87-7.84 (m, 1H, H5 Nap), 7.68-7.64 (m, 1H, H4 Nap), 7.53-7.26 (m, 9H, H2, H6, H7, H3 Nap, Ph), 6.04 (d, *J* = 5.1 Hz, 1H, H1'), 6.02 (d, *J* = 5.1 Hz, 1H, H1'), 5.33-5.28 (m, 1H, H2'), 5.10-5.01 (m, 2H, CH₂Ph), 4.67-4.63 (m, 0.5H, H3'), 4.63-4.59 (m, 0.5H, H3'), 4.55-4.48 (m, 1H, H5'), 4.48-4.40 (m, 1H, H5'), 4.26-4.20 (m, 1H, H4'), 3.79-3.71 (m, 2H, CH₂ gly).
³¹P NMR (202 MHz, CD₃OD) δ 4.93 (s).
MS (ES-VE) m/z: Found: 653.19 -M - H⁺), 688.64 (M + Cl⁻), C₂₉H₂₈ClN₆O₈P required: (M⁺) 654.99.
HPLC Reverse-phase HPLC eluting with H₂O/CH₃CN from 90/10 to 0/100 in 35 minutes, 1 ml/min, λ = 254 nm, two peaks with t_{R} 15.84 min., t_{R} 15.35 min.

### Comparative Example 13 - 5'-([(Cyclohexyloxy-glycin-N-yl)-naphth-1-yl]-phosphatyl) 8-chloroadenosine M

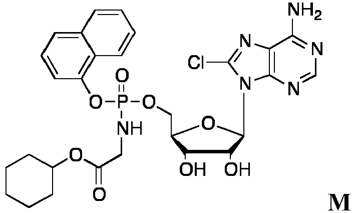

Procedure: 2',3'-*O*-Isopropylidene-8-chloroadenosine (100 mg, 0.29 mmol) was dissolved in anhydrous THF (20 mL), and *^{t}*BuMgCl (1.0 M in THF, 0.88 mL, 0.88 mmol) was added slowly, followed by naphthalene-1-yl glycine cyclohexyl ester phosphorochloridate (335 mg, 0.88 mmol) dissolved in anhydrous THF (1.5 mL). The mixture was stirred at room temperature overnight. Purification by silica gel CC (0-3% MeOH/CH₂Cl₂) gave a residue that was stirred at room temperature in HCOOH/H₂O (3/2, 10 mL) overnight. The solvents were evaporated *in vacuo* and the product purified twice by preparative TLC (10% MeOH/ CH₂Cl₂) to afford the product as a white solid.
Yield: (7 mg, 4 % over two steps).
¹H NMR (500 MHz, CD₃OD) δ 8.15-8.04 (m, 2 H, H2, H8 Nap), 7.89-7.85 (m, 1H, H5 Nap), 7.70-7.65 (m, 1H, H4 Nap), 7.55-7.44 (m, 3H, H2, H6, H7 Nap), 7.42-7.33 (m, 1H, H3 Nap), 6.04 (d, *J* = 6.0 Hz, 0.5 H, H1'), 6.02 (d, *J* = 6.0 Hz, 0.5H, H1'), 5.32-5.28 (m, 1H, H2'), 4.70-4.63 (m, 1.5H, H3', CH cHex), 4.61-4.58 (m, 1H, H3'), 4.57-4.50 (m, 1H, H5'), 4.49-4.42 (m, 1H, H5'), 4.27-4.23 (m, 1H, H4'), 3.72-3.62 (m, 2H, CH₂ gly), 1.81-1.64 (m, 4H, CH₂ cHex), 1.56-1.49 (m, 1H, CH₂ gly), 1.39-1.22 (m, 5H, CH₂ gly).
³¹P NMR (202 MHz, CD₃OD) δ 4.96 (s), 4.94 (s).
MS (ES+) m/z: Found: 669.01 (M + Na⁺), 685.12 (M + K⁺), C₂₈H₃₂ClN₆O₈P required: (M⁺) 647.02.
HPLC Reverse-phase HPLC eluting with H₂O/CH₃CN from 90/10 to 0/100 in 35 minutes, 1 ml/min, λ = 254 nm, two peaks with t_{R} 17.52 min., t_{R} 17.00 min.

### Comparative Example 14 - 5'-([(ethyloxy-glycin-N-yl)-naphth-1-yl]-phosphatyl) 8-chloroadenosine N

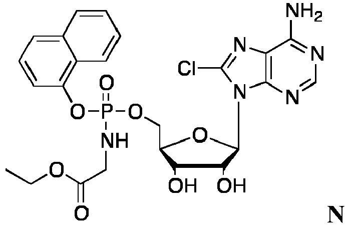

Procedure: 8-Chloroadenosine (100 mg, 0.33 mmol) was dissolved in anh. THF (30 mL), and *^{t}*BuMgCl (1.0 M in THF, 0.33 mL, 0.33 mmol) was added slowly, followed by Naphth-1-yl-glycine ethyl ester phosphorochloridate (434.5 mg, 1.33 mmol) dissolved in anh. THF (3 mL). The mixture was stirred at rt overnight. The crude was purified first by silica gel CC (0-6% MeOH/DCM) and secondly by prep. TLC (5% MeOH/DCM) to yield a white solid.
Yield: (7 mg, 4 %).
¹H NMR (500 MHz, CD₃OD) δ 8.15-8.07 (m, 1.5H, H2, H8 Nap) 8.05 (s, 0.5 H, H2), 7.89-7.85 (m, 1H, H5 Nap), 7.69-7.66 (m, 1H, H4 Nap), 7.55-7.33 (m, 4H, H2, H6, H7, H3 Nap), 6.04 (d, *J* = 5.1 Hz, 0.5H, H1'), 6.02 (d, *J* = 5.1 Hz, 0.5H, H1'), 5.35-5.29 (m, 1H, H2'), 4.69-4.66 (m, 0.5H, H3'), 4.64-4.61 (m, 0.5H, H3'), 4.56-4.50 (m, 1H, H5'), 4.49-4.43 (m, 1H, H5'), 4.27-4.22 (m, 1H, H4'), 4.10-4.00 (m, 2H, CH₂ Et), 3.73-3.62 (m, 2H, CH₂ gly), 1.21-1.13 (m, 3H, CH₃ Et).
³¹P NMR (202 MHz, CD₃OD) δ 4.96 (s), 4.94 (s).
MS (ES -VE) m/z: Found: 591.10 -M - H⁺), 627.08 (M + Cl⁻), C₂₄H₂₆CN₆O₈P
required: (M⁺) 592.93.
HPLC Reverse-phase HPLC eluting with H₂O/CH₃CN from 90/10 to 0/100 in 35 minutes, 1 ml/min, λ = 254 nm, two peaks with t_{R} 13.88

### Comparative Example 15 - 5'-([(Pent-1-yIoxy-glycin-N-yl)-naphth-1-yl]-phosphatyl) 8-chloroadenosine O

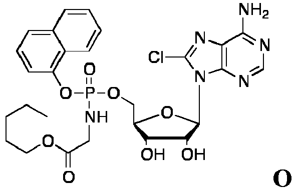

Procedure: 8-Chloroadenosine (130 mg, 0.43 mmol) was dissolved in anh. THF (40 mL), and t-BuMgCl (1.0 M in THF, 1eq, 0.43 mL, 0.43 eq) was added slowly, followed by Naphth-1-yl-glycine pent-1-yl ester phosphorochloridate (637.36 mg, 1.72 mmol) dissolved in anh. THF (5 mL). The mixture was stirred at rt overnight. The crude was purified first by silica gel CC (0-6% MeOH/DCM) and secondly by prep. TLC (7% MeOH/DCM), to yield the product as a white solid.
Yield: (39 mg, 15 %.)
¹H NMR (500 MHz, CD₃OD) δ 8.15-8.07 (m, 1.5H, H2, H8 Nap), 8.05 (s, 0.5H, H2), 7.87-7.82 (m, 1H, H5 Nap), 7.68-7.64 (m, 1H, H4 Nap), 7.54- .42 (m, 3H, H2, H6, H7 Nap), 7.35 (t, *J* = 8.0 Hz, 1H, H3 Nap), 6.04 (d, *J* = 4.7 Hz, 1H, H1'), 6.02 (d, *J* = 4.7 Hz, 1H, H1'), 5.37-5.25 (m, 1H, H2'), 4.69-4.65 (m, 0.5H, H3'), 4.64-4.60 (m, 0.5H, H3'), 4.58-4.50 (m, 1H, H5'), 4.50-4.41 (m, 1H, H5'), 4.29-4.22 (m, 1H, H4'), 4.03-9.94 (m, 2H, C*H*₂CH₂CH₂CH₂CH₃ Pen), 3.75-3.64 (m, 2H, CH₂ gly), 1.57-1.47 (m, 2H, CH₂C*H*₂CH₂CH₂CH₃ Pen), 1.31-1.22 (m, 4H, CH₂CH₂C*H₂*C*H₂*CH₃ Pen), 0.86 (t, *J* = 6.9 Hz, 3H, CH₂CH₂CH₂CH₂C*H₃* Pen).
³¹P NMR (202 MHz, CD₃OD) δ 4.93 (s).
MS (ES+) m/z: Found: 657.19 (M + Na⁺), C₂₇H₃₂CIN₆O₈P required: (M⁺) 635.01.
HPLC Reverse-phase HPLC eluting with H₂O/CH₃CN from 90/10 to 0/100 in 35 minutes, 1 ml/min, λ = 254 nm, two peaks with t_{R} 17.91min., t_{R} 17.39 min.

### Comparative Example 16 - 5'-([(Benzyloxy-L-phenylalanin-N-yl)-naphth-1-yl]-phosphatyl) 8-chloroadenosine P

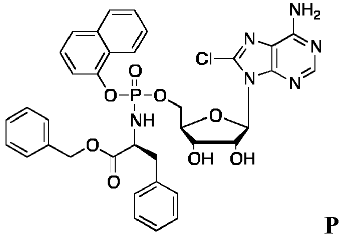

Procedure: 2',3'-*O*-Isopropylidene-8-chloroadenosine (100 mg, 0.29 mmol) was dissolved in anhydrous THF (20 mL), and *^{t}*BuMgCl (1.0 M in THF, 0.88 mL, 0.88 mmol) was added slowly, followed by naphthalene-1-yl-L-phenylalanine cyclohexyl ester phosphorochloridate (422 mg, 0.88 mmol) dissolved in anhydrous THF (2.5 mL). The mixture was stirred at room temperature overnight. Purification by silica gel CC (0-2% MeOH/CH₂Cl₂) gave a residue that was stirred at room temperature in HCOOH/H₂O (3/2, 10 mL) overnight. The solvents were evaporated *in vacuo* and the product purified twice by preparative TLC (7% MeOH/ CH₂Cl₂) to obtain a white foam.
Yield: (12 mg, 6 % over two steps).
¹H NMR (500 MHz, CD₃OD) δ 8.08 (s, 0.5H, H2), 8.03 (s, 0.5H, H2), 8.00-7.97 (m, 1H, H8 Naph), 7.85-7.82 (m, 1H, H5 Nap), 7.64-7.61 (m, 1H, H4 Nap), 7.52-7.47 (m, 1H, H2 Nap), 7.44-7.38 (m, 1H, H6 Nap), 7.28-7.20 (m, 5H, Ph), 7.19-7.11 (m, 5H, Ph), 7.05-7.02 (m, 1H, H7 Nap), 7.00-6.96 (m, 1H, H3 Nap), 6.02-6.00 (m, 1 H, H1'), 5.32-5.25 (m, 1H, H2'), 4.98-4.88 (m, 2H, CH₂Ph), 4.58-4.54 (m, 0.5H, H3'), 4.52-4.49 (m, 0.5H, H3'), 4.34-4.10 (m, 4H, H4', H5', CH phe), 3.01-2.90 (m, 1H, CH₂ phe), 2.83-2.76 (m, 1H, CH₂ phe).
³¹P NMR (202 MHz, CD₃OD) δ 3.66 (s), 3.46 (s).
MS (ES+) m/z: Found: 767.21 (M + Na⁺), C₃₆H₃₄ClN₆O₈P required: (M⁺) 745.12.
HPLC Reverse-phase HPLC eluting with H₂O/CH₃CN from 90/10 to 0/100 in 35 minutes, 1 ml/min, λ = 254 nm, two peaks with t_{R} 20.48 min., t_{R} 20.03 min.

### Comparative Example 17 - 5'-([(Pent-1-yloxy-L-phenylalanin-N-yl)-naphth-1-yl]-phosphatyl) 8-chloroadenosine Q

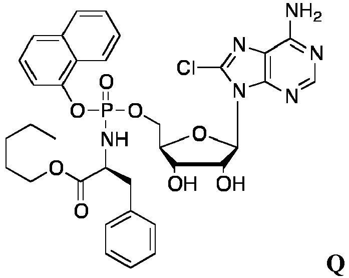

Procedure: 2',3'-*O*-Isopropylidene-8-chloroadenosine (100 mg, 0.29 mmol) was dissolved in anhydrous THF (20 mL), and *^{t}*BuMgCl (1.0 M in THF, 0.88 mL, 0.88 mmol) was added slowly, followed by naphthalene- 1-yl-L-phenylalanine-1-pentyl ester phosphorochloridate (404 mg, 0.88 mmol) dissolved in anhydrous THF (2 mL). The mixture was stirred at room temperature overnight. Purification by silica gel CC (0-2% MeOH/CH₂Cl₂) gave a residue that was stirred at room temperature in HCOOH/H₂O (3/2, 10 mL) overnight. The solvents were evaporated *in vacuo* and the product purified twice by preparative TLC (7% MeOH/ CH₂Cl2) to obtain a white foam.
Yield: (20 mg, 10 % over two steps).
¹H NMR (500 MHz, CD₃OD) δ 8.11 (s, 0.5H, H2),8.05 (s, 0.5H, H2), 8.04-8.00 (m, 1H, H8 Nap), 7.87-7.83 (m, 1H, H5 Nap), 7.67-7.61 (m, 1H, H4 Nap), 7.54-7.43 (m, 2H, H2, H6 Nap), 7.32-7.26 (m, 2H, Ph), 7.22-7.12 (m, 3H, Ph), 7.11-7.08 (m, 1H, H7 Nap), 7.07-7.04 (m, 1H, H3 Nap), 6.04-6.00 (m, 1H, H1'), 5.32-5.26 (m, 1H, H2'), 4.58-4.54 (m, 0.5H, H3'), 4.53-4.50 (m, 0.5H, H3'), 4.37-4.31 (m, 0.5H, H5'), 4.29-4.24 (m, 0.5H, H5'), 4.18-4.05 (m, 3H, H5', H4', CH phe), 3.90-3.79 (m, 2H, C*H₂*CH₂CH₂CH₂CH₃ Pen), 3.00-2.90 (m, 1H, CH₂ phe), 2.85-2.78 (m, 1H, CH₂ phe), 1.45-1.35 (m, 2H, CH₂C*H₂*CH₂CH₂CH₃ Pen), 1.25-1.09 (m, 4H, CH₂CH₂C*H₂*C*H₂*CH₃ Pen), 0.83 (t, *J* = 7.2 Hz, 3H, CH₂CH₂CH₂CH₂C*H₃* Pen), 0.80 (t, *J* = 7.2 Hz, 3H, CH₂CH₂CH₂CH₂C*H₃* Pen).
³¹P NMR (202 MHz, CD₃OD) δ 3.72 (s), 3.39 (s).
MS (ES+) m/z: Found: 747.23 (M + Na⁺), C₃₄H₃₈ClN₆O₈P required: (M⁺) 725.13
HPLC Reverse-phase HPLC eluting with H₂O/CH₃CN from 90/10 to 0/100 in 35 minutes, 1 ml/min, λ = 254 nm, two peaks with t_{R} 22.04 min., t_{R} 21.48 min.

### Comparative Example 18 - 5'-([(Cyclohexyloxy-L-phenylalanin-N-yl)-naphth-1-yl]-phosphatyl) 8-chloroadenosine R

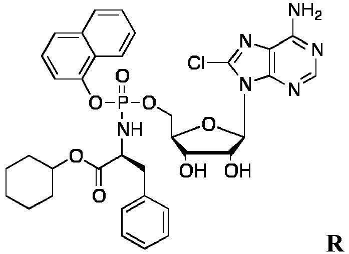

Procedure: 2',3'-*O*-Isopropylidene-8-chloroadenosine (123 mg, 0.36 mmol) was dissolved in anhydrous THF (20 mL), and *^{t}*BuMgCl (1.0 M in THF, 1.1 mL, 1.1 mmol) was added slowly, followed by naphthalene-1-yl-L-phenylalaninecyclohexyl ester phosphorochloridate (498 mg, 1.1 mmol) dissolved in anhydrous THF (2 mL). The mixture was stirred at room temperature overnight. Purification by silica gel CC (0-2.5 % MeOH/CH₂Cl₂) gave a residue that was stirred at room temperature in HCOOH/H₂O (3/2, 10 mL) overnight. The solvents were evaporated *in vacuo* and the product purified twice by preparative TLC (10 % MeOH/ CH₂Cl₂) to obtain a white foam.
Yield: (53 mg, 21 % over two steps).
¹H NMR (500 MHz, CD₃OD) δ 8.10 (s, 0.5H, H2), 8.05 (s, 0.5H, H2), 8.04-8.00 (m, 1H, H8 Nap), 7.86-7.82 (m, 1H, H5 Nap), 7.65-7.61 (m, 1H, H4 Nap), 7.53-7.48 (m, 1H, H2), 7.47-7.42 (m, 1H, H6), 7.32-7.22 (m, 2H, Ph), 7.21-7.12 (m, 3H, Ph), 7.11-7.08 (m, 1H, H7 Nap), 7.07-7.04 (m, 1H, H3 Nap), 6.04-6.01 (m, 1H, H1'), 5.32-5.27 (m, 1H, H2'), 4.59-4.50 (m, 2H, H3', CH cHex), 4.39-4.26 (m, 1H, H5'), 4.20-4.04 (m, 3H, H5', H4', CH phe), 2.99-2.90 (m, 1H, CH₂ phe), 2.86-2.78 (m, 1H, CH₂ phe), 1.69-1.52 (m, 4H, CH₂ cHex), 1.50-1.41 (m, 1H, CH₂ cHex), 1.32-1.12 (m, 5H, CH₂ cHex).
³¹P NMR (202 MHz, CD₃OD) δ 3.73 (s), 3.74 (s).
MS (ES+) m/z: Found: 759.19 (M + Na⁺), C₃₅H₃₈ClN₆O₈P required: (M⁺) 737.14.
HPLC Reverse-phase HPLC eluting with H₂O/CH₃CN from 90/10 to 0/100 in 35 minutes, 1 ml/min, λ = 254 nm, two peaks with t_{R} 21.81 min., t_{R} 21.27 min.

### Comparative Example 19 - 5'-([(Ethyloxy-L-phenylalanin-N-yl)-naphth-1-yl]-phosphatyl) 8-chloroadenosine S

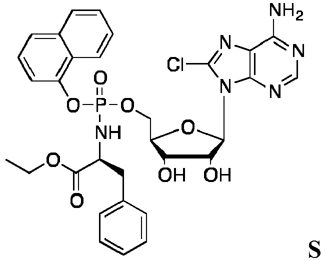

Procedure: 2',3'-*O*-Isopropylidene-8-chloroadenosine (170 mg, 0.50 mmol) was dissolved in anhydrous THF (30 mL), and *^{t}*BuMgCl (1.0 M in THF, 1.5 mL, 1.5 mmol) was added slowly, followed by naphthalene-1-yl-L-phenylalanine-1-ethyl ester phosphorochloridate (1.05 g, 2.5 mmol) dissolved in anhydrous THF (2 mL). The mixture was stirred at room temperature overnight. Purification by silica gel CC (0-2% MeOH/CH₂Cl₂) gave a residue that was stirred at room temperature in HCOOH/H₂O (3/2, 10 mL) overnight. The solvents were evaporated *in vacuo* and the product purified twice by preparative TLC (7% MeOH/ CH₂Cl₂) to obtain a white foam.
Yield: 90 mg, 26 % over two steps.
¹H NMR (500 MHz, CD₃OD) δ 8.09 (s, 0.5H, H2), 8.04 (s, 0.5H, H2), 8.00 (d, *J =* 8.4 Hz, 1H, H8 Nap), 7.80 (d, *J =* 8.2 Hz, 1H, H5 Nap), 7.62-7.58 (m, 1H, H4 Nap), 7.50-7.40 (m, 2H, H2, H6), 7.32-7.23 (m, 1H, Ph), 7.20-7.07 (m, 4H, H7, Ph), 7.05-7.01 (m, 1H, H3 Nap), 6.05-6.01 (m, 1H, H1'), 5.33-5.28 (m, 1H, H2'), 4.60-4.53 (m, 1H, H3'), 4.38-4.31 (m, 0.5H, H5'), 4.30-4.24 (m, 0.5H, H5'), 4.20-4.10 (m, 4.5H, H5', H4', CH phe, CH₂ Et), 4.09-4.03 (m, 0.5H, H5'), 3.97-3.85 (m, 2H, CH₂ phe), 1.07-0.98 (m, 3H, CH₃ Et).
³¹P NMR (202 MHz, CD₃OD) δ 3.73 (s), 3. 42 (s).
MS (ES+) m/z: Found: 705.18 (M + Na⁺), C₃₁H₃₂ClN₆O₈P required: (M⁺) 683.05.
HPLC Reverse-phase HPLC eluting with H₂O/CH₃CN from 90/10 to 0/100 in 35 minutes, 1 ml/min, λ = 254 nm, two peaks with t_{R} 17.00 min., t_{R} 16.53 min.

### Comparative Example 20 - 5'-([(Benzyloxy-D-alanin-N-yl)-phenyl]-phosphatyl) 8-chloroadenosine T

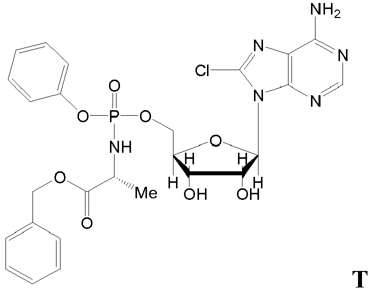

8-Chloroadenosine (0.31 g; 1.03 mmol) was dissolved in anh. THF under Ar and *t*BuMgCl (1.0 M in THF, 1 eq, 1.2 mL, 1.2 mmol) was added dropwise at rt. The mixture was stirred for 10 min before addition of the appropriate phosphorochloridate (0.91 g, 2.57 mmol) in anh. THF. The mixture was stirred overnight and the solvent evaporated in *vacuo.* The residue was purified by silica gel CC (3-5% MeOH/DCM) followed by another CC (4% MeOH/CHCl₃) and prep. TLC (mobile phase: 5% MeOH/CHCl₃) and EtOAc/H₂O wash. Yield: 62 mg, 11%.
¹H NMR (500 MHz, CD₃OD) δ 8.18, 8.16 (2 x s, 1H, H-2), 7.36 - 7.07 (m, 10H, Ar), 6.05, 6.02 (2 x d, *J=* 4.6, 5.0, 1H, H-1'), 5.32 (m, 1H, H-2'), 5.14 - 4.46 (m, 2H, CH₂ (Bn)), 4.67, 4.61 (2 x t, *J=* 5.3, 5.2, 1H, H-3'), 4.46 - 4.26 (m, 2H, H-5'), 4.21 (m, 1H, H-4'), 3.89 (m, 1H, CHCH₃), 1.24, 1.21 (2 x d*, J =* 7.3, 7.0, 3H, CH₃).
³¹P NMR (202 MHz, CD₃OD) δ 3.6, 3.4 (2 x s, diastereoisomers).
HR-MS (ES+) m/z: Found: (M + H⁺) Found 619.1490. C₂₆H₂₈ClN₆O₈P required: (M⁺) 619.1473.
HPLC Reverse-phase HPLC eluting with H₂O/CH₃OH from 90/10 to 0/100 in 30 minutes, 1 ml/min, λ = 254 nm, one peak with t_{R} 17.00 min.
HPLC Reverse-phase HPLC eluting with H₂O/CH₃CN from 90/10 to 0/100 in 30 minutes, 1 ml/min, λ = 254 nm, one peak with t_{R} 11.6 min.

### Comparative Example 21 - 5'-([(Benzyloxy-L-phenylalanin-N-yl)-phenyl]-phosphatyl) 8-chloroadenosine U

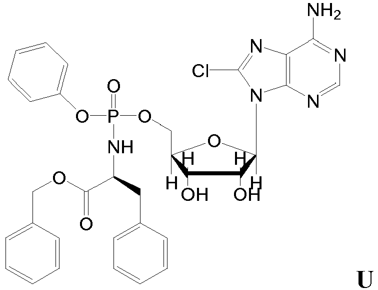

2',3'-*O*-isopropylidene-8-chloroadenosine (0.25 g, 0.73 mmol) was dissolved in anh. THF (30 mL), and *t*BuMgCl (1.0 M in THF, 6 eq, 4.4 mL, 4.4 mmol) was added. The appropriate phosphorochloridate (1.61 g, 3.75 mmol) in anh. THF(3 mL) was added and the mixture was stirred overnight. The solvent was evaporated *in vacuo.* Purification by silica gel CC (3% MeOH/DCM) gave a residue that was stirred at rt in HCOOH/H₂O (30 + 20 mL) overnight. Silica gel CC (3-5.5% MeOH/DCM) followed by prep. TLC (10% MeOH/DCM) gave the desired product. Yield: 76 mg, 15% over 2 steps.

¹H NMR (500 MHz, CD₃OD) δ 8.14, 8.13 (2 x s, 1H, H-2, diastereoisomers), 7.29 - 6.96 (m, 15H, Ar), 6.03 (m, 1H, H-1'), 5.32, 5.28 (2 x t, *J =* 5.4, 5.1, 1H, H-2'), 5.03 - 4.98 (m, 2H, CH₂ (Bn)), 4.56, 4.51 (2 x t, *J* = 5.3, 5.1, 1H, H-3'), 4.26 - 4.04 (m, 4H, H-4', H-5', CHCH₂ (Phe)), 2.97, 2.83 (2 x m, 2H, CH₂CH (Phe)).
³¹P NMR (202 MHz, **CD₃OD)** δ 3.3, 3.1 (2 x s, diastereoisomers).
HR-MS (ES+) m/z: Found: (M + Na⁺) Found 717.1632. C₃₂H₃₂ClN₆O₈P required: (M+Na+) 717.1605.

### Comparative Example 22 - 5'-([(Benzyloxy-L-leucin-N-yl)-phenyl]-phosphatyl) 8-chloroadenosine V

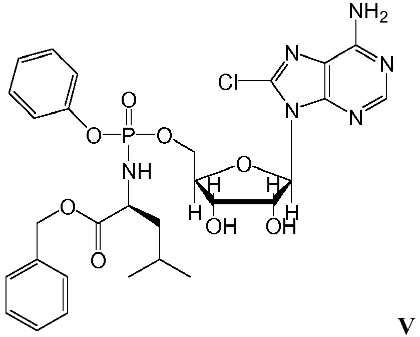

2',3'-*O*-isopropylidene-8-chloroadenosine (80 mg, 0.23 mmol) was dissolved in anh. THF (30 mL), and *t*BuMgCl (1.0 M in THF, 6 eq, 1.4 mL, 1.4 mmol) was added. The appropriate phosphorochloridate (0.49 g, 1.24 mmol) in anh. THF (3 mL) was added and the mixture was stirred overnight. Purification by silica gel CC (2% MeOH/DCM) gave a residue that was stirred at rt in HCOOH/H₂O (18 + 12 mL) overnight. The product was purified by prep. TLC (3-3.55% MeOH/DCM). Yield: 83 mg, 55% over 2 steps.

¹H NMR (500, MHz, MeOD) δ 8.17, 8.15 (2 x s, 1H, H-2), 7.38 - 7.25, 7.20 - 7.09 (2 x m, 10H, Ar), 6.03 (m, 1H, H-1'), 5.35, 5.27 (2 x t, *J =* 5.4, 5.1, 1H, H-2'), 5.11 - 5.04 (m, 2H, CH₂ (Bn)), 4.61, 4.57 (2 x t, *J* = 5.3, 5.1, 1H, H-3'), 4.45 - 4.24 (m, 2H, H-5'), 4.19 (m, 1H, H-4'), 3.88 (m, 1H, CHNH), 1.71 - 1.38 (m, 3H, CH₂CH(CH₃)₂), 0.86, 0.83, 0.79, 0.76 (4 x d, *J* = 6.7, 6.6, 6.3, 6.2, 6H, CH(CH₃)₂, diastereoisomers).
³¹P NMR (202 MHz, CD₃OD) δ 3.8, 3.6 (2 x s, diastereoisomers).
HR-MS (ES+) m/z: Found: (M + H⁺) Found 661.1965. C₂₉H₃₄ClN₆O₈P required: (M⁺) 661.1943.
HPLC Reverse-phase HPLC eluting with H₂O/CH₃CN from 90/10 to 0/100 in 30 minutes, 1 ml/min, λ = 254 nm, two peaks with t_{R} 13.7 min, t_{R} 13.9 min.

### Comparative Example 23 - 5'-([(Isopropyloxy-L-alanin-N-yl)-phenyl]-phosphatyl) 8-chloroadenosine W

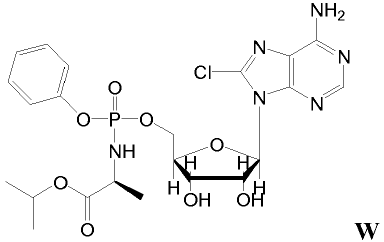

8-Chloroadenosine (0.30 g, 0.99 mmol) was dissolved in a mixture of anh. pyridine and THF (8 + 12 mL). The solution was cooled to 0°C and NMI (0.4 mL, 5.0 mmol) was added dropwise. The mixture was stirred for 10 min before phosphorochloridate (1.26 g, 4.12 mmol) dissolved in anh. THF (2 mL) was added slowly. After overnight stirring, additional NMI (0.4 mL, 5.0 mmol) was added and the mixture stirred for 48 h. *t*BuMgCl (0.1 M in THF, 1.0 mL, 1.0 mmol) and phosphorochloridate (1.33 g, 4.35 mmol) was added and the mixture was stirred for 24 h. The solvents were evaporated. The residue was purified twice by silica gel CC (5-10% MeOH/DCM and 4-5% MeOH/DCM) followed by preparative TLC (4 x 5% MeOH/DCM). The desired product was finally dissolved in EtOAc and washed with water. Yield: 15 mg, 2%
³¹P NMR (202 MHz, CD₃OD) δ 3.60, 3.55 (2 x s, diastereoisomers). ¹H NMR (500 MHz, CD₃OD) δ 7.35 - 7.27, 7.19 - 7.13 (2 x m, 6H, Ph, H-2), 6.04 (m, 1H, H-1'), 5.34, 5.29 (2 x t, *J* = 5.0, 4.9, 1H, H-2', diastereoisomers), 4.91 (m, 1H, CH (iPr)), 4.66 (m, 1H, H-3'), 4.45, 4.33 (2 x m, 2H, H-5'), 4.22 (m, 1H, H-4'), 3.85, 3.77 (2 x m, 1H, CH (Ala), diastereoisomers), 1.34 - 1.27, 1.23 - 1.17 (2 x m, 9H, 3 x CH₃).
HPLC Reverse-phase HPLC eluting with H₂O/CH₃OH from 90/10 to 0/100 in 30 minutes, 1 ml/min, λ = 254 nm, two peaks with t_{R} 15.1 min, t_{R} 15.9 min.

### Comparative Example 24 - 5'-([(Cyclohexyloxy-L-alanin-N-yl)-phenyl]-phosphatyl) 8-chloroadenosine X

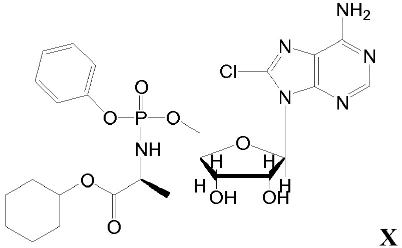

8-Chloroadenosine (0.21 g, 0.70 mmol) was dissolved in a mixture of anh. pyridine and anh. THF (8 + 12 mL), *t*BuMgCl (1.0 M in THF, 0.7 mL, 0.7 mmol) was added dropwise at 0°C and the mixture was stirred for 10 min. The appropriate phosphorochloridate (0.5 g, 1.4 mmol) was dissolved in anh. THF (2 mL) and added slowly to the mixture. The mixture was stirred for 24 h before the solvents were evaporated. The residue was purified by silica gel CC (5% MeOH/DCM) followed by preparative TLC (5% MeOH/DCM). This was followed by another preparative TLC (10% MeOH/DCM) and subsequent EtOAc/H₂O wash. The product was obtained by evaporation of the organic phase. Yield: 8 mg, 2%.

¹H NMR (500 MHz, CD₃OD) δ 8.19, 8.17 (2 x s, H-2, diastereoisomers), 7.34 - 7.27 (m, 2H, Ph), 7.19 - 7.13 (m, 3H, Ph.), 6.04 (d, *J =* 4.5, 1H, H-1 '), 5.35, 5.29 (2 x m, 1H, H-2', diastereoisomers), 4.73 - 4.62 (m, 2H, H-3', CH (cHx)), 4.45, 4.34 (2 x m, 2H, H-5'), 4.22 (m, 1H, H-4'), 3.88, 3.80 (2 x m, 1H, CH (Ala)), 1.82 - 1.67, 1.54, 1.46 - 1.31 (3 x m, 10H, cHx), 1.29, 1.22 (2 x d, *J=* 7.0, 7.1, 3H, CH₃).
³¹P NMR (202 MHz, **CD₃OD)** δ 3.6, 3.5 (2 x s, diastereoisomers).
HPLC Reverse-phase HPLC eluting with H₂O/CH₃OH from 90/10 to 0/100 in 30 minutes, 1 ml/min, λ = 254 nm, two peaks with t_{R} 18.0 min, t_{R} 18.6 min

### Comparative Example 25 - 5'-([(Neopentyloxy-2,2-dimethylglycin-N-yl)-phenyl]-phosphatyl) 8-chloroadenosine Y

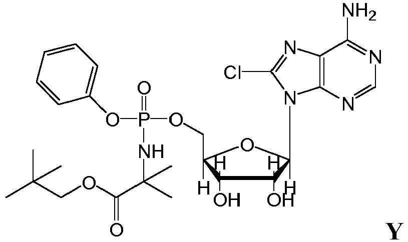

8-Chloroadenosine (0.30 g, 0.99 mmol) was dissolved in a mixture of anh. pyridine and anh. THF (2 + 3), *t*BuMgCl (1.0 M in THF, 1.0 mL, 1.0 mmol) was added dropwise at 0°C and the mixture was stirred for 10 min. The appropriate phosphorochloridate (1.6 g, 4.5 mmol) was dissolved in anh. THF and added slowly to the mixture. The mixture was stirred at rt for 48 h. The solvent was evaporated and the residue purified by silica gel CC (3% MeOH/DCM), prep. TLC (mobile phase: 10% MeOH/DCM) and EtOAc/H₂O wash. This was followed by prep. TLC (mobile phase: 10% MeOH/CHCl₃) to give the desired product. Yield: 13 mg, 2%.

¹H NMR (500 MHz, CD₃OD) δ 8.2, 8.1 (2 x s, 1H, H-2, diastereoisomers), 7.33 - 7.27 (m, 2H, Ph), 7.19 - 7.12 (m, 3H, Ph), 6.04 (m, 1H, H-1'), 5.33, 5.28 (2 x t, *J=* 5.2, 4.9, 1H, H-2') 4.67, 4.63 (2 x t, *J=* 5.4, 5.2, 1H, H-3'), 4.46, 4.37 (2 x m, 2H, H-5'), 4.22 (m, 1H, H-4'), 3.79 - 3.76 (m, 2H, CH₂*t*Bu), 1.45, 1.44, 1.43, 1.41 (4 x s, CH₃ dimethylglycine, diastereoisomers), 0.94 (s, 9H, *t*Bu).
³¹P NMR (202 MHz, **CD₃OD)** δ 2.0, 1.9 (2 x s, diastereoisomers).
HPLC Reverse-phase HPLC eluting with H₂O/CH₃OH from 90/10 to 0/100 in 30 minutes, 1 ml/min, λ = 254 nm, two peaks with t_{R} 18.5 min, t_{R} 18.8 min.

### Comparative Example 26 - 5'-([(Methyloxy-L-methionin-N-yl)phenyl]-phosphatyl)8-chloroadenosine Z

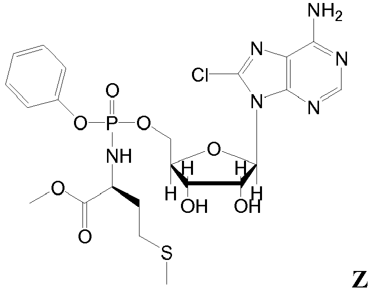

2',3'-*O*-Isopropylidene-8-chloroadenosine (0.18 g, 0.49 mmol) was dissolved in anh. THF (20 mL) and *t*BuMgCl (1.0 M in THF, 10 eq, 5.0 mL, 5.0 mmol) was added. Phosphorochloridate (1.26 g, 3.73 mmol) in anh. THF (3 mL) was added and the mixture was stirred overnight. The solvent was evaporated *in vacuo.* The residue obtained after silica gel CC (3% MeOH/DCM) was stirred overnight in a HCOOH/H₂O mixture (6 + 4 mL). Evaporation of the solvents gave a residue that was purified twice by prep. TLC (5% MeOH/DCM, 10% MeOH/DCM), dissolved in EtOAc and washed with H₂O. Yield: 15 mg, 5% over 2 steps.

³¹P NMR (202 MHz, CD₃OD) δ 3.63, 3.61 (2 x s, diastereoisomers). ¹H NMR (500 MHz, CD₃OD) δ 8.20, 8.17 (2 x s, 1H, H-2, diastereoisomers), 7.36 - 7.28 (m, 2H, Ph), 7.21 - 7.14 (m, 3H, Ph), 6.05, 6.03 (2 x d, *J* = 5.0, 4.7, 1H, H-1'), 5.37, 5.30 (2 x t, *J* = 5.3, 5.1, 1H, H-2'), 4.65 (t, *J* = 5.2, 1H, H-3'), 4.51 - 4.29 (m, 2H, H-5'), 4.24, 4.20 (2 x q, *J* = 4.6, 4.9, 1H, H-4'), 4.03, 3.93 (2 x m, 1H, CH (Met)), 3.653, 3.646 (2 x s, 3H, CH₃O), 2.51 - 2.44, 2.40 - 2.28 (2 x m, 2H, CH₂S), 2.01, 1.98 (2 x s, 3H, CH₃S), 1.95 - 1.81, 1.79 - 1.70 (2 x m, 2H, CH₂CH (Met)).
HR-MS (ES+) m/z: Found: (M + Na⁺) Found 625.1006. C₂₂H₂₈ClN₆O₈P required: (M+Na⁺) 625.1013.

### Comparative Example 27 - 5'-([(Benzyloxy-L-valin-N-yl)-phenyl]-phosphatyl) 8-chloroadenosine AA

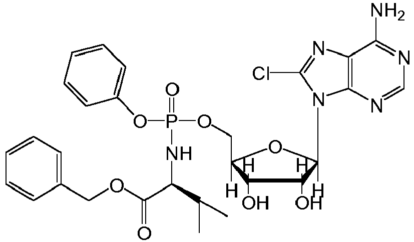

2',3'-O-Cyclopentylidene-8-chloroadenosine (0.15 g, 0.41 mmol) was dissolved in anh. THF (15) and *t*BuMgCl (1.0 M in THF, 3.eq, 1.2 mL. 1.2 mmol) was added slowly followed by phosphorochloridate (0.89 g, 2.33 mmol) dissolved in anh. THF (3 mL). The mixture was stirred at rt overnight. Purification by silica gel CC (0-2.5% MeOH/DCM) gave a residue that was stirred at rt in HCOOH/H₂O (6 + 4 mL) overnight. The solvents were evaporated *in vacuo* and the product purified twice by silica gel CC (4%MeOH/DCM, 3.5%-4% MeOH/DCM). Yield: 0.11 g, 41% over 2 steps.
MS: m/z = [M(³⁵Cl) + H]⁺ calc./exp.: 647.1786/647.1786 ³¹P NMR (202 MHz, CD₃OD) δ 4.3, 4.2 (2 x s, diastereoisomers). ¹H NMR (500 MHz, CD₃OD) δ 8.17, 8.15 (2 x s, 1H, H-2, diastereoisomers), 7.45 - 7.08 (m, 10H, Ar), 6.04 (m 1H, H-1'), 5.34, 5.28 (2 x m, 1H, H-2'), 5.11 - 5.03 (m, 2H, CH₂ (Bn)), 4.65, 4.61 (2 x t, *J* = 5.3, 5.2, 1H, H-3'), 4.42, 4.31 (2 x m, 2H, H-5'), 4.20 (m, 1H, H-4'), 3.64 (m, 1H, CHNH (Val)), 1.94 (m, 1H, CH(CH₃)₂), 0.84, 0.81, 0.77, 0.76 (4 x d, *J =* 6.8, 6.8, 5.4, 6H, 2 x CH₃, diastereoisomers.
HR-MS (ES+) m/z: Found: (M + H⁺) Found 647.1786. C₂₈H₃₂ClN₆O₈P required: (M⁺) 647.1786.

HPLC Reverse-phase HPLC eluting with H₂O/CH₃OH from 90/10 to 0/100 in 30 minutes, 1 ml/min, λ = 254 nm, one peak with t_{R} 19.0 min.
HPLC Reverse-phase HPLC eluting with H₂O/CH₃CN from 90/10 to 0/100 in 30 minutes, 1 ml/min, λ = 254 nm, two peaks with t_{R} 13.1 min.

### Comparative Example 28 - 8-Chloroadenosine 5'-O-bis(benzyloxy-L-alanin-N-yl) phosphate AB

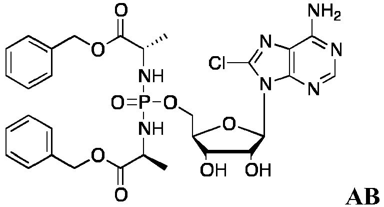

Procedure: 8-chloroadenosine (250 mg, 0.83 mmol) was suspended in (CH₃)₃P0₃ (5 mL), and POCl₃ (77.36 µL, 0.83 mmol) was added dropwise at -5 °C. The reaction mixture was left stirring for 4 hours at room temperature. A solution of L-alanine-O-benzyl ester *p*-TSA salt (1.46 g, 4.15 mmol) dissolved in anhydrous CH₂Cl₂ (5 mL) was added followed by diisopropyl ethyl amine (1.45 mL, 8.3 mmol) at -78 °C. After stirring at room temperature for 20 hours, water was added and the layers were separated. The aqueous phase was extracted with dichloromethane and the organic phase washed with brine. The combined organic layers were dried over Na₂SO₄ and concentrated. The residue was purified by column chromatography (gradient elution of CH₂Cl₂/MeOH=96/4 to 93/7) to give a white foam.
Yield: (245 mg, 42 % over two steps).
¹H NMR (500 MHz, CD₃OD) δ 8.21 (s, 1H, H2), 7.36-7.26 (m, 10H, Ph), 6.02 (d, *J* = 4.8 Hz, 1H, H1'), 5.30 (t, *J* = 5.5 Hz, 1H, H2'), 5.14-5.02 (m, 4H, CH₂Ph), 4.62 (dd, *J* = 7.5, 5.0 Hz, 1H, H3'), 4.31-4.24 (m, 1H, H5'), 4.18-4.11 (m, 2H, H5', H4'), 3.95-3.82 (m, 2H, CH ala), 1.28 (d*, J =* 7.2 Hz, 3H, CH₃ ala), 1.23 (d, *J =* 7.2 Hz, 3H, CH₃ ala).
³¹P NMR (202 MHz, CD₃OD) δ 13.54 (s).
MS (ES+) m/z: Found: 704.1 (M + H⁺), 726.1 (M + Na⁺), C₃₀H₃₅ClN₇O₉P required: (M⁺) 704.07.
HPLC Reverse-phase HPLC eluting with H₂O/CH₃CN from 90/10 to 0/100 in 35 minutes, 1 ml/min, 1 = 254.

### Comparative Example 29 - 8-Chloroadenosine 5'-O-bis(benzyloxy-glycin-N-yl) phosphate AC

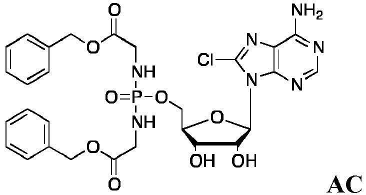

Procedure: 8-chloroadenosine (100 mg, 0.33 mmol) was suspended in (CH₃)₃PO₃ (5 mL), and POCl₃ (30.89 uL, 0.33 mmol) was added dropwise at -5 °C. The reaction mixture was left stirring for 4 hours at room temperature. A solution of glycine-O-benzyl ester hydrochloride salt (333 mg, 1.65 mmol) dissolved in anhydrous CH₂Cl₂ (5 mL) was added followed by diisopropyl ethyl amine (0.57 mL, 3.3 mmol) at -78 °C. After stirring at room temperature for 20 hours, water was added and the layers were separated. The aqueous phase was extracted with dichloromethane and the organic phase washed with brine. The combined organic layers were dried over Na₂SO₄ and concentrated. The residue was purified by column chromatography (gradient elution of CH₂Cl₂/MeOH=96/4 to 93/7) to give a white foam.
Yield: (60 mg, 27 %).
¹H NMR (500 MHz, CD₃OD) δ 8.23 (s, 1H, H2), 7.35-7.26 (m, 10H, Ph), 6.03 (d, *J* = 5.1 Hz, 1H, H1'), 5.31 (dd, *J=* 5.5, 5.1 Hz, 1H, H2'), 5.11-5.08 (m, 4H, CH₂Ph), 4.59 (t, 5.5, 5.1 Hz, 1H, H3'), 4.34-4.28 (m, 1H, H5'), 4.27-4.16 (m, 2H, H5', H4'), 3.70-3.64 (m, 4H, CH₂ gly). ³¹P NMR (202 MHz, CD₃OD) δ 15.96 (s).
MS (ES+) m/z: Found: 704.1 (M + H⁺), 726.1 (M + Na⁺), C₂₈H₃₁ClN₇O₉P required: (M⁺) 676.01.
HPLC Reverse-phase HPLC eluting with H₂O/CH₃OH from 100/0 to 0/100 in 35 minutes, 1 ml/min, λ = 254 nm, one peak with t_{R} 23.23 min.

### Stereochemistry and Method of Determination

### Isomer separation and characterization

Both benzyl 2-(((((2*R*,3*S*,4*R*,5*R*)-5-(6-amino-8-chloro-9*H*-purin-9-yl)-3,4-dihydroxytetra-hydrofuran-2-yl)methoxy)(phenoxy)phosphoryl)amino)acetate (compound B) and (2*S*)-Benzyl 2-(((((2*R*,3*S*,4*R*,5*R*)-5-(6-amino-8-chloro-9*H*-purin-9-yl)-3,4-dihydroxy-tetrahydrofuran-2-yl)methoxy)(naphthalen-1-yloxy)phosphoryl)amino)propanoate (compound D) may exist as diastereomeric forms in the same way as other proTides such as NUC-1031 (acelarin) may do due to the presence of a chiral phosphorus atom. During synthesis, the coupling reaction typically involves the formation of a new chiral centre and the above 2 proTides are each formed as a mixture of two diastereoisomers. The same is true for other compounds described in this application.

Since the phosphate centre is chiral in the compounds of the present invention and the compounds may independently exist as Rp and Sp diastereoisomers it is possible for the compounds to be present as mixed Rp and Sp or one pure diastereoisomer.

The diastereomers can be detected individually by ³¹P-NMR and separated by chromatography, each isolated peak providing one of the two ³¹P-NMR signals present in the mixture.

The separation technique may be chromatography, e.g. column chromatography, preparative thin layer chromatography, preparative HPLC or flash purification by Biotage Isolera™. When the separation technique is preparative HPLC a reverse phase column C-18 maybe used. An example of reverse phase column is Varian Pursuit XRs 5 C-18. When the separation technique is Biotage Isolera™ a Reverse phase cartridge may be used. An example of reverse phase cartridge is SNAPUltra C-18.

On the basis of comparison of ³¹P chemical shift, 1H NMR spectra, and HPLC retention times with those of other ProTides known in the literature, isomer A of compound B was tentatively assigned as the *R*p diastereomer and isomer B of compound B was tentatively assigned as the Sp diastereomer. Similarly, isomer A of compound D was tentatively assigned as the Sp diastereomer and isomer B of compound D was tentatively assigned as the *R*p diastereomer. The absolute structures may also be determined by conventional X-ray crystallographic analysis.

The (*R*p) and *S*p) isomers of the two compounds were separated by prep-HPLC under the following conditions:
**Equipment:** Varian Prostar (LC Worksttion-Varian Prostar 335 LC detector)
**Flow rate:** 20 mL/min
**Column:** Varian Pursuit XRs 5 C-18, 150x21
**Temperature:** ambient
**Feed:** dissolved in MeOH
**Loading:** 20 mg
**Solvent:** Isocratic 55% MeOH in water

The (*R*p) and *S*p) isomer of each compound was separated by isolera Biotage under the following condition:
**Equipment: Isolera Biotage™**
**Flow rate: 12 mL/min**
**Column:** SNAP Ultra C-18 12g
**Temperature:** ambient
**Feed:** dissolved in MeOH
**Loading:** 50 mg
**Solvent:** Isocratic 55% MeOH in water
The supporting experimental evidence is shown below

HPLC traces for (2*S*)-Benzyl 2-(((((2*R*,3*S*,4*R*,5*R*)-5-(6-amino-8-chloro-9*H*-purin-9-yl)-3,4-dihydroxy-tetrahydrofuran-2-yl)methoxy)(naphthalen-1-yloxy)phosphoryl)amino)propanoate (compound D) are shown in Figure 9.

### Compound D isomer A

¹H-NMR (500 MHz; MeOD-*d4*): δ_{H} 8.07 (1H, d *J=* 8.5Hz, *H*-Napht), 8.05 (1H, s, 2-H), 7.87 (1H, d *J=* 8.5Hz, *H*-Napht), 7.67 (1H, d *J=* 8.5Hz, *H*-Napht) 7.54-7.48 (2H, m, *H*-Napht), 7.41-7.30 (1H, m, *H*-Napht), 7.36-7.33 (1H, m, *H*-Napht), 7.26-7.22 (5H, m, -CH₂*Ph*)*,* 6.03 (1H, *dJ=* 5.0 Hz, H-1'), 5.33 (1H, t *J=* 5.0Hz, H-2'), 5.01, 4.98 (AB, *J*_{AB} = 12.3 Hz, CH₂Ph), 4.65 (1H t *J=* 5.5Hz, H-3'), 4.49-4.45 (1H, m, Hₐ-5'), 4.41-4.36 (1H, m, H_{b}-5'), 4.22-4.20 (1H, m, H-4'), 3.94-3.90 (1H, m, -*CH*CH₃), 1.17 (1H, d *J* = 7.0 Hz, CH₃).³¹P NMR (202 MHz, MeOD-*d4*): δ_{P} 3.93 (IP, s). Reverse-phase HPLC, eluting with H₂O/ CH₃CN from 100/10 to 0/100 in 30 min; 1 mL/min, λ = 254 nm, showed a peak with t_{R=} 16.43 min

³¹P NMR (202 MHz, MeOD-*d4*): δ_{P} 3.93

### Compound D isomer B

¹H-NMR (500 MHz; MeOD-*d4*): δ_{H} 8.10 (1H, s, H-2), 8.08 (1H, d *J*= 8.5Hz, *H*-Napht), 7.87 (1H, d *J*= 8.5Hz, *H*-Napht),7.67 (1H, d *J*= 8.5Hz, *H*-Napht), 7.53-7.50 (1H, m, *H*-Napht), 7.48-7.44 (1H, m, *H*-Napht), 7.40-7.38 (1H, m, *H*-Napht), 7.33-7.27 (6H, m, *H*-Napht and - CH₂Ph), 6.02 (1H, d *J* = 5.0 Hz, H-1'), 5.28 (1H, t *J*= 5.0Hz, H-2'), 5.04, 5.02 (AB, *J*_{AB} = 12.2 Hz, CH₂Ph), 4.63 (1Ht *J*= 5.5Hz, H-3'), 4.48-4.46 (1H, m, Hₐ-5'), 4.38-4.35 (1H, m, H_{b}-5'), 4.23-4.20 (1H, m, H-4'),4.05-4.01 (1H, m, -*CH*CH₃), 1.17 (1H, d *J* = 7.0 Hz, CH₃).³¹P NMR (202 MHz, MeOD-*d4*): δ_{P} 3.83 (IP, s). Reverse-phase HPLC, eluting with H₂O/CH₃CN from 100/10 to 0/100 in 30 min; 1 mL/min, λ = 254 nm, showed a peak with t_{R}= 16.59 min

³¹P NMR (202 MHz, MeOD-*d4*): δ_{P} 3.83

HPLC traces for benzyl 2-(((((2*R*,3*S*,4*R*,5*R*)-5-(6-amino-8-chloro-9*H*-purin-9-yl)-3,4-dihydroxytetra-hydrofuran-2-yl)methoxy)(phenoxy)phosphoryl)amino)acetate (compound B) are shown in Figure 10.

### Compound B isomer A

¹H-NMR (500 MHz; MeOD-*d4*): δ_{H} 8.03 (1H, s, H-2), 8.23-7.13 (7H, m, CH₂*Ph* and *Ph),* 7.05-6.99 (3H, m, *Ph),* 5.90 (1H, d *J* =5.0 Hz, H-1'), 5.22 (1H, t *J*= 5.0Hz, H-2'), 5.01, 4.97 (AB, *J*_{AB} = 12.2 Hz, CH₂Ph), 4.48 (1Ht *J*= 5.5Hz, H-3'), 4.53-4.47 (1H, m, Hₐ-5'), 4.23-4.18 (1H, m, H_{b}-5'), 4.08-4.050 (1H, m, H-4'),3.56 (1H, d *J*= 8.5Hz, -C*H₂ₐ*NH₂), 3.54 (1H, d *J*= 8.5Hz, -*CH₂ₐ*NH₂). ³¹P NMR (202 MHz, MeOD-*d4*): δ_{P} 4.63 (1P, s). ³¹P NMR (202 MHz, MeOD-d4): δ_{P} 4.64

Reverse-phase HPLC, eluting with H₂O/ CH₃CN from 100/10 to 0/100 in 30 min; 1 mL/min, λ = 254 nm, showed a peak with t_{R}= 12.58 min

### Compound B isomer B

¹H-NMR (500 MHz; MeOD-*d4*): δ_{H} 8.06 (1H, s, 2-H), 7.23-7.16 (7H, m, -CH₂*Ph* and *Ph),* 7.05-6.99 (3H, m, *Ph),* 5.92 (1H, d *J* = 5.0 Hz, H-1'), 5.20 (1H, t *J=* 5.0Hz, H-2'), 5.01, 4.99 (AB, *J*_{AB} = 12.2 Hz, CH₂Ph), 4.53 (1H t *J*= 5.5Hz, H-3'), 4.33-4.23 (2H, m, H-5'), 4.12-4.07 (1H, m, H-4'), 361-3.53 (2H, m, -*CH₂*NH₂). ³¹P NMR (202 MHz, MeOD-*d4*): δ_{P} 4.57 (1P, s).

Reverse-phase HPLC, eluting with H₂O/CH₃CN from 100/10 to 0/100 in 30 min; 1 mL/min, λ = 254 nm, showed a peak with t_{R}= 12.88 min

### 2. Biological Experimental Examples

Exemplified compounds embodying the present invention were assessed for their ability to target cancer stem cells and for their anti-cancer potency.

### Example 30 - In vitro cytotoxicity analyses

The following cell lines are referred to in the experimental data set out in Table 2 below:
Lovo: cell line derived from a metastatic site in a patient with colon cancer
MCF7: human oestrogen sensitive mammary epithelial cell line
PC3: human prostate cancer cell line
A549: cancerous lung tissue derived cells
L1210: lymphoblast cell line derived from a mouse with lymphocytic leukaemia
FM3A: mammary carcinoma murine cells
CEM: human T-lymphoblasts, derived from the blood of a patient with acute lymphoblastic leukaemia
HeLa: epithelial cells from a patient with cervical adenocarcinoma
Compound D and certain comparator compounds were assayed for their cytotoxic activity in cancer cell lines, according to the assays below.

### L1210, CEM and HeLa cell line Cytotoxicity Assay

Murine leukemia L1210, human T-lymphocyte CEM, and human cervix carcinoma HeLa cells were obtained from the American Type Culture Collection (ATCC) (Rockville, MD). All cells were maintained in Dulbecco's modified Eagle's medium (DMEM) (Invitrogen, Carlsbad, CA) with 10% fetal bovine serum (FBS) (Biochrom AG, Berlin, Germany), 10 mM Hepes, and 1mM sodiumpyruvate (Invitrogen). Cells were grown at 37 °C in a humidified incubator with a gas phase of 5% CO₂. Monolayer cells (HeLa and HeLa) were seeded in 96-well microtiter plates (Nunc, Roskilde, Denmark) at 10 000 cells/well. After 24 h, an equal volume of fresh medium containing the test compounds was added. On day 5, cells were trypsinized and counted in a Coulter counter (Analis, Suarlee, Belgium). Suspension cells (L1210, CEM) were seeded in 96-well microtiter plates (Nunc) at 60 000 cells/well in the presence of a given amount of the test compounds. The cells were allowed to proliferate for 48 h (L1210) or 72 h (CEM) and were then counted in a Coulter counter. The 50% inhibitory concentration (IC₅₀) was defined as the compound concentration required to reduce cell proliferation by 50%. The cytostatic activity of the compounds was determined after 3 days, as outlined above.

### MCF7, LoVo, A549 and PC3 cell lines Cytotoxicity Assay

Anti-tumour evaluation in MCF7, LoVo, A549 and PC3 cell lines was performed by MTT assay. Compounds were prepared as 0.1-100 mM stock solutions dissolved in DMSO and stored at -20°C. Cells were seeded into 96-well microtitre plates at a density of 5x10³ cells per well and allowed 24 hours to adhere. Decimal compound dilutions were prepared in medium immediately prior to each assay (final concentration 0.1-100 µM). Experimental medium was DMEM +10% FCS (PC3 and Lovo) or RPMI +10% heat inactivated FCS (A549 and MCF7). Following 96h compound exposure at 37°C, 5% CO₂, MTT reagent (Sigma Aldrich) was added to each well (final concentration 0.5 mg/ml). Incubation at 37°C for 4h allowed reduction of MTT by viable cells to an insoluble formazan product. MTT was removed and formazan solubilised by addition of 10% Triton X-100 in PBS. Absorbance was read on a Tecan Sunrise spectrophotometer at 540 nm as a measure of cell viability; thus inhibition relative to control was determined (IC₅₀).

The results of the initial screening are presented in Table 2. A represents an absolute IC₅₀ of from 0.1 to 5, B represents an absolute IC₅₀ greater than 5 and up to 15, C represents an absolute IC₅₀ of greater than 15 and up to 100; and D represents an absolute IC₅₀ of greater than 100.

**Table 2**

| Compound | Lovo | MCF7 | PC3 | A549 | L1210 | FM3A | CEM | HeLa |
|---|---|---|---|---|---|---|---|---|
| 8-CI-Adenosine | A | A | A | A | A | A | A | A |
| w | A | C | A | C | C | C | C | C |
| X | A | A | A | C | - | - | - | - |
| C | A | C | A | C | C | C | C | B |
| Y | A | C | A | D | C | C | C | c |
| A | A | C | C | C | C | D | C | C |
| B | A | C | A | B | B | A | B | A |
| T | A | C | A | C | C | C | C | B |
| AA | A | A | C | C | C | C | C | B |
| Z | C | C | C | D | C | D | C | C |
| V | A | A | A | A | C | C | B | A |
| U | A | A | A | A | C | C | B | A |
| | | | | | | | | |
| D | | | | | B | | A | A |
| F | | | | | C | | B | A |
| G | | | | | C | | B | A |
| E | | | | | B | | A | A |
| H | | | | | B | | A | A |
| I | | | | | B | | B | A |
| J | | | | | B | | B | B |
| K | | | | | C | | B | A |
| L | | | | | B | | A | A |
| M | | | | | C | | C | C |
| N | | | | | C | | C | A |
| O | | | | | A | | A | A |
| P | | | | | B | | A | A |
| Q | | | | | B | | A | A |
| R | | | | | B | | B | A |
| S | | | | | c | | B | A |
| A | | | | | C | | C | C |
| B | | | | | B | | B | A |
| T | | | | | C | | C | B |
| U | | | | | C | | B | A |
| C | | | | | C | | C | B |
| V | | | | | c | | c | A |
| AB | | | | | B | | A | A |
| AC | | | | | A | | A | A |

All of the compounds tested showed inhibitory activity against certain cell lines.

### Example 31- Further in vitro cytotoxicity analyses

A compound of the invention and a subset of comparator compounds were then assayed for their cytotoxic activity in a broader array of different solid tumours and haematological malignancies using the following assay.

### Solid tumour and haematological malignancy assay

*In vitro* viability assay were performed to assess the effects of compounds on cell viability in selected cell lines over 72 hrs using the CellTiterGlo (CTG, Promega-G7573) assay. The tests were performed in duplicates with treatment of compounds at 9 points, 3.16 folds titration in 96 well plates over ∼72 hrs. The compound starting concentrations were 198 µM. Cell viability assay using CellTiterGlo in 96-well plate were performed. Compound treatment 72 hrs, standard growth conditions, duplicates. Compounds were dissolved to 40mM with thawed 100%. Compounds were serially diluted at 3.16 fold in thawed DMSO, and warmed to 37°C before being dissolved in media (2µl+200µl). After compounds were dissolved in media, media containing compounds were warmed to 37°C in incubator and then compounds in media were added to cell plates (50µl+50µl) in duplicates. The compounds' final concentrations were from 198µM to 19.9nM. All compound solubilities were checked and recorded again, then the plates were transferred to CO₂ tissue culture incubator immediately and incubated for 3 days. DMSO final concentration is 0.5%.

The following cell lines were tested and are referred to in the Table 3 below:

**Table 3**

| Cell line | Malignancy | Cell line | Malignancy |
|---|---|---|---|
| MOLT-4 | Acute lymphoblastic leukaemia | HEL92.1.7 | Erythroleukaemia |
| CCRFCEM | Acute lymphoblastic leukaemia | HL-60 | Promyelocytic leukaemia |
| RL | Non-Hodgkin's lymphoma | MV4-11 | Biphenotypic B myelomonocytic leukemia |
| HS445 | Hodgkin lymphoma | HepG2 | Hepatocellular carcinoma |
| RPMI8226 | Human multiple myeloma | HT29 | Colon adenocarcinoma |
| K562 | Chronic myelogenous leukaemia | BxPC-3 | Pancreatic cancer |
| KG-1 | Acute myelogenous leukaemia | MCF-7 | Breast adenocarcinoma |
| THP-1 | Acute monocytic leukaemia | MiaPaCa2 | Breast adenocarcinoma |
| Z-138 | Mantle cell lymphoma | MDAMB231 | Human breast adenocarcinoma |
| NCI-H929 | Plasmacytoma | SW620 | Colon adenocarcinoma |
| Jurkat | acute T cell leukaemia | | |

The results of the further screening are presented in Table 4. "A" represents an absolute IC₅₀ of from 0.1 to 5; "B" represents an absolute IC₅₀ greater than 5 and up to 15; "C" represents an absolute IC₅₀ of greater than 15 and up to 100; and "D" represents an absolute IC₅₀ of greater than 100.

**Table 4**

| **Cmpd.** | **CCRFCEM** | | **HEL92.1.7** | | **HS445** | | **KG-1** | | **Jurkat** | | **K562** | | **THP-1** | |
|---|---|---|---|---|---|---|---|---|---|---|---|---|---|---|
| | **IC₅₀** | **MI_{%}** | **IC₅₀** | **MI_{%}** | **IC₅₀** | **MI_{%}** | **IC₅₀** | **MI_{%}** | **IC₅₀** | **MI_{%}** | **IC₅₀** | **MI_{%}** | **IC₅₀** | **MI_{%}** |
| **8CIA** | **A** | **83** | **A** | **95** | **B** | **74** | **A** | **99** | **A** | **91** | **A** | **85** | **A** | **94** |
| **C** | B | 91 | C | 90 | D | 49 | D | 38 | C | 91 | C | 92 | C | 77 |
| **A** | B | 109 | B | 86 | D | 55 | D | 43 | C | 87 | B | 92 | C | 85 |
| **B** | A | 85 | A | 99 | B | 78 | A | 99 | A | 94 | A | 97 | A | 86 |
| **V** | B | 100 | A | 100 | C | 99 | B | 99 | B | 108 | A | 100 | A | 100 |
| **D** | A | 100 | A | 100 | B | 99 | B | 99 | A | 98 | A | 100 | A | 99 |
| **E** | A | 100 | A | 100 | C | 99 | A | 99 | A | 97 | A | 99 | A | 97 |
| **L** | B | 90 | A | 92 | | | B | 88 | | | B | 87 | | |
| **O** | C | 100 | C | 100 | | | C | 99 | | | B | 100 | | |
| **H** | B | 100 | A | 100 | | | B | 99 | | | A | 100 | | |
| **P** | B | 100 | A | 100 | | | B | 99 | | | A | 100 | | |
| **Q** | B | 100 | A | 100 | | | B | 99 | | | A | 100 | | |
| **AC** | A | 93 | A | 96 | B | 78 | A | 102 | A | 95 | A | 96 | A | 94 |

| **Cpnd** | **MiaPaCa-2** | | **SW620** | | **HepG2** | | **HT29** | | **MCF7** | | **BxPC-3** | | **MDAMB231** | |
|---|---|---|---|---|---|---|---|---|---|---|---|---|---|---|
| | **IC₅₀** | **MI_{%}** | **IC₅₀** | **MI_{%}** | **IC₅₀** | **MI_{%}** | **IC₅₀** | **MI_{%}** | **IC₅₀** | **MI_{%}** | **IC₅₀** | **MI_{%}** | **IC₅₀** | **MI_{%}** |
| **8CIA** | **A** | 93 | **A** | 88 | **A** | 82 | **A** | 79 | **A** | 75 | **B** | 67 | **A** | 74 |
| **C** | B | 98 | C | 97 | C | 84 | B | 98 | C | 100 | C | 80 | | |
| **A** | B | 106 | C | 85 | B | 85 | C | 86 | C | 94 | C | 66 | | |
| **B** | A | 96 | A | 87 | A | 86 | A | 86 | A | 92 | B | 68 | B | 76 |
| **V** | A | 99 | A | 100 | A | 100 | A | 92 | A | 97 | B | 99 | C | 99 |
| **D** | A | 99 | A | 99 | A | 102 | A | 92 | A | 99 | B | 99 | | |
| **E** | A | 100 | A | 99 | A | 101 | A | 91 | A | 98 | B | 92 | | |
| **L** | | | | | | | | | | | | | | |
| **O** | | | | | | | | | | | | | | |
| **H** | | | | | | | | | A | 99 | | | B | 99 |
| **P** | | | | | | | | | | | | | | |
| **Q** | | | | | | | | | | | | | | |
| **AC** | A | 94 | A | 94 | A | 87 | A | 87 | A | 87 | B | 68 | | |

| CpnD | **NCI-H929** | | MV4-11 | | **RL** | | **RPMI-8226** | | MOLT-4 | | HL-60 | | **Z138** | |
|---|---|---|---|---|---|---|---|---|---|---|---|---|---|---|
| | ICso | **MI_{%}** | ICso | **MI_{%}** | ICso | **MI_{%}** | ICso | **MI_{%}** | ICso | **MI_{%}** | **IC₅₀** | **MI_{%}** | **IC₅₀** | **MI_{%}** |
| **8CIA** | A | 98 | A | 100 | B | 85 | A | 98 | A | 99 | A | 99 | A | 100 |
| C | B | 101 | A | 92 | C | 94 | B | 91 | A | 95 | B | 84 | A | 86 |
| **A** | C | 103 | A | 100 | B | 98 | B | 96 | C | 110 | B | 94 | A | 99 |
| B | B | 98 | A | 99 | B | 73 | A | 99 | A | 99 | A | 99 | A | 100 |
| V | A | 99 | A | 99 | C | 51 | A | 100 | A | 100 | B | 100 | A | 102 |
| D | A | 98 | A | 100 | B | 74 | A | 100 | A | 99 | B | 100 | A | 100 |
| E | A | 98 | A | 100 | B | 77 | A | 100 | A | 99 | A | 100 | A | 100 |
| L | C | 98 | A | 97 | B | 74 | A | 98 | | | B | 94 | | |
| **O** | C | 99 | A | 96 | C | 27 | B | 100 | | | C | 100 | | |
| **H** | A | 98 | A | 100 | C | 28 | A | 100 | | | B | 100 | | |
| P | A | 99 | A | 100 | B | 66 | A | 100 | | | A | 100 | | |
| Q | B | 99 | A | 100 | B | 79 | A | 100 | | | B | 100 | | |
| AC | A | 103 | A | 100 | A | 94 | A | 98 | A | 100 | A | 100 | A | 98 |

Isomers A and B of compound D were tested separately and the results are presented in Table 5:

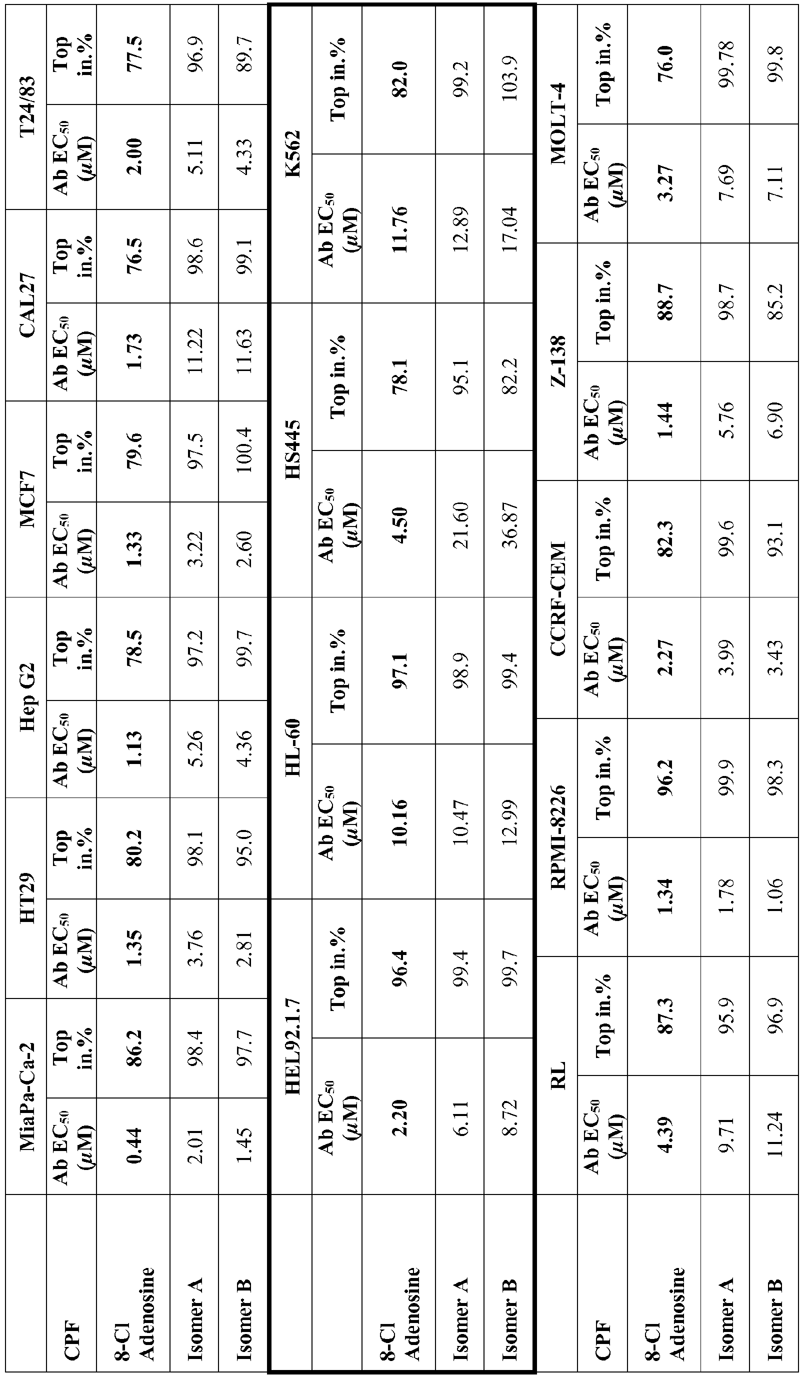

### Example 32 Assessment of cytotoxicity and cancer stem cell activity

A further comparative analysis of the toxicity of compounds in the KG1a cell line over an extended dose range was carried out, and the relative effect assessed of the compounds on the leukaemic stem cell compartment within the KGIa cell line, across the entire dose range. Thus, experimental tests were performed on certain compounds of the present invention to assess their ability to target cancer stem cells in a leukaemic cell line. The acute myeloid leukaemia (AML) cell line, KG1a, was employed to assess the relative effect of compounds on the stem cell compartment. The KG1a cell line was selected because it manifests a minor stem cell-like compartment with a distinct immunophenotype (Lin⁻/CD34⁺/CD38⁻/CD123⁺).

### Materials and Methods

### KG1a cell culture conditions

Cells of the KGIa cell line were maintained in RPMI medium (Invitrogen, Paisley, UK) supplemented with 100units/ml penicillin, 100µg/ml streptomycin and 20% foetal calf serum. Cells were subsequently aliquoted (10⁵ cells/100µl) into 96-well plates and were incubated at 37°C in a humidified 5% carbon dioxide atmosphere for 72 hrs in the presence of nucleoside analogues and their respective nucleotide analogues (known as ProTides) at concentrations that were experimentally determined for each series of compounds. In addition, control cultures were carried out to which no drug was added. Cells were subsequently harvested by centrifugation and were analyzed by flow cytometry using the Annexin V assay.

### Measurement of in vitro apoptosis

Cultured cells were harvested by centrifugation and then resuspended in 195ul of calcium-rich buffer. Subsequently, 5µl of Annexin V (Caltag Medsystems, Botolph Claydon, UK) was added to the cell suspension and cells were incubated in the dark for 10 mins prior to washing. Cells were finally resuspended in 190µl of calcium-rich buffer together with 10µl of propidium iodide. Apoptosis was assessed by dual-colour immunofluorescent flow cytometry as described previously. Subsequently LD₅₀ values (the dose required to kill 50% of the cells in a culture) were calculated for each nucleoside analogue and ProTide.

### Immunophenotypic identification of the leukaemic stem cell compartment

KGIa cells were cultured for 72 hrs in the presence of a wide range of concentrations of each compound assayed. Cells were then harvested and labelled with a cocktail of anti-lineage antibodies (PE-cy7), anti-CD34 (FITC), anti-CD38 (PE) and anti-CD123 (PERCP cy5). The sub-population expressing a leukaemic stem cell (LSC) phenotype were subsequently identified and were expressed as a percentage of all viable cells left in the culture. The percentages of stem cells remaining were then plotted on a dose-response graph and the effects of the compounds were compared with 8-chloroadenosine.

### Statistical analysis

The data obtained in these experiments were evaluated using one way ANOVA. All data was confirmed as Gaussian or a Gaussian approximation using the omnibus K2 test. LD₅₀ values were calculated from the non-linear regression and line of best-fit analysis of the sigmoidal dose-response curves. All statistical analyses were performed using Graphpad Prism 6.0 software (Graphpad Software Inc., San Diego, CA).

### Results

### In vitro cytotoxicity

The *in vitro* drug sensitivity was measured using the Annexin V/propidium iodide assay.

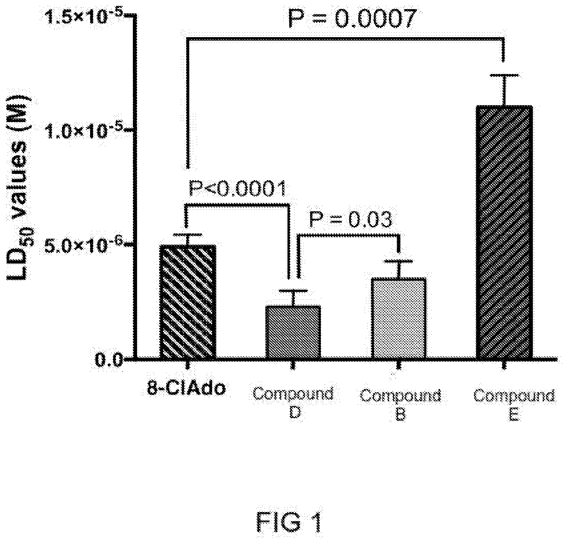

Figure 1 compares the LD₅₀ values for 8-chloroadenosine and Comparative Compounds D, B and E in unsorted populations of KG1a cells. As can be seen from this Figure, Compound D is significantly (P<0.0001) more cytotoxic in respect of cancer cell populations as a whole than is the parent prodrug compound 8-chloroadenosine. The LD50 values calculated are also shown in Table 6.

**Table 6**

| | KG-1 a unsorted LD₅₀ (µM) | KG-1 a (CD34⁺/CD38⁻/CD123⁺) % compartment |
|---|---|---|
| **Control** | - | 3.3 |
| **8-chloroadenosine** | 9.7 | 3.7 |
| **B** | 7.1 | 2.7 |
| **C** | 1.6 | 3.3 |
| **D** | 3.8 | 2.5 |
| **E** | 20 | 3.5 |

### Anti-Cancer Stem Cell Activity

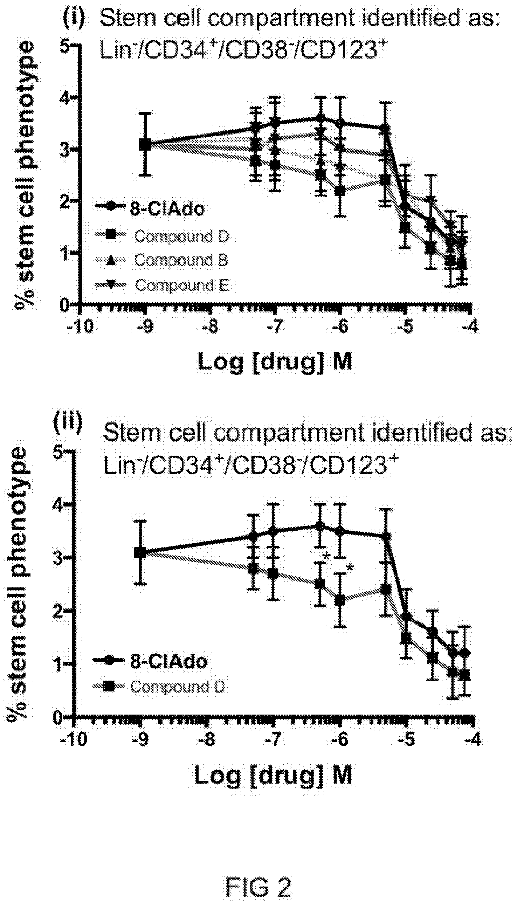

The leukaemic stem cell (LSC) targeting capacity of 8-chloroadenosine and phosphoramidate compounds B, C, D and E are also illustrated in Table 6, as well as in Figure 2. All data presented in the Table and Figure are the mean (± SD) of three independent experiments.

Figure 2 shows the individual sigmoidal dose response curve for 8-chloroadenosine phosphoramidate compounds D, B and E.

All of the compounds tested, including 8-chloroadenosine, at certain doses, reduced the proportion of leukaemic stem cells (LSCs) present among the KGIa population. Of these compounds, Comparative Compound B and Compound D demonstrated particularly effective preferential targeting of LSCs when compared to 8-chloroadenosine. These effects were observed at concentrations up to 1µM. There was no significant difference with respect to each other in the ability of B and D to deplete LSCs at the concentrations shown in Figure 2(i).

The significant differences between the anti-cancer stem cell selectivity of Compound D and 8-chloroadenosine is clearly represented in Figure 2(ii) where the results generated in respect of Comparative Compounds B and E have been removed from the graph. Targeting of cancer stem cells by Compound D leads to a statistically significantly reduction in the proportion of LSCs present after treatment with Compound D or 8-chloroadenosine at concentrations of 1 x 10⁻⁶ and 5 x 10⁻⁶ M.

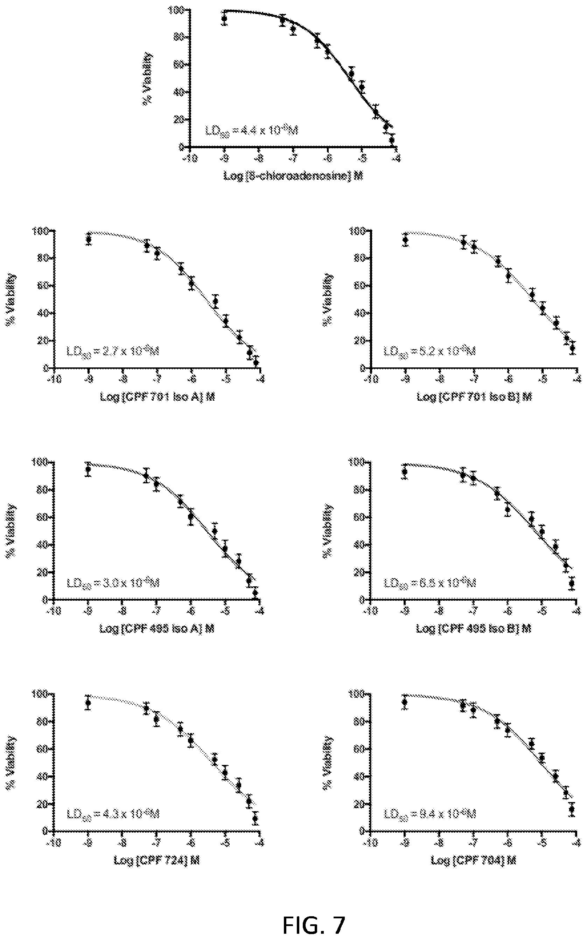

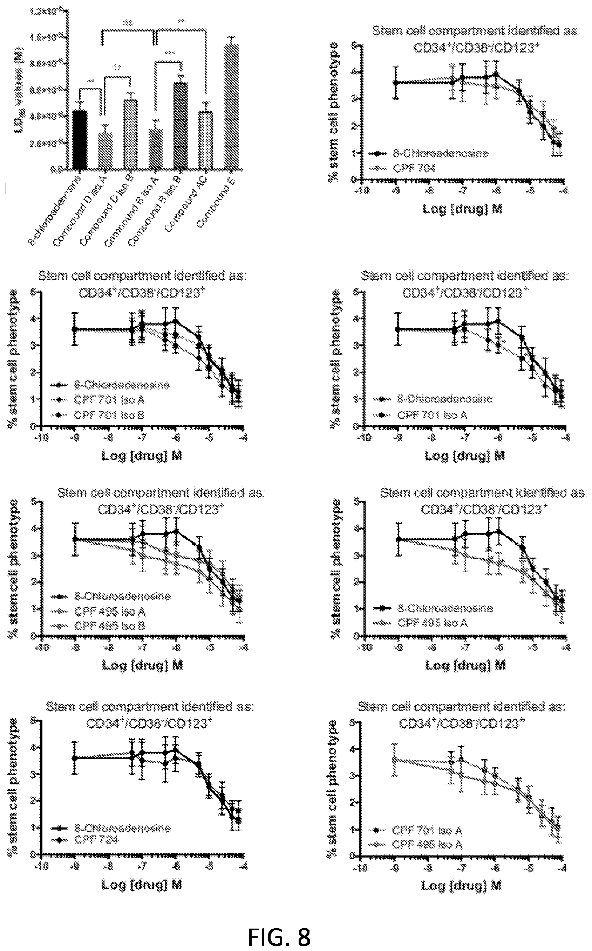

The results of further investigation of the anti-cancer stem cell activity of Compounds AC and E, and Isomers A and B of both Comparative Compound B and Compound D are shown in Figures 7 and 8.
From the results set out in Figure 7, it can be seen that Isomer A of Comparative Compound B was significantly more potent than either 8-chloroadenosine, or Isomer B of Comparative Compound B. Similarly, Isomer A of Compound D was significantly more potent than either 8-chloroadenosine, or Isomer B of Compound D. Compound E was less potent than 8-chloroadenosine, whilst Compound AC was not as cytotoxic as Isomer A of Comparative Compound B, or Isomer A of Compound D.

The results of cancer stem cell targeting studies, set out in Figure 8, illustrate that both Isomer A of Compound B and Isomer A of Compound D have a statistically significantly increased ability to target cancer stem cells as compared to 8-chloroadenosine. There was no statistically significant difference in the cancer stem cell targeting activity of Isomer A of Compound B as compared to Isomer A of Compound D. In contrast, Compound AC showed no evidence of a capacity to target cancer stem cells.

Table 7 compares the control of stem cells demonstrated by compound D of this invention with that of equivalent experiments conducted on ProTides of clofarabine and cladribine (comparative compounds 1 and 2 respectively; WO2006/100439).

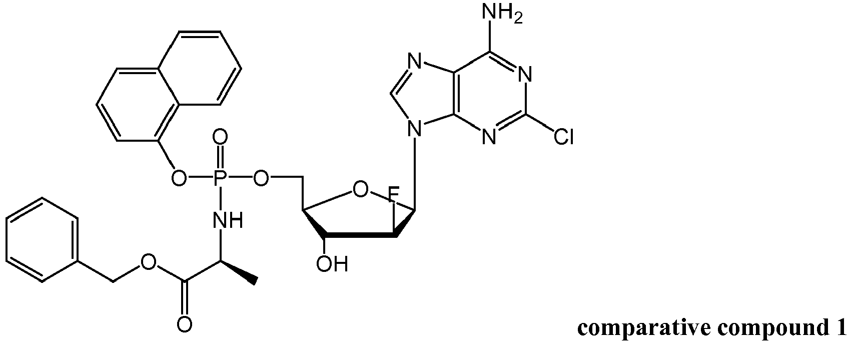

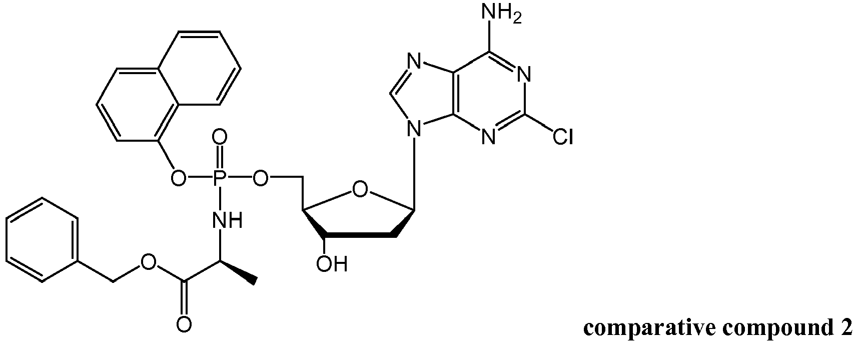

**Table 7**

| As can be seen, compound D provides better control of cancer stem cells than the clofarabine or cladribine equivalent. | | | |
|---|---|---|---|
| | KG-1a unsorted LD_{5O} (µM) | KG-1a (CD34⁺/CD38⁻/CD123⁺) % compartment | Change relative to control (%) |
| **Control - 8-CI-A series** | - | 3.3 | - |
| **8-chloroadenosine** | 9.7 | 3.7 | +0.4 |
| **Compound D** | 3.8 | 2.5 | -0.8 |
| **Control - clofarabine series** | - | 3.5 | - |
| **Clofarabine** | 0.02 | 3.3 | -0.2 |
| **Comparative compound 1** | 0.14 | 3.3 | -0.2 |
| **Control - cladribine series** | - | 4.0 | - |
| **Cladribine** | 0.18 | 6.0 | +2.0 |
| **Comparative compound 2** | 0.82 | 3.5 | -0.5 |

### Example 33 - Cytotoxicity assessment under both normal conditions and under induced resistance

The cytotoxicity and intracellular accumulation of 8-C1-ATP for compound D and two comparative compounds A and B were compared with 8-C1-A under both normal conditions and under induced resistance (*i.e.* with inhibitors).

### Cell Culture

HL-60, CTS, and K-562 are leukaemia cell lines, which were obtained from the American Type Culture Collection (ATCC), Middlesex. HL-60 cell line is of acute promyelocytic leukaemia; CTS cell line is of acute myeloid leukaemia; and K-562 cell line is of chronic myeloid leukaemia.
HL-60, CTS, and K-562 cell lines were cultured in RPMI-1640 medium (Sigma Aldrich, UK), which was supplemented with 10% Fetal Bovine Serum (FBS) (PAA Laboratories), 1% amphotericin B (5.5 ml) and 1% penicillin/streptomycin (5.5 ml) (PAA Laboratories) and grown in flasks at 37°C incubator with 5% CO₂.

### Treating Cells and Extracting Samples

Cell lines with 3x10⁶ cells/ml were used. Cells were treated with 1µl of 20µM of each 8-C1-A, compound A, compound B and compound D and incubated for 2 hours at 37°C with 5% CO₂. After incubation, the cells were centrifuged (ambient, 1200 rpm, 5 minutes), the supernatant was removed, and the pellet was washed with 1ml of PBS and centrifuged (ambient, 1200 rpm, 5 minutes). The supernatant was removed, the pellet was reconstituted in 100µl of PBS and 100µl of 0.8M perchloric acid. This was then centrifuged (ambient, 1200 rpm, 5 minutes) and 180µl of the supernatant was transferred to new tubes and stored at -80°C until time of analysis.
For inhibitor studies the cell lines were treated in the same way as described above but before treatment with drugs, a number of inhibitors were added:
1) Nitrobenzylthioinosine (NBTI) (Sigma-Aldrich, St. Louis, MO) inhibits hENT1.
2) A-134974 dihydrochloride hydrate (Sigma-Aldrich, St. Louis, MO) inhibits adenosine kinase.
3) EHNA hydrochloride (Sigma-Aldrich, St. Louis, MO) inhibits adenosine deaminase.
Cells were treated with 1µl of 10µM of each inhibitor and left for 5 minutes before adding the drug. The cells were then incubated for 2 hours at 37°C with 5% CO₂.

### LC-MS/MS Analysis

The analytes were resolved using an ultra-performance liquid chromatography system (Accela UPLC, Thermo Scientific, UK) equipped with a Biobasic Ax5µm, 50x2.1mm column (Thermo Electron Corporation, Murrieta, CA, USA) and mobile phase consisting of a mixture of 10mM NH4Ac in ACN/H₂O (30:70v/v), pH6.0 (A), and 1mM NH4Ac in ACN/H₂O (30:70v/v), pH10.5 (B). The mobile phase gradient was employed, comprising: buffer A=95% at 0-0.5 minutes, from 95 to 0% over 1.25 minutes, held at 0% for 1.75 minutes, from 0-95% over 0.1 minutes, ending with 95% for 2.9 minutes, all at a flow rate of 500µl/min.

For compounds detection a triple stage quadrupole Vantage mass spectrometry system (Thermo Scientific, UK) equipped with an electrospray ion source was used. Samples were analysed in the Multiple Reaction Monitoring, negative ion modes at spray voltage 3000V.

### Adenosine 5'-triphosphate (ATP) Assay

ATP ViaLightTM plus assay kit (Lonza, USA: Product No. LT07-121) was used for treating the cells in luminescence compatible 96 well plates (initial concentration of cells was 1x10⁴ cells/well) with 8-C1-A and ProTides, concentrations: 0, 0.1, 0.5, 1,5 and 10µM were added to cells, respectively, followed by incubation for 72 hours at 37°C incubator with 5% CO₂. For inhibitor studies, 10µM concentration of each inhibitor was added before drugs and incubated for 72 hours at 37°C with 5% CO₂.
After incubation, 50µl of cell lysis reagent was added to the 96 well plates to release the intracellular ATP, followed by 100µl of ATP monitoring reagent (AMR). The luminescent values of each well were determined using FLUOstar OPTIMA microplate reader (BMG Labtech) which converted ATP into light by using luciferase enzyme.

### Drug Stability

10ml of human plasma sample (Sera laboratories, UK) was treated with 1µM of EHNA. 1ml from this was taken along with 1µl of 20µM concentration of each drug (8-C1-A, compound A, compound B and compound D) which was added to new tubes and incubated for 2 hours at 37°C with 5% CO₂. After incubation, 100µl of each sample was taken and 300µl of 100% methanol was added and left on ice for 30 minutes. After 30 minutes, the mixture was centrifuged (14000 rpm, 4 °C, 10 minutes), the supernatant was transferred to new tubes and evaporated under speed vac. Then the dried pellet was reconstituted in 100µl of 10% Acetonitrile and transferred to LC-MS vials for injection in to the UPLC-MS/MS system.

### Statistical Analysis

The dose-response curves of cytotoxicity of the drugs was determined using non-linear regression analysis of percentage cell viability versus concentration and EC₅₀ values were obtained. The intracellular assay was conducted in five repeats for each condition. Intracellular assay was determined using paired T test (two-tailed) analysis of 8-ChloroATP/ATP concentration and p values were obtained. Drug stability in human plasma was determined using two-way ANOVA analysis. For all the analysis, Prism Software program (GraphPad Software) was used.

### Cytotoxicity assay in haematological cancer cell lines

The cytotoxicity assay results in the presence or absence of inhibitors are summarised in Table 8.

| Table 8: EC₅₀ values of 8-C1-A and ProTides when HL-60, CTS and K-562 cancer cell lines are treated under various conditions. | | | | |
|---|---|---|---|---|
| | | EC₅₀ (µM) | | |
| | | HL-60 | CTS | K-562 |
| Control | 8-C1-A | 0.64 | 0.32 | 0.64 |
| | Compound A | 12.4 | - | - |
| | Compound B | 0.49 | 16.3 | 2.86 |
| | Compound D | 0.33 | 2.64 | 6.63 |
| NBTI | 8-C1-A | 0.96 | 5.48 | 4.69 |
| | Compound A | - | 270 | - |
| | Compound B | 8.76 | 63.9 | 12.8 |
| | Compound D | 1.23 | 1.46 | 1.73 |
| A-134974 | 8-C1-A | 1.44x10¹⁰ | - | 1.451x10⁻⁸ |
| | Compound A | - | 28.0 | - |
| | Compound B | 14.62 | 1.845x10-⁸ | - |
| | Compound D | 21.3 | 10.3 | 19.7 |

### Intracellular levels of 8-ChloroATP/ATP in haematological cancer cell lines

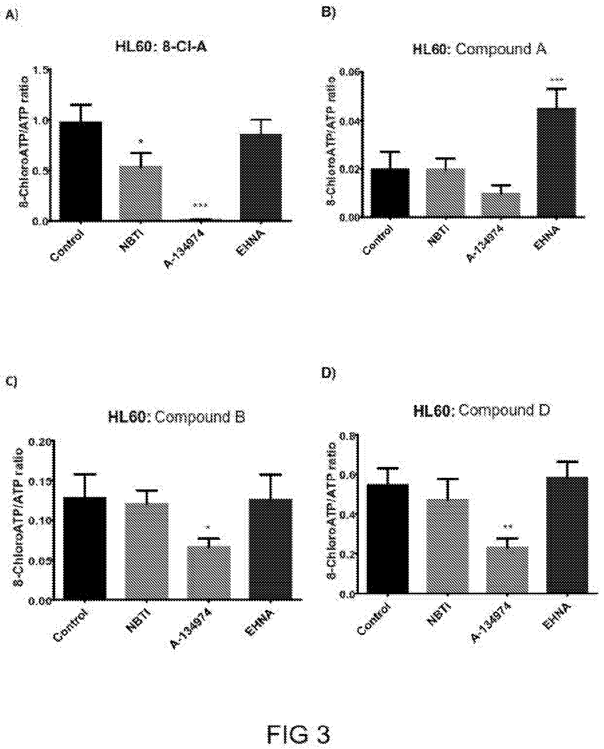

Figure 3A shows the intracellular accumulation of the active metabolite, 8-C1-ATP after treating HL-60 cell line with 8-C1-A alone or 8-C1-A with the inhibitors NBTI and A-134974. 8-C1-A alone produced 8-ChloroATP in equal concentrations to the endogenous metabolite ATP. Thus, the mean 8-C1-ATP/ATP ratio produced was found to be nearly 1. After inhibiting hENT1, this ratio was reduced by 50% (p value=0.0156), indicating that hENT1 inhibition partially blocked the uptake of 8-C1-A inside the cells. However, after inhibiting adenosine kinase, ratio was almost zero (p value=0.0004), indicating that adenosine kinase is required to activate 8-C1-A into 8-C1-ATP.

Figure 3B shows the intracellular accumulation 8-C1-ATP after treating HL-60 cells with comparative compound A alone or comparative compound A with the inhibitors NBTI and A-134974. Comparative compound A alone produced 8-C1-ATP/ATP ratio of 0.02. Inhibiting hENT1 did not produce significant difference in intracellular accumulation of 8-C1-ATP (p value=0.9568), showing that comparative compound A uptake is independent of hENT1. Blocking adenosine kinase did not produce significant difference in intracellular accumulation of 8-C1-ATP (p value=0.1016), indicating that comparative compound A activation is independent of adenosine kinase.

Figure 3C shows the intracellular accumulation of 8-C1-ATP after treating HL-60 cells with comparative compound B alone or comparative compound B with the inhibitors NBTI and A-134974. Comparative compound B alone produced 8-C1-ATP/ATP ratio of 0.13. Blocking hENT1 did not produce significant difference in intracellular accumulation of 8-C1-ATP (p value=0.9064), indicating that comparative compound B uptake is independent of hENT1. However, after inhibiting adenosine kinase, this ratio decreased by 50% (p value=0.0386), showing that comparative compound B activation is partially dependent on adenosine kinase.

Figure 3D shows the intracellular accumulation of 8-C1-ATP after treating HL-60 cells with compound D alone or compound D with the inhibitors NBTI and A-134974. Compound D alone produced 8-C1-ATP/ATP ratio of 0.6. Inhibiting hENT1 did not produce significant difference in intracellular accumulation of 8-C1-ATP (p value=0.4483), demonstrating that compound D uptake is independent of hENT1. However, after inhibiting adenosine kinase, the 8-C1-ATP/ATP ratio was reduced by 50% (p value=0.0082), indicating that compound D activation is partially dependent on adenosine kinase.

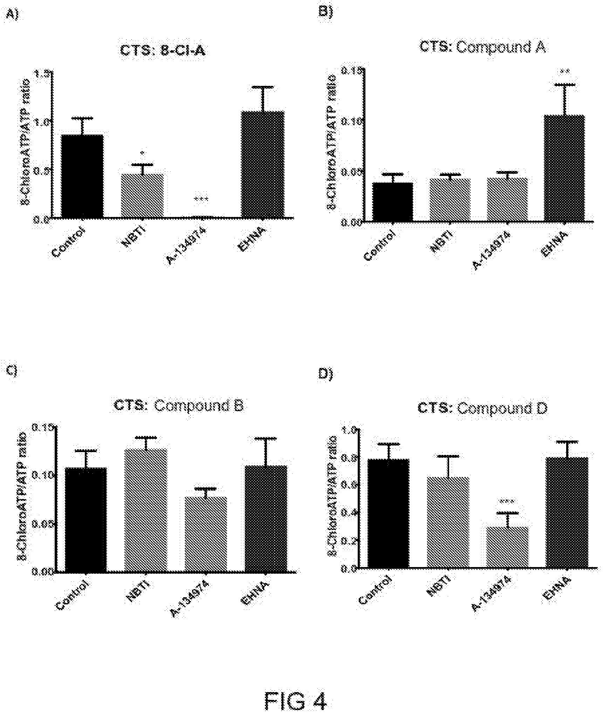

Figure 4A shows the intracellular accumulation of 8-C1-ATP after treating CTS cell line with 8-C1-A alone or 8-C1-A with the inhibitors NBTI and A-134974. 8-C1-A alone produced 8-Cl-ATP/ATP ratio of 0.7. However, after inhibiting hENT1, the 8-C1-ATP/ATP ratio decreased by 50% (p value=0.0156), indicating that 8-C1-A partially depends on hENT1 for entering the cell. However, after inhibiting adenosine kinase with A-134974, this ratio was almost zero (p value=0.0004), indicating that 8-C1-A requires adenosine kinase for activation into 8-C1-ATP.

Figure 4B shows the intracellular accumulation of 8-C1-ATP after treating CTS cells with comparative compound A alone or comparative compound A with the inhibitors NBTI and A-134974. Comparative compound A alone produced 8-C1-ATP/ATP ratio of 0.04. Blocking hENT1 did not produce significant difference in intracellular accumulation of 8-C1-ATP (p value=0.2627), demonstrating that comparative compound A did not require hENT1 transporter for entering the cell. Likewise, A-134974 did not produce significant difference in intracellular accumulation of 8-C1-ATP (p value=0.1588), indicating that comparative compound A activation is independent of adenosine kinase.

Figure 4C shows the intracellular accumulation of 8-C1-ATP after treating CTS with comparative compound B alone or comparative compound B with inhibitors NBTI and A-134974. Comparative compound B alone produced 8-C1-ATP/ATP ratio of 0.10. Inhibiting hENT1 did not produce significant difference in intracellular accumulation of 8-C1-ATP (p value=0.2725), showing that comparative compound B enters the cell without using hENT. Likewise, inhibiting adenosine kinase did not produce significant difference in intracellular accumulation of 8-C1-ATP (p value=0.9138), indicating comparative compound B is activated independent of adenosine kinase.

Figure 4D shows the intracellular accumulation of 8-C1-ATP after treating CTS cells with compound D alone or compound D with inhibitors NBTI and A-134974. Compound D alone produced 8-C1-ATP/ATP ratio of 0.8. Inhibiting hENT1 did not produce significant difference in intracellular accumulation of 8-C1-ATP (p value=0.1252), demonstrating that compound D crosses the cell membrane without using hENT1. However, blocking adenosine kinase decreased this ratio by 60% (p value=0.0009), showing that compound D activation partially requires adenosine kinase.

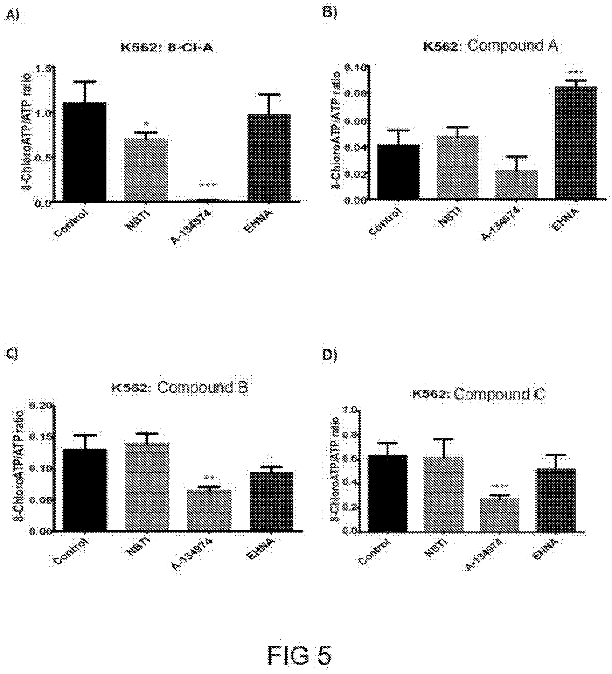

Figure 5A shows the intracellular accumulation of the active metabolite, 8-C1-ATP after treating K-562 cell line with 8-C1-A alone or 8-C1-A with the inhibitors NBTI and A-134974. 8-C1-A alone produced 8-C1-ATP in equal concentrations to the endogenous metabolite ATP, the ratio was nearly 1. However, after inhibiting hENT1, the mean 8-C1-ATP/ATP ratio was reduced by 30% (p value=0.0149), demonstrating that 8-C1-A is partially dependent on hENT1. However, after inhibiting adenosine kinase, the mean 8-C1-ATP/ATP ratio was almost zero (p value=0.0002), showing that 8-C1-A activation requires adenosine kinase.

Figure 5B shows the intracellular accumulation of 8-C1-ATP after treating K-562 cells with comparative compound A alone or comparative compound A with the inhibitors NBTI and A-134974. Comparative compound A alone produced 8-C1-ATP/ATP ratio of 0.04. Blocking hENT1 did not produce significant difference in intracellular accumulation of 8-ChloroATP (p value=0.1960), indicating that comparative compound A enters the cells independently of hENT1. Similarly, inhibiting adenosine kinase did not produce significant difference in 8-C1-ATP concentration (p value=0.1061), showing that compound A activation is independent of adenosine kinase.

Figure 5C shows that the intracellular accumulation of 8-C1-ATP after treating K-562 with comparative compound B alone or comparative compound B with the inhibitors NBTI and A-134974. Comparative compound B alone produced 8-C1-ATP/ATP ratio of 0.14. Inhibiting hENT1 did not produce significant difference in 8-C1-ATP concentration (p value=0.4797), indicating that comparative compound B enters the cells independently of hENT1. However, inhibiting adenosine kinase decreased the 8-C1-ATP/ATP ratio by 50% (p value=0.0092). This signifies that compound B activation is partially dependent on adenosine kinase.

Figure 5D shows the intracellular accumulation of 8-C1-ATP after treating K-562 cells with compound D alone or compound D with the inhibitors NBTI and A-134974. Compound D alone produced 8-C1-ATP/ATP ratio of 0.6. Blocking hENT1 did not produce significant difference in intracellular accumulation of 8-C1-ATP (p value=0.5517), showing that compound D enters the cells independently of hENT1. However, blocking adenosine kinase decreased the 8-C1-ATP/ATP ratio by 50% (p value <0.0001). This indicates that compound D activation is partially dependent on adenosine kinase.

### Stability of the drugs in human plasma

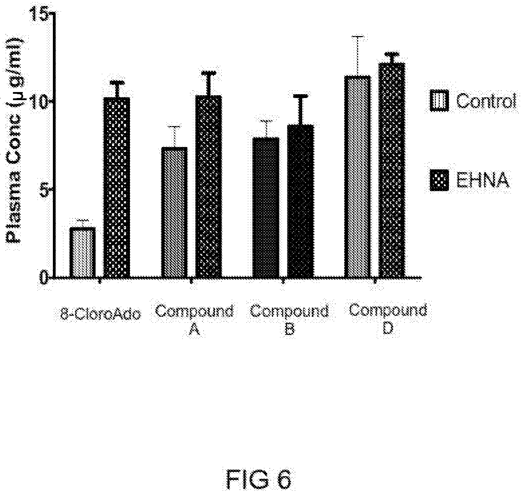

Figure 6 shows that plasma concentration of 8-C1-A was increased by ∼3-folds when adenosine deaminase was blocked by EHNA inhibitor compared to control when adenosine deaminase was not inhibited. The plasma concentration of compound D was the highest when compared with all the other compounds, whether adenosine deaminase was inhibited or not. This indicates that compound D is the most stable compound in human plasma, resisting degradation by adenosine deaminase. Comparative compound B had a low plasma concentration in the presence of EHNA and control compared to other compounds, indicating that comparative compound A resists adenosine deaminase degradation more effectively than comparative compound B.

### Discussion

In HL-60 cell line, compound D was found to be potent, with EC₅₀ 0.33µM. However, compound D generated less intracellular 8-C1-ATP/ATP than 8-C1-A (compare Figure 3A and 3D, control).

When the hENT1 inhibitor NBTI was added, cytotoxicity assay showed compound D to be most potent in CTS and K-562 cell lines. Similarly, intracellular accumulation assay showed that compound D generated higher intracellular 8-C1-ATP/ATP in CTS and K-562 cell lines. Furthermore, when comparing the potency of 8-C1-A and compound D in HL-60 cancer cell line, 8-C1-A was found to be more potent as it had lower EC₅₀ value (0.96µM) than compound D (1.23µM). When comparing the intracellular accumulation of 8-C1-ATP generated by 8-C1-A and compound D, 8-C1-A was found to have slightly higher 8-C1-ATP/ATP ratio than compound D. The data shows that compound D was most effective in CTS AND K-562 cell lines but less effective in HL-60. The data shows that compound D cellular uptake is independent of the membrane transporter hENT1. Therefore, compound D could be effective in treating patients with cancer who have a non-functional hENT1 transporter.

When the adenosine kinase A-134974 was added, cytotoxicity assay showed comparative compound B to be most potent in HL-60 cell line. Likewise, intracellular assay showed that comparative compound B generated a higher ratio of 8-C1-ATP/ATP than the other compounds in HL-60 cells in the presence of the inhibitor. This indicates that comparative compound B is potent and activated independent of adenosine kinase. However, cytotoxicity assay and intracellular assay showed compound D to be the most potent drug, which generated a high 8-C1-ATP/ATP ratio in CTS and K-562 cell lines. This shows that compound D activation is independent of adenosine kinase. Hypoxia leads to ATP depletion, and the activity of adenosine kinase becomes low in hypoxia. Therefore, compound D could be useful in treating patients with hypoxic tumours.

The drug stability assay showed that there was a ∼3-fold increase in human plasma concentration of 8-C1-A when adenosine deaminase was inhibited by EHNA. This shows that 8-C1-A is a much stronger substrate for adenosine deaminase in plasma (e.g. the plasma concentration of 8-C1-A without EHNA was 3µg/ml which increased to 10µg/ml in the presence of EHNA). This suggests that following administration in patients 8-C1-A could be extensively degraded in the plasma before reaching the tissues to exert its effects. Therefore, 8-C1-A would not be potent in patients with high plasma activity of adenosine deaminase.

Cytotoxicity assay showed that after 8-C1-A, comparative compound B was most potent in all the cell lines. However, intracellular accumulation showed compound D generated more than 70% 8-C1-ATP/ATP than comparative compound B in all the cell lines. Plasma concentrations also showed that compound D had the highest plasma concentration when compared to other compounds. Out of all the agents tested, comparative compound A showed the least potency with the least intracellular accumulation of 8-C1-ATP/ATP. On the other hand, compound D showed the strongest potency as well as the highest intracellular accumulation of 8-C1-ATP.

### Conclusion

Comparative compound B and compound D cellular uptake is independent of hENT1 nucleoside transporter proteins. The activation of both compounds is independent of adenosine kinase. This means that compounds of the invention are able to overcome the resistance mechanisms associated with 8-C1-A.

## Claims

1. (2*S*)-Benzyl 2-(((((2*R*,3*S*,4*R*,5*R*)-5-(6-amino-8-chloro-9*H*-purin-9-yl)-3,4-dihydroxy-tetrahydrofuran-2-yl)methoxy)(naphthalen-1-yloxy)phosphoryl)amino)propanoate: 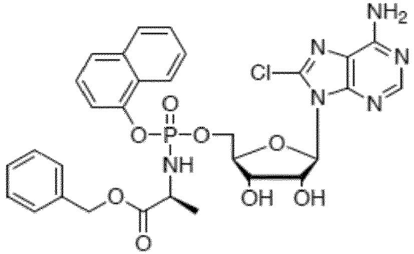 or a pharmaceutically acceptable salt, ester, salt of an ester or solvate thereof.

2. A compound of claim 1, wherein the compound is (2*S*)-Benzyl 2-(((((2*R*,3*S*,4*R*,5*R*)-5-(6-amino-*S*-chloro-9*H*-purin-9-yl)-3,4-dihydroxy-tetrahydrofuran-2-yl)methoxy)(naphthalen-1-yloxy)-(*Sp*)-phosphoryl)amino)propanoate in substantially diastereoisomerically pure form or a pharmaceutically acceptable salt, ester, salt of an ester or solvate thereof.

3. A compound of claim 1, wherein the compound is (2*S*)-Benzyl 2-(((((2*R*,3*S*,4*R*,5*R*)-5-(6-amino-8-chloro-9*H*-purin-9-yl)-3,4-dihydroxy-tetrahydrofuran-2-yl)methoxy)(naphthalen-1-yloxy)-(*Rp*)-phosphoryl)amino)propanoate in substantially diastereoisomerically pure form or a pharmaceutically acceptable salt, ester, salt of an ester or solvate thereof.

4. A compound of claim 1, wherein the compound is (2*S*)-Benzyl 2-(((((2*R*,3*S*,4*R*,5*R*)-5-(6-amino-8-chloro-9*H*-purin-9-yl)-3,4-dihydroxy-tetrahydrofuran-2-yl)methoxy)(naphthalen-1-yloxy)phosphoryl)amino)propanoate.

5. A compound of claim 1, wherein the compound is (2*S*)-Benzyl 2-(((((2*R*,3*S*,4*R*,5*R*)-5-(6-amino-8-chloro-9*H*-purin-9-yl)-3,4-dihydroxy-tetrahydrofuran-2-yl)methoxy)(naphthalen-1-yloxy)-(*Sp*)-phosphoryl)amino)propanoate in substantially diastereoisomerically pure form.

6. A compound of claim 1, wherein the compound is (2*S*)-Benzyl 2-(((((2*R*,3*S*,4*R*,5*R*)-5-(6-amino-8-chloro-9*H*-purin-9-yl)-3,4-dihydroxy-tetrahydrofuran-2-yl)methoxy)(naphthalen-1-yloxy)-(*Rp*)-phosphoryl)amino)propanoate in substantially diastereoisomerically pure form.

7. A compound of any one of claims 1 to 6 for medical use.

8. A compound of any one of claims 1 to 6 for use in treating cancer.

9. A compound for use of claim 8, wherein the cancer is selected from the group consisting of leukaemia, multiple myeloma, lung cancer, liver cancer, breast cancer, head and neck cancer, neuroblastoma, thyroid carcinoma, skin cancer, oral squamous cell carcinoma, urinary bladder cancer, Leydig cell tumour, colon cancer, colorectal cancer and gynaecological cancers.

10. A compound for use of claim 9, wherein the cancer is leukaemia.

11. The compound for use of claim 10, wherein the leukaemia is selected from the group comprising acute lymphoblastic leukaemia, acute myelogenous leukaemia, acute premyelocytic leukaemia, acute lymphocytic leukaemia, chronic myelogenous leukaemia, chronic lymphocytic leukaemia, monoblastic leukaemia and hairy cell leukaemia.

12. A compound for use according to claim 9 for use in targeting cancer stem cells in the treatment of cancer.

13. A compound for use according to claim 12, wherein the cancer is selected from leukaemia, lymphoma, breast cancer, lung cancer, colon cancer, prostate cancer, ovarian cancer, skin cancer, bladder cancer, biliary cancer and pancreatic cancer.

14. A compound for use according to claim 12, wherein the cancer is selected from breast cancer, CNS cancer, colon cancer, Ewing's sarcoma, melanoma, liver cancer, cholangiocarcinoma, ovarian cancer, pancreatic cancer, non small cell lung cancer, bladder cancer, acute myeloid leukaemia, B-acute lymphoblastic leukaemia, B-acute lymphoblastic leukaemia, multiple myeloma and T-acute lymphoblastic leukaemia.

15. A pharmaceutical formulation comprising a compound of any one of claims 1 to 6 and a pharmaceutically acceptable excipient.

## Patentansprüche

1. (2*S*)-Benzyl-2-(((((2*R*,3*S*,4*R*,5*R*)-5-(6-amino-8-chlor-9*H*-purin-9-yl)-3,4-dihydroxy-tetrahydrofuran-2-yl)methoxy)(naphthalen-1-yloxy)phosphoryl)amino)propanoat: 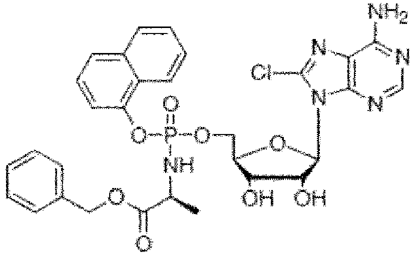 oder pharmazeutisch annehmbares/annehmbaren Salz, Ester, Salz eines Esters oder Solvat davon.

2. Verbindung nach Anspruch 1, wobei die Verbindung (2*S*)-Benzyl-2-(((((2*R*,3*S*,4*R*,5*R*)-5-(6-amino-8-chlor-9*H*-purin-9-yl)-3,4-dihydroxy-tetrahydrofuran-2-yl)methoxy)(naphthalen-1-yloxy)-(*Sp*)-phosphoryl)amino)propanoat in im Wesentlichen diastereoisomer reiner Form oder ein pharmazeutisch annehmbares/annehmbarer Salz, Ester, Salz eines Esters oder Solvat davon ist.

3. Verbindung nach Anspruch 1, wobei die Verbindung (2*S*)-Benzyl-2-(((((2*R*,3*S*,4*R*,5*R*)-5-(6-amino-8-chlor-9*H*-purin-9-yl)-3,4-dihydroxy-tetrahydrofuran-2-yl)methoxy)(naphthalen-1-yloxy)-(*Rp*)-phosphoryl)amino)propanoat in im Wesentlichen diastereoisomer reiner Form oder ein pharmazeutisch annehmbares/annehmbarer Salz, Ester, Salz eines Esters oder Solvat davon ist.

4. Verbindung nach Anspruch 1, wobei die Verbindung (2*S*)-Benzyl-2-(((((2*R*,3*S*,4*R*,5*R*)-5-(6-amino-8-chlor-9*H*-purin-9-yl)-3,4-dihydroxy-tetrahydrofuran-2-yl)methoxy)(naphthalen-1 -yloxy)phosphoryl)amino)propanoat ist.

5. Verbindung nach Anspruch 1, wobei die Verbindung (2*S*)-Benzyl-2-(((((2*R*,3*S*,4*R*,5*R*)-5-(6-amino-8-chlor-9*H*-purin-9-yl)-3,4-dihydroxy-tetrahydrofuran-2-yl)methoxy)(naphthalen-1-yloxy)-(*Sp*)-phosphoryl)amino)propanoat in im Wesentlichen diastereoisomer reiner Form ist.

6. Verbindung nach Anspruch 1, wobei die Verbindung (2*S*)-Benzyl-2-(((((2*R*,3*S*,4*R*,5*R*)-5-(6-amino-8-chlor-9*H*-purin-9-yl)-3,4-dihydroxy-tetrahydrofuran-2-yl)methoxy)(naphthalen-1-yloxy)-(*Rp*)-phosphoryl)amino)propanoat in im Wesentlichen diastereoisomer reiner Form ist.

7. Verbindung nach einem der Ansprüche 1 bis 6 zur medizinischen Verwendung.

8. Verbindung nach einem der Ansprüche 1 bis 6 zur Verwendung bei der Behandlung von Krebs.

9. Verbindung zur Verwendung nach Anspruch 8, wobei der Krebs ausgewählt ist aus der Gruppe bestehend aus Leukämie, multiplem Myelom, Lungenkrebs, Leberkrebs, Brustkrebs, Kopf- und Halskrebs, Neuroblastom, Schilddrüsenkarzinom, Hautkrebs, oralem Plattenepithelkarzinom, Harnblasenkrebs, Leydig-Zelltumor, Dickdarmkrebs, Darmkrebs und gynäkologischem Krebs.

10. Verbindung zur Verwendung nach Anspruch 9, wobei der Krebs Leukämie ist.

11. Verbindung zur Verwendung nach Anspruch 10, wobei die Leukämie ausgewählt ist aus der Gruppe, die akute lymphoblastische Leukämie, akute myeloische Leukämie, akute prämyelozytische Leukämie, akute lymphatische Leukämie, chronische myeloische Leukämie, chronische lymphatische Leukämie, monoblastische Leukämie und Haarzellenleukämie umfasst.

12. Verbindung zur Verwendung nach Anspruch 9 zur Verwendung bei der Anvisierung von Krebsstammzellen bei der Behandlung von Krebs.

13. Verbindung zur Verwendung nach Anspruch 12, wobei der Krebs ausgewählt ist aus Leukämie, Lymphom, Brustkrebs, Lungenkrebs, Dickdarmkrebs, Prostatakrebs, Eierstockkrebs, Hautkrebs, Blasenkrebs, Gallenkrebs und Bauchspeicheldrüsenkrebs.

14. Verbindung zur Verwendung nach Anspruch 12, wobei der Krebs ausgewählt ist aus Brustkrebs, ZNS-Krebs, Dickdarmkrebs, Ewing-Sarkom, Melanom, Leberkrebs, Cholangiokarzinom, Eierstockkrebs, Bauchspeicheldrüsenkrebs, nichtkleinzelligem Lungenkrebs, Blasenkrebs, akuter myeloischer Leukämie, B-akuter lymphoblastischer Leukämie, B-akuter lymphoblastischer Leukämie, multiplem Myelom und T-akuter lymphoblastischer Leukämie.

15. Pharmazeutische Zusammensetzung, umfassend eine Verbindung nach einem der Ansprüche 1 bis 6 und einen pharmazeutisch annehmbaren Hilfsstoff.

## Revendications

1. 2-(((((2*R*,3*S*,*4*R,5*R*)-5-(6-amino-8-chloro-9*H*-purin-9-yl)-3,4-dihydroxy-tétrahydrofuran-2-yl)méthoxy)(naphtalèn-1-yloxy)phosphoryl)amino)propanoate de (2*S*)-benzyle : 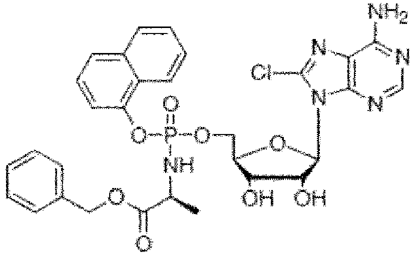 ou un sel, un ester, un sel d'un ester ou un solvate pharmaceutiquement acceptable de celui-ci.

2. Composé selon la revendication 1, dans lequel le composé est le 2-(((((2*R*,3*S*,4*R*,5*R*)-5-(6-amino-8-chloro-9*H*-purin-9-yl)-3,4-dihydroxy-tétrahydrofuran-2-yl)méthoxy)(naphtalèn-1-yloxy)-(*Sp*)-phosphoryl)amino)propanoate de (2*S*)-benzyle sous forme sensiblement diastéréoisomériquement pure ou un sel, un ester, un sel d'un ester ou un solvate pharmaceutiquement acceptable de celui-ci.

3. Composé selon la revendication 1, dans lequel le composé est le 2-(((((2*R*,3*S*,4*R*,5*R*)-5-(6-amino-8-chloro-9*H*-purin-9-yl)-3,4-dihydroxy-tétrahydrofuran-2-yl)méthoxy)(naphtalèn-1-yloxy)-(*Rp*)-phosphoryl)amino)propanoate de (2*S*)-benzyle sous forme sensiblement diastéréoisomériquement pure ou un sel, un ester, un sel d'un ester ou un solvate pharmaceutiquement acceptable de celui-ci.

4. Composé selon la revendication 1, dans lequel le composé est le 2-(((((2*R*,3*S*,4*R*,5*R*)-5-(6-amino-8-chloro-9*H*-purin-9-yl)-3,4-dihydroxy-tétrahydrofuran-2-yl)méthoxy)(naphtalèn-1-yloxy)phosphoryl)amino)propanoate de (2*S*)-benzyle.

5. Composé selon la revendication 1, dans lequel le composé est le 2-(((((2*R*,3*S*,4*R*,5*R*)-5-(6-amino-8-chloro-9*H*-purin-9-yl)-3,4-dihydroxy-tétrahydrofuran-2-yl)méthoxy)(naphtalèn-1-yloxy)-(*Sp*)-phosphoryl)amino)propanoate de (2*S*)-benzyle sous forme sensiblement diastéréoisomériquement pure.

6. Composé selon la revendication 1, dans lequel le composé est le 2-(((((2*R*,3*S*,4*R*,5*R*)-5-(6-amino-8-chloro-9*H*-purin-9-yl)-3,4-dihydroxy-tétrahydrofuran-2-yl)méthoxy)(naphtalèn-1-yloxy)-(*Rp*)-phosphoryl)amino)propanoate de (2*S*)-benzyle sous forme sensiblement diastéréoisomériquement pure.

7. Composé selon l'une quelconque des revendications 1 à 6 pour une utilisation médicale.

8. Composé selon l'une quelconque des revendications 1 à 6 destiné à être utilisé dans le traitement du cancer.

9. Composé destiné à être utilisé selon la revendication 8, dans lequel le cancer est choisi dans le groupe constitué par la leucémie, le myélome multiple, le cancer du poumon, le cancer du foie, le cancer du sein, le cancer de la tête et du cou, le neuroblastome, le carcinome thyroïdien, le cancer de la peau, le carcinome à cellules squameuses oral, le cancer de la vessie urinaire, la tumeur à cellules de Leydig, le cancer du côlon, le cancer colo-rectal et les cancers gynécologiques.

10. Composé destiné à être utilisé selon la revendication 9, dans lequel le cancer est la leucémie.

11. Composé destiné à être utilisé selon la revendication 10, dans lequel la leucémie est choisie dans le groupe composé par la leucémie lymphoblastique aiguë, la leucémie myéloïde aiguë, la leucémie pré-myélocytaire aiguë, la leucémie lymphocytaire aiguë, la leucémie myéloïde chronique, la leucémie lymphocytaire chronique, la leucémie monoblastique et la leucémie à tricholeucocytes.

12. Composé destiné à être utilisé selon la revendication 9 destiné à cibler les cellules souches cancéreuses dans le traitement du cancer.

13. Composé destiné à être utilisé selon la revendication 12, dans lequel le cancer est choisi parmi la leucémie, le lymphome, le cancer du sein, le cancer du poumon, le cancer du côlon, le cancer de la prostate, le cancer ovarien, le cancer de la peau, le cancer de la vessie, le cancer biliaire et le cancer pancréatique.

14. Composé destiné à être utilisé selon la revendication 12, dans lequel le cancer est choisi parmi le cancer du sein, le cancer du SNC, le cancer du côlon, le sarcome d'Ewing, le mélanome, le cancer du foie, le cholangiocarcinome, le cancer ovarien, le cancer pancréatique, le cancer du poumon non à petites cellules, le cancer de la vessie, la leucémie myéloïde aiguë, la leucémie lymphoblastique B-aiguë, la leucémie lymphoblastique B-aiguë, le myélome multiple et la leucémie lymphoblastique T-aiguë.

15. Formulation pharmaceutique comprenant un composé selon l'une quelconque des revendications 1 à 6 et un excipient pharmaceutiquement acceptable.
